# EUROPEAN PATENT APPLICATION

(11) **EP 4 649 961 A2**
(43) Date of publication of application: **19.11.2025**
(21) Application number: 25203135.6
(22) Date of filing: 14.05.2021
(51) Int. Cl.: A61K 45/00

(54) **METHODS AND COMPOSITIONS FOR TREATING ANEMIA USING ACTRIIB LIGAND TRAPS AND MTOR INHIBITORS**

(30) Priority: 15.05.2020 US 202063025955 P
(62) Divisional of application: 21803281.1
(71) Applicant: Celgene Corporation, Princeton, NJ 08543 (US)
(72) Inventor: ACAR, Melih, Daly City, 94014 (US); SCHWICKART, Martin, San Francisco, 94158 (US); JUPELLI, Madhulika, San Francisco, 94518 (US)
(74) Representative: Carpmaels & Ransford LLP

(57) **Abstract**

Provided herein are methods of treating anemia or for enhancing late stage erythropoiesis in a subject comprising administering to the subject an activin type IIB (ActRIIB) ligand trap and an mTOR inhibitor.

## Description

### CROSS-REFERENCE TO RELATED APPLICATIONS

This application claims the benefit of U.S. Provisional Application No. 63/025,955, filed May 15, 2020, the content of which is incorporated by reference herein in its entirety.

### 1. REFERENCE TO SEQUENCE LISTING SUBMITTED ELECTRONICALLY

This application incorporates by reference in its entirety the Computer Readable Form (CRF) of a Sequence Listing submitted herewith. The Sequence Listing text file submitted herewith, entitled "14247-488-228_SEQ_LISTING.txt", was created on May 7, 2021, and is 73,399 bytes in size.

### 2. FIELD

Provided herein are methods of treating anemia or for enhancing late stage erythropoiesis in a subject comprising administering to the subject an activin type IIB (ActRIIB) ligand trap and an mTOR inhibitor.

### 3. BACKGROUND

Anemia is a decrease in number of red blood cells or less than the normal quantity of hemoglobin in the blood. Anemia can also be caused by decreased oxygen-binding ability of the hemoglobin.

Anemia can be caused by ineffective erythropoiesis. Ineffective erythropoiesis is present if active erythropoiesis takes place but mature red blood cells fail to develop at a proper rate. Progenitor cells undergo apoptosis before the stage of mature red blood cells is reached. Myelodysplastic syndromes (MDS) comprises hematopoietic stem-cell disorders characterized by ineffective hematopoiesis. Moreover, MDS disorders include disorders characterized by ring sideroblasts. Ring sideroblasts are abnormal erythroblasts. Furthermore, certain somatic mutations associated with MDS cause ring sideroblast formation and ineffective erythropoiesis. Dominant mutations in splicing factor 3B1 (SF3B 1) are associated with the formation of ring sideroblasts. Ring sideroblasts are erythroblasts in which there are a minimum of five iron-containing (siderotic) granules covering at least one third of the circumference of the nucleus. *See,* e.g., Mufti et al., 2008, Haematologica 93(11):1712-7. Ring sideroblasts contain iron-loaded mitochondria. The presence of ring sideroblasts can be detected by Prussian blue staining and visualization. Ring sideroblasts can be detected in peripheral blood and/or bone marrow smears.

Anemia can be associated with chronic myelomonocytic leukemia (CMML). The most common sign of CMML is having too many monocytes, which can settle in liver and spleen, causing them to enlarge.

In addition, anemia can be associated with thalassemia. There are two main types of thalassemia: alpha and beta. Hemoglobin molecules are made of alpha and beta chains that can be affected by mutations. In thalassemia, the production of either the alpha or beta chains is reduced, resulting in either alpha-thalassemia or beta-thalassemia. Beta-thalassemia, as one of the most common inherited hemoglobinopathies worldwide, is due to autosomal mutations in the gene encoding β-globin, which induce an absence of or low-level synthesis of this protein in erythropoietic cells (Weatherall, 2001, Nature Reviews Genetics 2(4):245-255). About 80 to 90 million people (~ 1.5% of the global population) are carriers of beta-thalassemia with approximately 60,000 symptomatic individuals born annually (Modell et al., 2007, Scand. J. Clin. Lab. Invest. 67(1):39-69). The annual incidence of symptomatic individuals is estimated at 1 in 100,000 worldwide and 1 in 10,000 in the European Union (Galanello and Origa, 2010, Orphanet. J. Rare Dis. 5:11). Incidence is highest in the Mediterranean region, the Middle East, and South East Asia (particularly India, Thailand and Indonesia; this region accounts for approximately 50% of affected births) and incidence is increasing worldwide (e.g., in Europe, the Americas and Australia) as a result of migration (Colah et al., 2010, Expert Rev. Hematol. 3(1):103-117; Modell et al., 2008, Bull. World Health Organ. 86(6):480-487).

Beta-thalassemias are characterized by a reduction of β-globin chains and a subsequent imbalance in globin chains (ratio of α chains to non-α chains) of the hemoglobin (Hb) molecule, which results in impaired erythropoiesis and other complications. Nearly 200 different mutations have been described in patients with beta-thalassemia that affect the beta-globin gene, for which patients may be either homozygous or compound heterozygous. Phenotypic effects, therefore, range widely in patients from slight impairment to complete inhibition of beta-globin chain synthesis (Thein, 2013, Cold Spring Harb. Perspect. Med. 3(5):a011700). In addition to deficient β-globin chains, patients may also present with β-thalassemia combined with structural variants such as HbE, leading to HbE/beta-thalassemia.

Given the current lack of safe and effective drug therapies to treat anemia associated with ineffective erythropoiesis, thalassemia, alpha-thalassemia, beta-thalassemia, MDS, or non-proliferative CMML, there is significant unmet medical need for the development of new therapies that specifically address the underlying pathophysiology of syndromes including anemia and complications of ineffective erythropoiesis.

Two related type II receptors, ActRIIA and ActRIIB, have been identified as the type II receptors for activins (Mathews and Vale, 1991, Cell 65:973-982; Attisano et al., 1992, Cell 68: 97-108). Besides activins, ActRIIA and ActRIIB can biochemically interact with several other TGF-beta family proteins, including BMP7, Nodal, GDF8, and GDF11 (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B.

Luspatercept, an ActRIIB ligand trap, has been described for treatment of various indications. *See, e.g.,* U.S. Patent Application Publication No. US 2018/0050085 A1, U.S. Patent No. 8,058,229, U.S. Patent No. 8,361,957, and U.S. Patent No. 8,343,933.

### 4. SUMMARY

Provided herein are methods of treating anemia or for enhancing late stage erythropoiesis in a subject comprising administering to the subject an ActRIIB ligand trap and administering to the subject an mTOR inhibitor.

In certain embodiments, the anemia is an anemia associated with ineffective erythropoiesis, thalassemia, alpha-thalassemia, beta-thalassemia, myelodysplastic syndromes (MDS), or non-proliferative chronic myelomonocytic leukemia.

In certain embodiments, the mTOR inhibitor is rapamycin. In certain embodiments, the mTOR inhibitor is a pharmaceutically acceptable salt or hydrate of rapamycin.

In certain embodiments, the mTOR inhibitor is rapamycin. In certain embodiments, the mTOR inhibitor is a pharmaceutically acceptable salt or hydrate of rapamycin. In certain embodiments, the mTOR inhibitor is Sirolimus. In certain embodiments, the mTOR inhibitor is a pharmaceutically acceptable salt or hydrate of Sirolimus. In certain embodiments, the mTOR inhibitor is deforolimus. In certain embodiments, the mTOR inhibitor is a pharmaceutically acceptable salt or hydrate of deforolimus. In certain embodiments, the mTOR inhibitor is everolimus. In certain embodiments, the mTOR inhibitor is a pharmaceutically acceptable salt or hydrate of everolimus. In certain embodiments, the mTOR inhibitor is temsirolimus. In certain embodiments, the mTOR inhibitor is a pharmaceutically acceptable salt or hydrate of temsirolimus. In certain embodiments, the mTOR inhibitor is ridaforolimus. In certain embodiments, the mTOR inhibitor is a pharmaceutically acceptable salt or hydrate of ridaforolimus. In certain embodiments, the mTOR inhibitor is tacrolimus (FK-506). In certain embodiments, the mTOR inhibitor is a pharmaceutically acceptable salt or hydrate of tacrolimus (FK-506). In certain embodiments, the mTOR inhibitor is zotarolimus (ABT-578). In certain embodiments, the mTOR inhibitor is a pharmaceutically acceptable salt or hydrate of zotarolimus (ABT-578). In certain embodiments, the mTOR inhibitor is a non-rapamycin analog mTOR inhibiting compound. In certain embodiments, the mTOR inhibitor is a pharmaceutically acceptable salt or hydrate of a non-rapamycin analog mTOR inhibiting compound. In certain embodiments, the mTOR inhibitor is an ATP competitive inhibitors.

In certain embodiments, the ActRIIB ligand trap is a polypeptide comprising an amino acid sequence that is at least: (a) 90% identical to SEQ ID NO:3; (b) 95% identical to SEQ ID NO:3; (c) 98% identical to SEQ ID NO:3; (d) SEQ ID NO:3; (e) 90% identical to SEQ ID NO:6; (f) 95% identical to SEQ ID NO:6; (g) 98% identical to SEQ ID NO:6; (h) SEQ ID NO:6; (i) 90% identical to SEQ ID NO:7; (j) 95% identical to SEQ ID NO:7; (k) 98% identical to SEQ ID NO:7; (l) SEQ ID NO:7; (m) 90% identical to SEQ ID NO:11; (n) 95% identical to SEQ ID NO:11; (o) 98% identical to SEQ ID NO:11; or (p) SEQ ID NO:11. In certain embodiments, the ActRIIB ligand trap is a product resulting from expression from an opening reading frame with the nucleotide sequence of SEQ ID NO:34 or a degenerate version of SEQ ID NO:34 that encodes SEQ ID NO:11. In certain embodiments, the ActRIIB ligand trap is a humanized fusion-protein consisting of the extracellular domain of ActRIIB and the human IgG1 Fc domain.

In certain embodiments, the method increases hemoglobin (HGB) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject prior to said treating.

In certain embodiments, the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HCT levels in the subject prior to said treating.

In certain embodiments, the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than MCV levels in the subject prior to said treating.

In certain embodiments, the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than CHC levels in the subject prior to said treating.

In certain embodiments, the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100% less than the RDW levels in the subject prior to said treating.

In certain embodiments, the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in the subject prior to said treating.

In certain embodiments, the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in a reference population. In a particular embodiment, the reference population consists of 1, 5, 10, 25, 50, 75, 100, 200, 250, 300, 400, 500, or 1000 individuals. In a particular embodiment, the reference population consists of healthy individuals. In a particular embodiment, the reference population consists of people of the same age, weight, and/or gender as the subject.

In certain embodiments, levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in the subject prior to said treating.

In certain embodiments, the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in a reference population. In a particular embodiment, the reference population consists of 1, 5, 10, 25, 50, 75, 100, 200, 250, 300, 400, 500, or 1000 individuals. In a particular embodiment, the reference population consists of healthy individuals. In a particular embodiment, the reference population consists of people of the same age, weight, and/or gender as the subject.

In certain embodiments, the mTOR inhibitor is administered before or concurrently with the administration of ActRIIB ligand trap. In certain embodiments, the subject has been previously treated with the mTOR inhibitor prior the administration of ActRIIB ligand trap. In certain embodiments, the subject has been previously treated with the ActRIIB ligand trap prior the administration of mTOR inhibitor.

In certain embodiments, the subject is red blood cell non-transfusion-dependent. In certain embodiments, the subject is red blood cell transfusion-dependent.

In certain embodiments, the ActRIIB ligand trap is administered at a dose of 0.6 mg/kg, 0.8 mg/kg, 1 mg/kg, 1.33 mg/kg, or 1.75 mg/kg. In certain embodiments, the ActRIIB ligand trap is administered to the subject once every 21 days. In certain embodiments, the ActRIIB ligand trap is administered twice per week. In certain embodiments, the ActRIIB ligand trap is administered once per week. In certain embodiments, the ActRIIB ligand trap is administered to the subject subcutaneously. In certain embodiments, the subject is a human.

In certain embodiments, the mTOR inhibitor is administered orally. In certain embodiments, the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg. In certain embodiments, the mTOR inhibitor is administered daily.

In another aspect, provide herein methods for treating anemia or for enhancing late stage erythropoiesis in a subject in need thereof, comprising: administering to the subject an ActRIIB ligand trap; and administering to the subject an mTOR inhibitor, wherein the method increases hemoglobin (HGB) levels in a reference population to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in the subject who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the levels of reticulocytes in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In yet another aspect, provide herein are methods for treating anemia or for enhancing late stage erythropoiesis in a subject in need thereof, comprising: administering to the subject an ActRIIB ligand trap; and administering to the subject an mTOR inhibitor, wherein the levels of white blood cells in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of individuals with anemia associated with ineffective erythropoiesis, thalassemia, alpha-thalassemia, beta-thalassemia, myelodysplastic syndromes (MDS), or non-proliferative chronic myelomonocytic leukemia (CMML). In certain embodiments, the reference population consists of healthy individuals.

### 5. BRIEF DESCRIPTION OF THE DRAWINGS

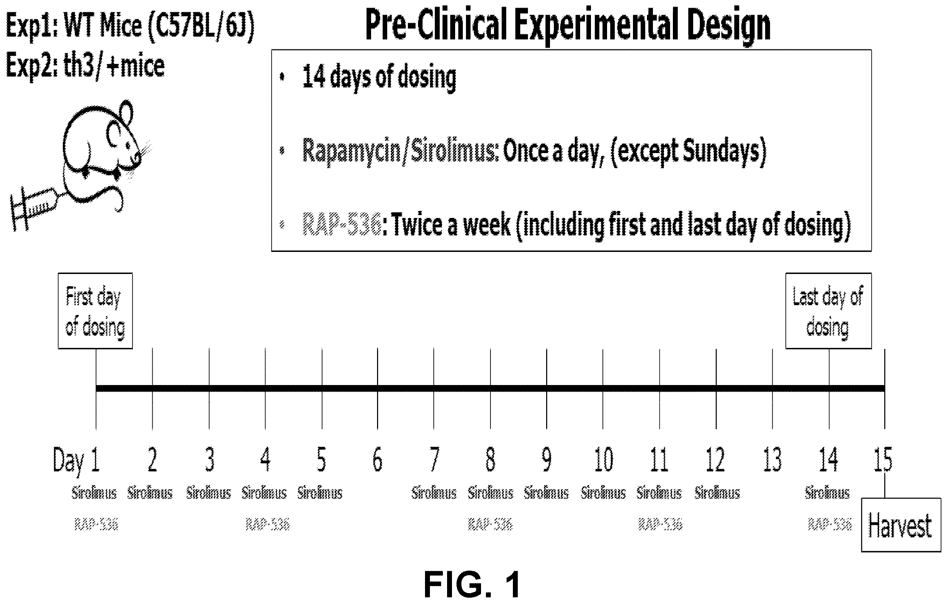

**FIG. 1** depicts the pre-clinical experimental design. For experiment 1 (Exp1), 10-12 week old Wild-type (C57BL/6J) female mice (Jackson Lab Stock# 000664) were used. For experiment 2 (Exp1), 9-18 week old B6.129P2-Hbb-b1^{tm1Unc} Hbb-b2^{tm1Unc}/J (th3/+) female mice on 000664 C57BL/6J background (Jackson Lab Stock# 002683, beta-thalassemia mouse model) were used. A murine version of luspatercept (RAP-536) in 1X Phosphate Buffered Saline (PBS) with a total volume of 150 µl and at 10 mg/kg dose was injected via subcutaneous (SC) route twice a week for two weeks. Rapamycin at 4 mg/kg dose in 1xPBS that contains 5% Tween 80, 5% PEG400, 4% Ethanol and in a total volume of 150 µl was injected via the intraperitoneal route everyday, except for day 6 and day 13, for two weeks. Tissues were harvested on day 15.

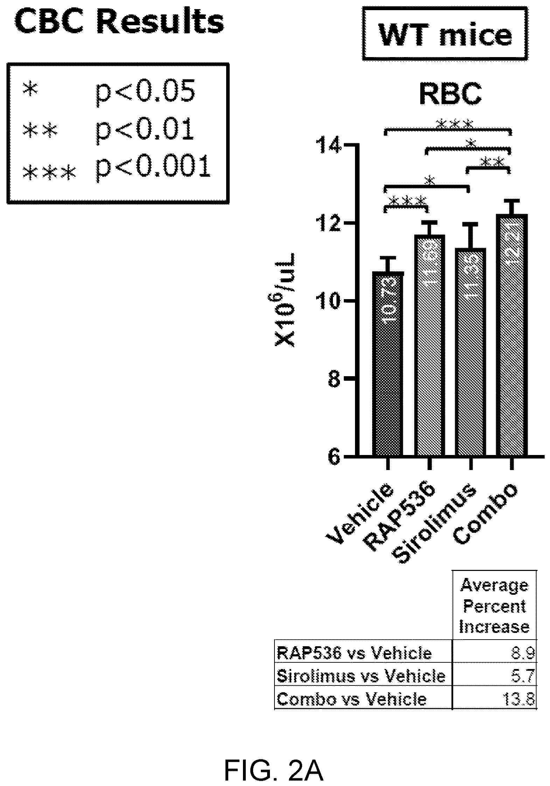

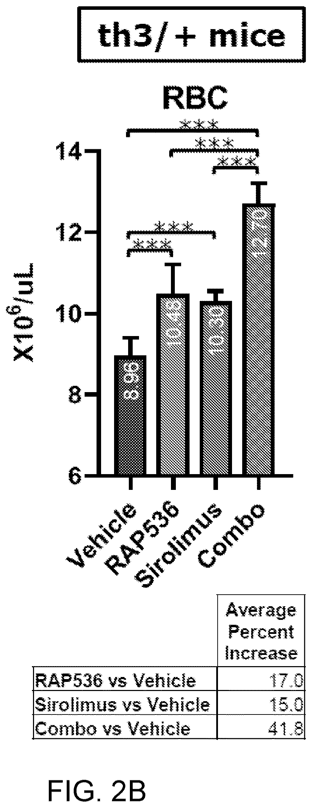

**FIGs. 2A** and **2B** demonstrate that the co-dosing of rapamycin and the murine version of luspatercept (RAP-536) increased the red blood cell (RBC) levels in blood more than single agent dosing. RAP-536 and rapamycin increased RBC levels at similar levels. The percentage increase of RBC in th3/+ mice (B6.129P2-Hbb-b1^{tm1Unc} Hbb-b2^{tm1Unc}/J) is greater than wild-type (C57BL/6J) mice. RBC numbers reached to wild-type levels in th3/+ mice (*B6.129P2-Hbb-b1^{tm1Unc} Hbb-b2^{tm1Unc}*/*J* mice on 000664 C57BL/6J background) upon RAP-536 or rapamycin dosing and exceed wild-type levels upon RAP-536 or rapamycin co-dosing.

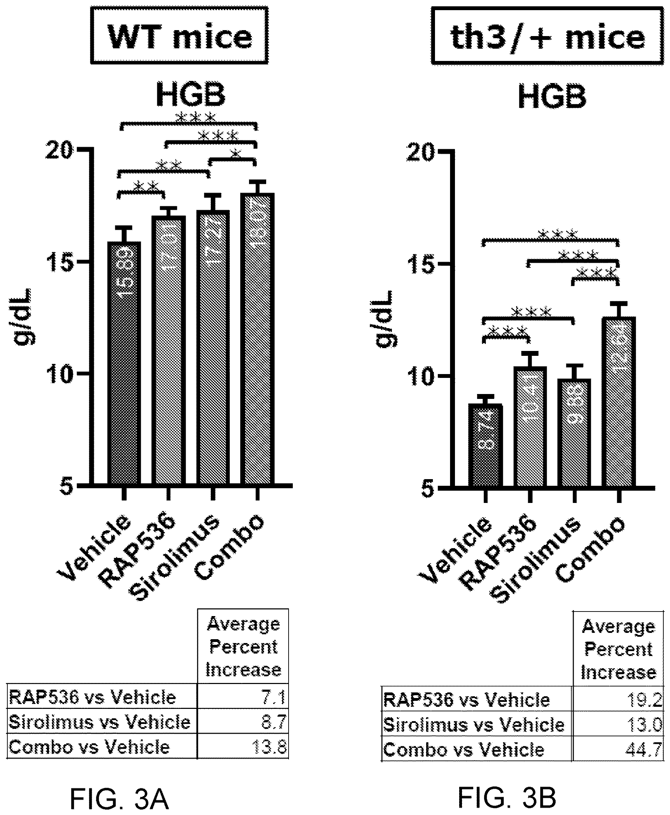

**FIGs. 3A** and **3B** demonstrate that rapamycin and RAP-536 co-dosing increased the hemoglobin (HGB) levels in blood more than a single agent dosing. The percentage increase in HGB levels was similar to the percentage increase in RBC levels. HGB levels in th3/+ mice is about half of the HGB levels in wild-type mice. Co-dosing increased HGB levels in th3/+ mice by more than 40%.

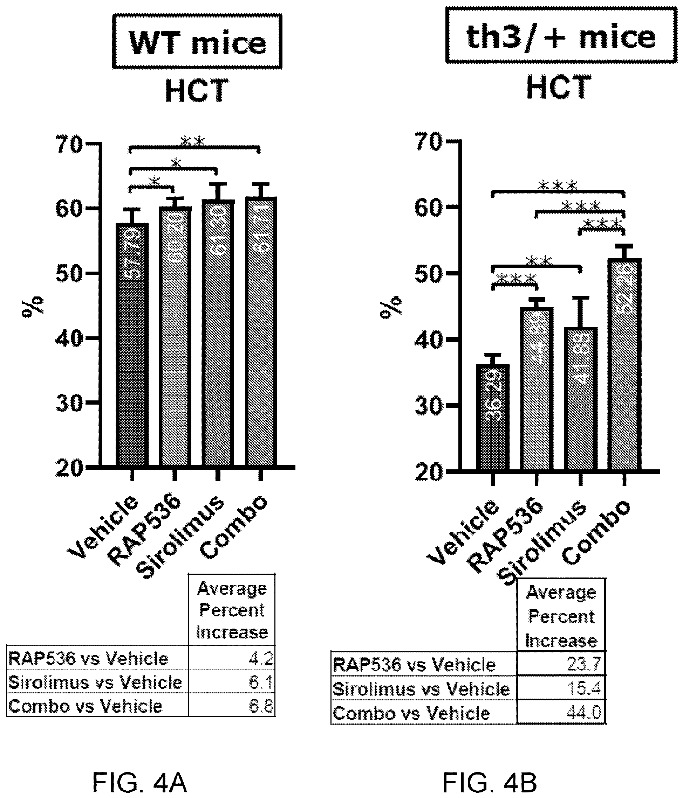

**FIGs. 4A** and **4B** demonstrate that rapamycin and RAP-536 co-dosing increased HCT levels in blood more than single agent dosing. HCT increase in wild-type mice is limited upon dosing. The HCT percentage increase in th3/+ mice correlates with RBC and HGB increase. Co-dosing was very effective in increasing RBC, HGB, and HCT especially in th3/+ mice.

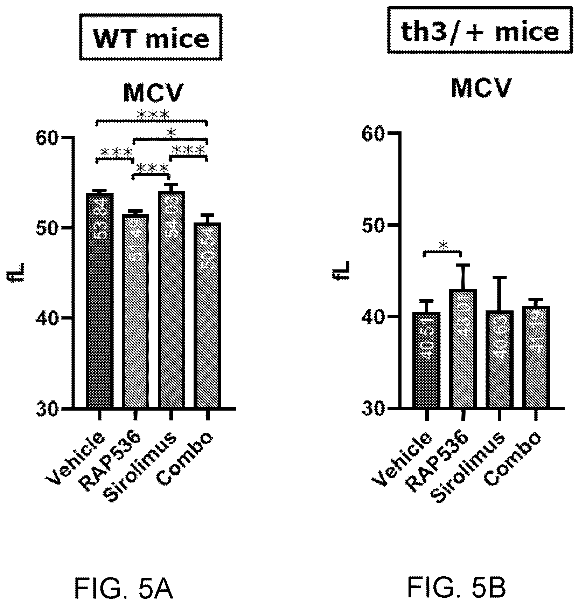

**FIGs. 5A** and **5B** demonstrate that rapamycin and RAP-536 co-dosing changed the RBC cell size (MCV) in wild-type mice. RAP-536 dosing reduced MCV in wild-type mice but increased the MCV levels in th3/+ mice. The co-dosing of RAP-536 and rapamycin reduced MCV in wild-type mice.

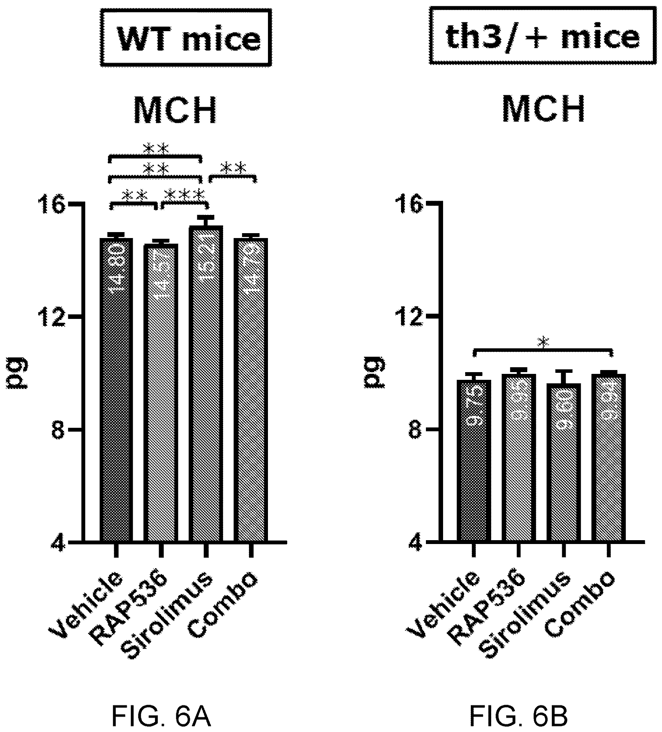

**FIGs. 6A** and **6B** show the mean corpuscular hemoglobin (MCH) levels upon rapamycin and RAP-536 co-dosing. In wild-type mice, RAP-536 dosing slightly reduces MCH levels. MCH levels are significantly lower in th3/+ mice compared to wild-type mice. The levels of HGB and RBC in th3/+ mice are lower than the levels of HGB and RBC wild-type mice, explaining why there is less total amount of HGB/cell (MCH) in th3/+ mice. MCH levels were higher upon rapamycin dosing but were lower upon RAP-536 dosing in wild-type mice, which indicates different mechanisms of action and therefore potential for additive/synergistic effect for red blood cell production.

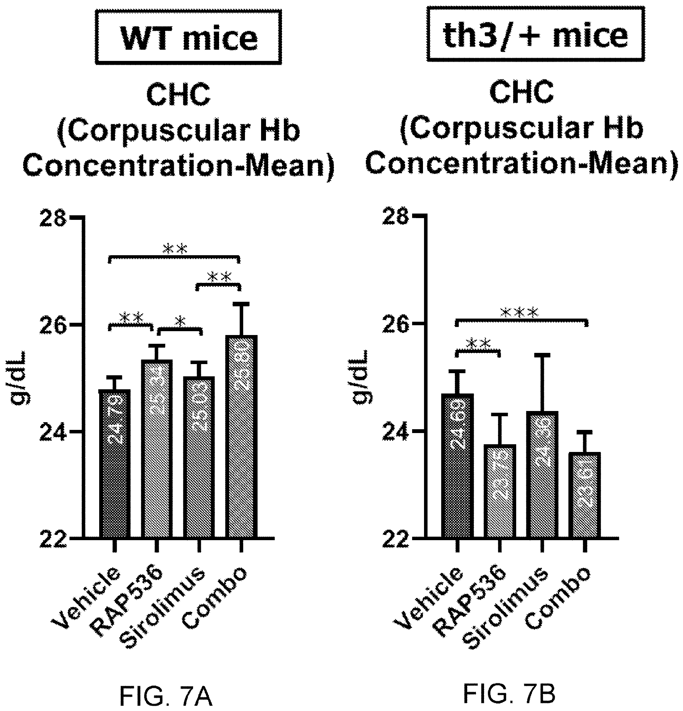

**FIGs. 7A** and **7B** demonstrate that rapamycin and RAP-536 co-dosing increased the corpuscular HGB concentration (CHC) in wild-type mice but decreased CHC in th3/+ mice.

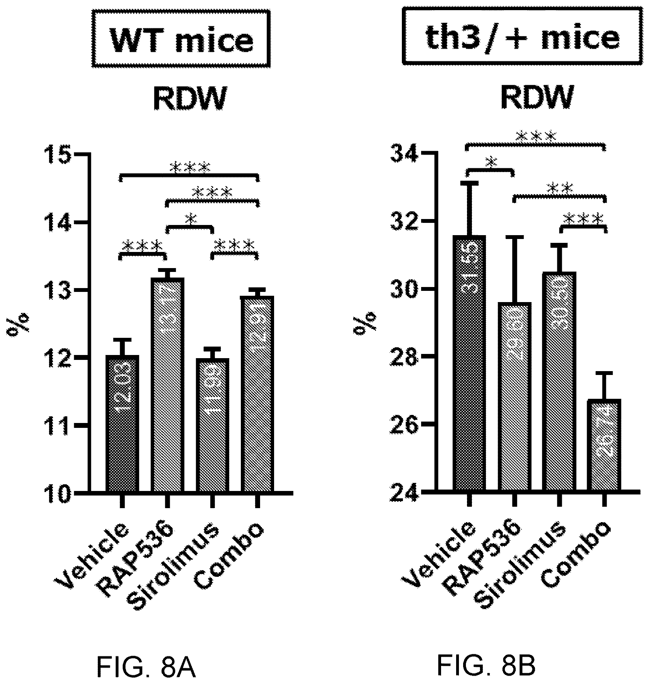

**FIGs. 8A** and **8B** demonstrate that rapamycin and RAP-536 co-dosing decreased the red blood cell distribution width (RDW) in th3/+ mice. RDW increased upon RAP-536 dosing or co-dosing of RAP-536 and rapamycin in wild-type mice. Note that RDW was higher in th3/+ compared to wild-type mice. In th3/+ mice, RDW was decreased in correlation with improvement of anemia by RAP-536 dosing and co-dosing of RAP-536 and rapamycin.

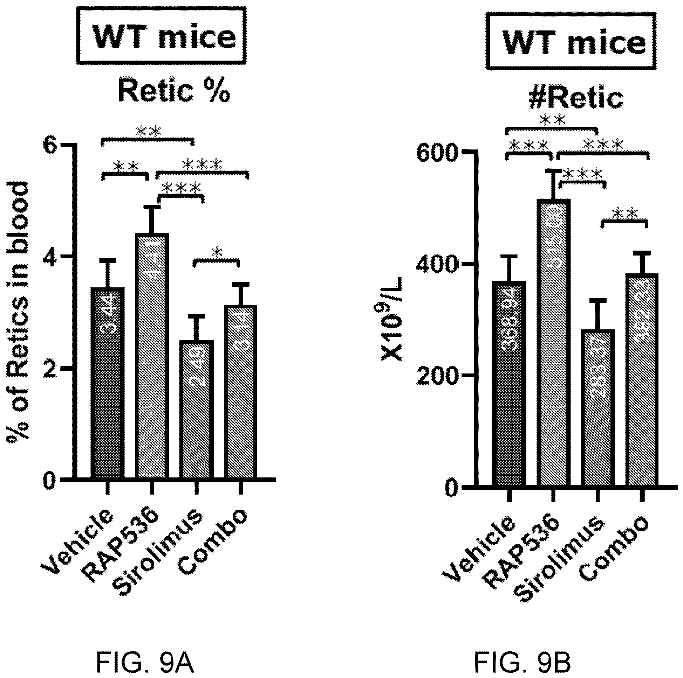

**FIGs. 9A** and **9B** demonstrate that RAP-536 dosing increased the reticulocyte levels in blood and rapamycin dosing reduced the reticulocyte levels in blood in wild-type mice, suggesting that the mechanism of action of RAP-536 and the mechanism of action of rapamycin on red blood cell production are different. Reticulocyte percentage and absolute numbers changed in the same way in blood in wild-type mice upon dosing. The co-dosing of both normalized reticulocyte levels.

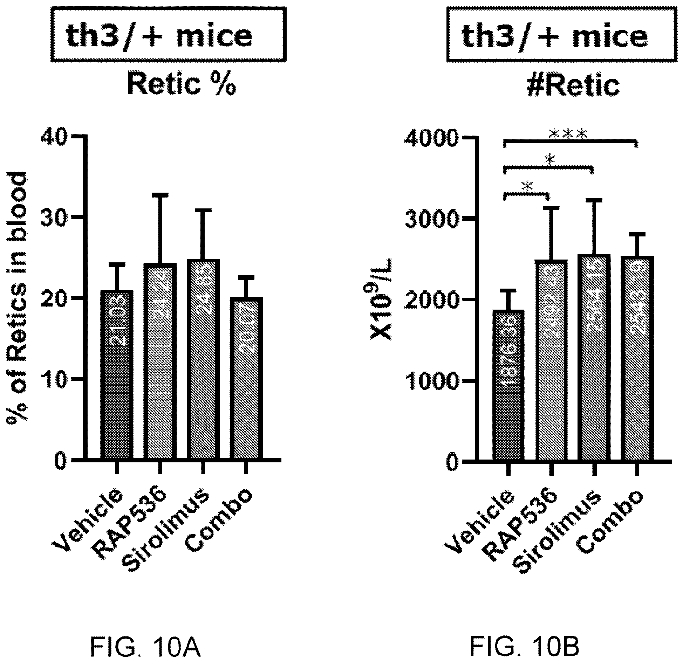

**FIGs. 10A** and **10B** demonstrate that rapamycin and RAP-536 co-dosing increased reticulocyte numbers but not the reticulocyte percentage in th3/+ mice. Reticulocyte percentage and absolute numbers were higher in th3/+ mice as compared to in wild-type mice. The reticulocyte absolute numbers in th3/+ mice increased significantly upon rapamycin dosing, RAP-536 dosing, and the co-dosing of both. The fact that the reticulocyte absolute numbers in th3/+ mice increased after both RAP-536 dosing and rapamycin dosing suggests that reticulocyte survival is enhanced in th3/+ mice.

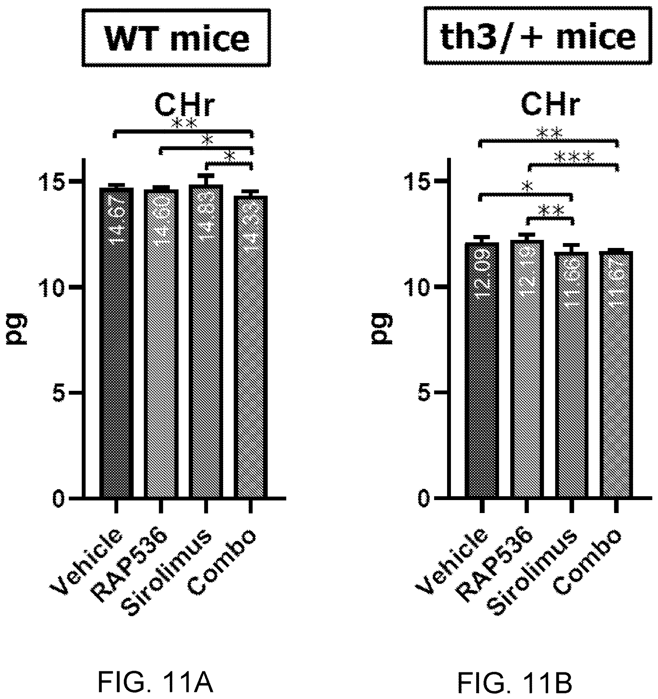

**FIGs. 11A** and **11B** demonstrate that rapamycin and RAP-536 co-dosing slightly reduced reticulocyte hemoglobin content (CHr - reticulocyte hemoglobin content) in wild-type mice and in th3/+ mice.

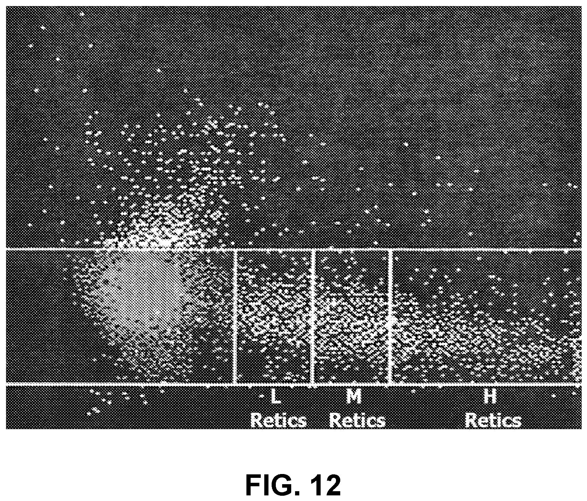

**FIG. 12** shows an exemplary reticulocyte composition in blood analyzed by an automated blood cell analyzer in the reticulocyte channel. H is High RNA levels, immature reticulocytes; M is Medium RNA levels; and L is Low RNA levels, mature reticulocytes. (*See e.g.,* Mori et al., 2003, Nephrology Dialysis Transplantation, 18(suppl 4):416).

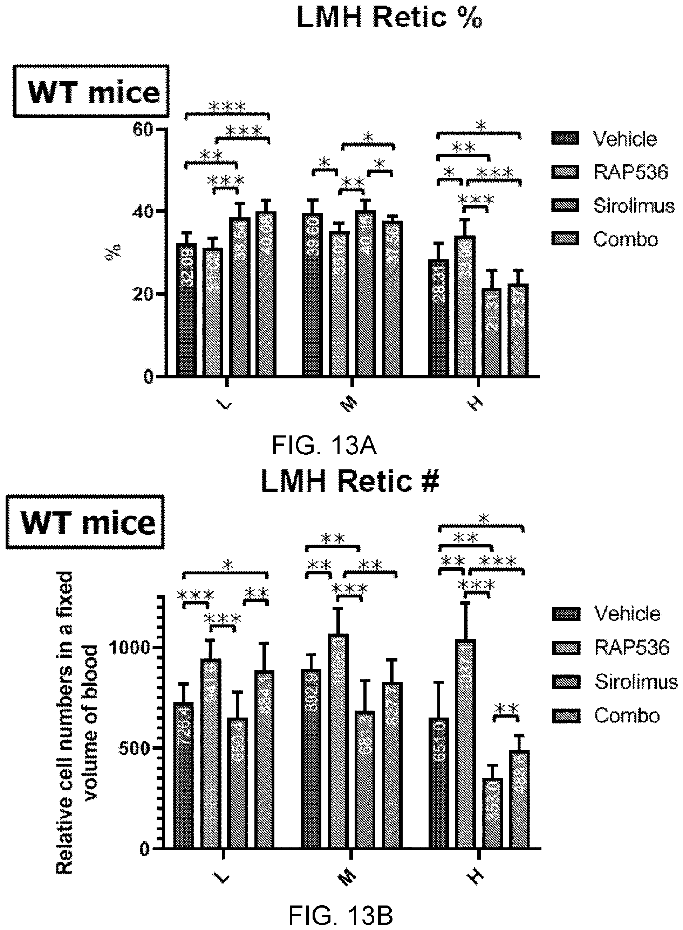

**FIGs. 13A** and **13B** show the reticulocyte composition in blood changed upon rapamycin and RAP-536 co-dosing in wild-type mice. Specifically, the H reticulocyte percentage increased and the M reticulocyte percentage decreased upon RAP-536 dosing. Overall reticulocyte numbers increased upon RAP-536 dosing. The H reticulocyte percentage decreased and the L and M reticulocyte percentages increased upon rapamycin dosing. Overall reticulocyte levels also decreased.

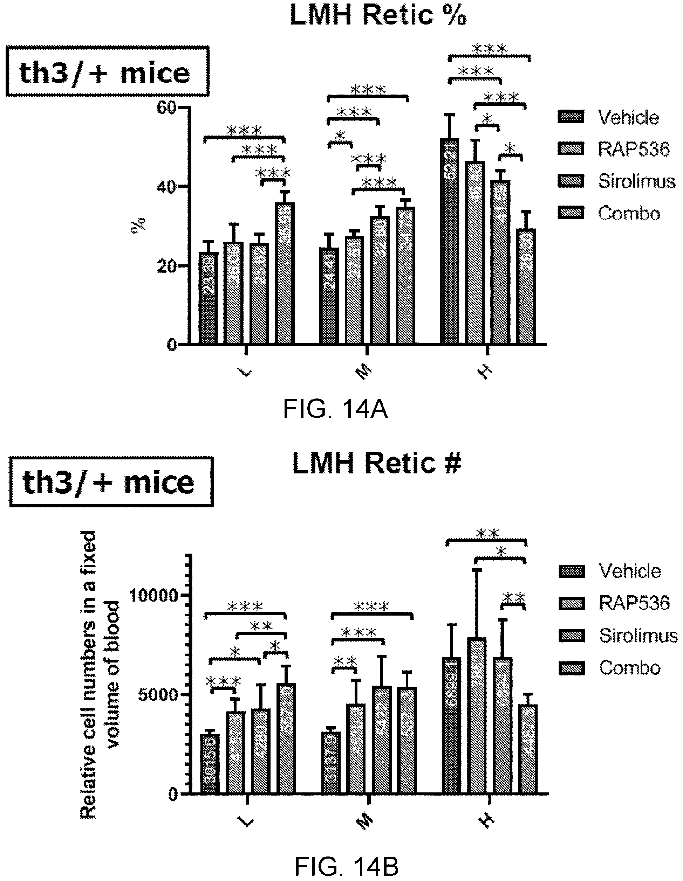

**FIGs. 14A** and **14B** depict that reticulocyte composition in blood changed upon rapamycin and RAP-536 co-dosing in th3/+ mice. Specifically, the H reticulocyte percentage decreased upon rapamycin and RAP-536 co-dosing in th3/+ mice. Rapamycin and RAP-536 co-dosing did not reduce the overall reticulocyte numbers in th3/+ mice. Yet, the H reticulocyte percentage decreased significantly, suggesting that reticulocyte maturation/survival was probably enhanced.

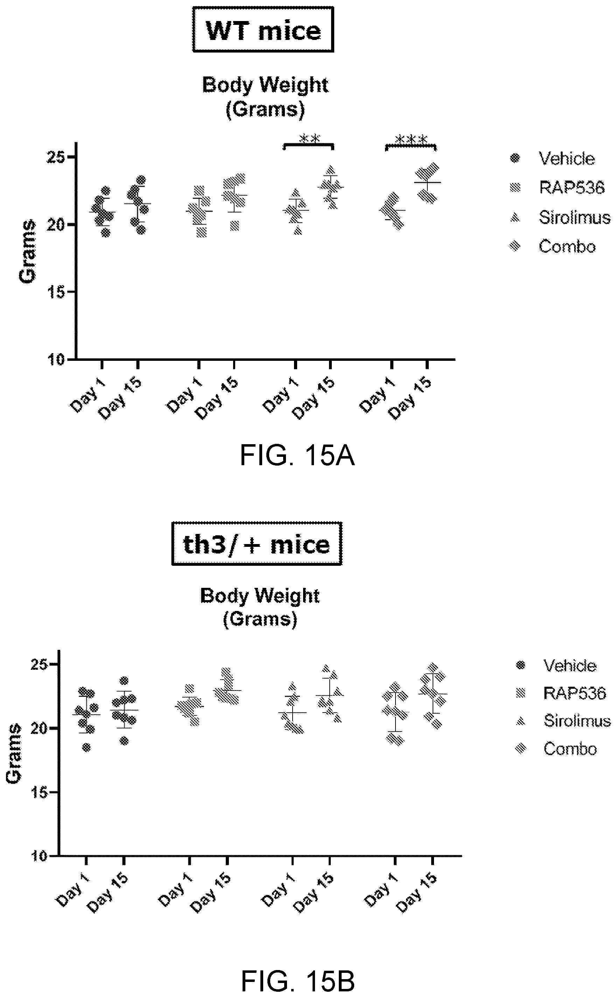

**FIGs. 15A** and **15B** demonstrate the changes to the body weight of wild-type mice and th3/+ mice.

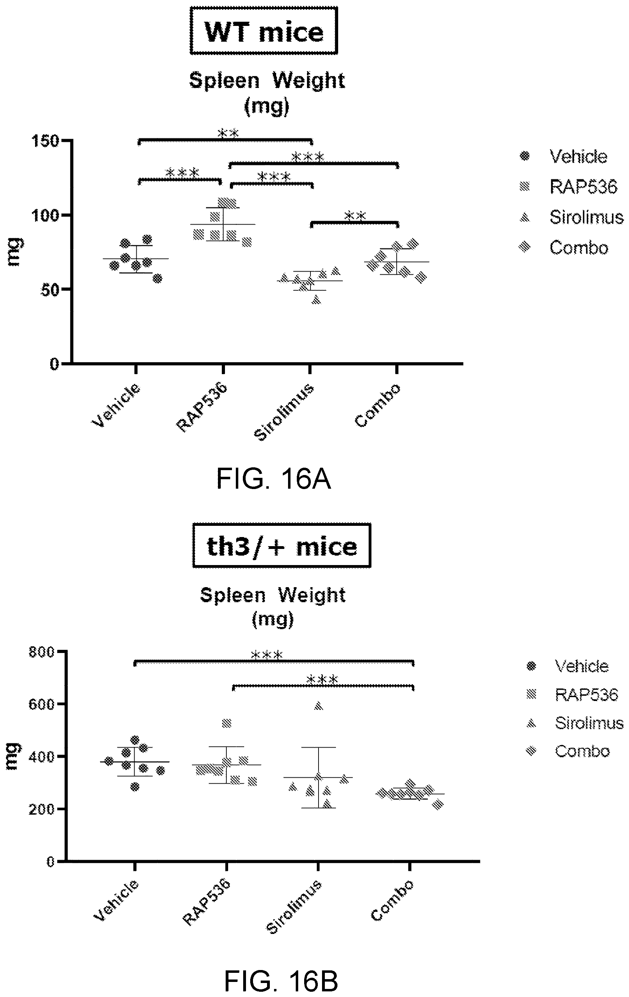

**FIGs. 16A** and **16B** demonstrate the changes to spleen size in wild-type mice and th3/+ mice. RAP-536 dosing slightly increased and rapamycin dosing reduced the spleen size in wild-type mice. The co-dosing of rapamycin and RAP-536 normalized spleen size in wild-type mice. In th3/+ mice, the co-dosing of rapamycin and RAP-536 not only reduced anemia but also reduced spleen size.

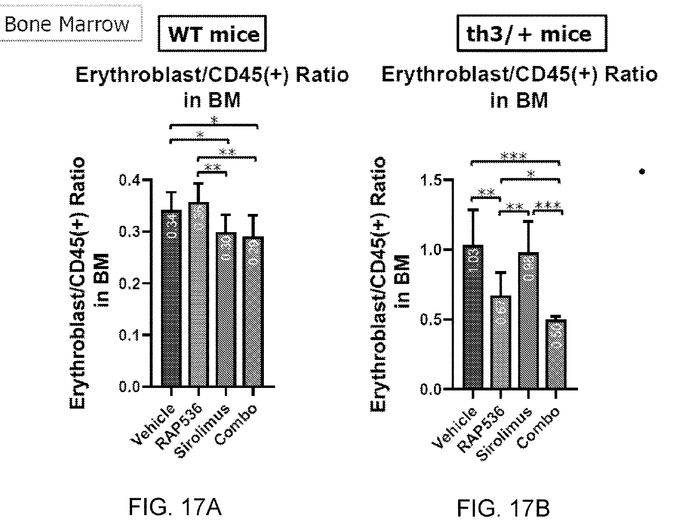

**FIGs. 17A** and **17B** demonstrate that the erythroblast frequency in the bone marrow decreased upon rapamycin and RAP-536 co-dosing in th3/+ mice. Specifically, the erythroblast /CD45(+) ratio in bone marrow was high in th3/+ mice and were normalized by the co-dosing of rapamycin and RAP-536.

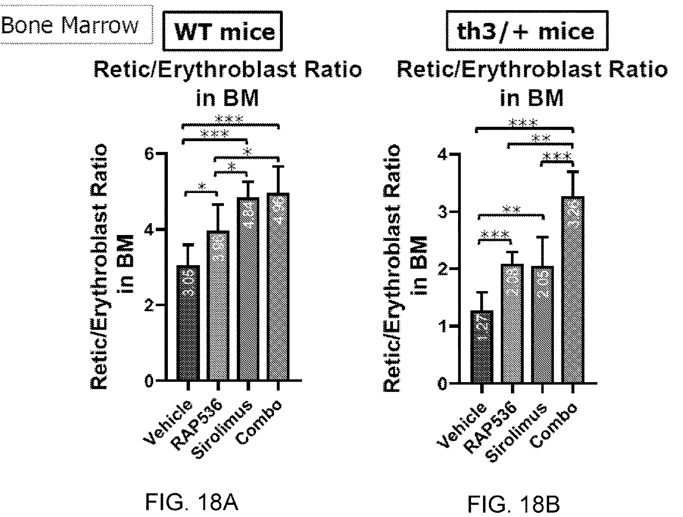

**FIGs. 18A** and **18B** demonstrate that more reticulocytes were produced and/or retained in the bone marrow in wild-type mice and th3/+ mice upon the co-dosing of rapamycin and RAP-536 than the dosing of either rapamycin or RAP-536 alone. The ratio of reticulocytes to erythroblast increased in wild-type mice and in th3/+ mice upon RAP-536 dosing and the co-dosing of rapamycin and RAP-536.

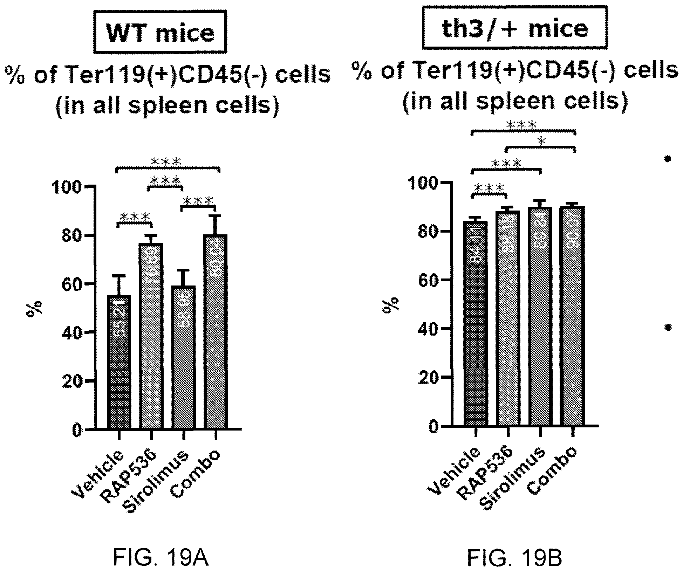

**FIGs. 19A** and **19B** demonstrate that the RAP-536 dosing and the co-dosing of rapamycin and RAP-536 increased the Ter119(+) cell frequency in spleen of both wild-type mice and th3/+ mice.

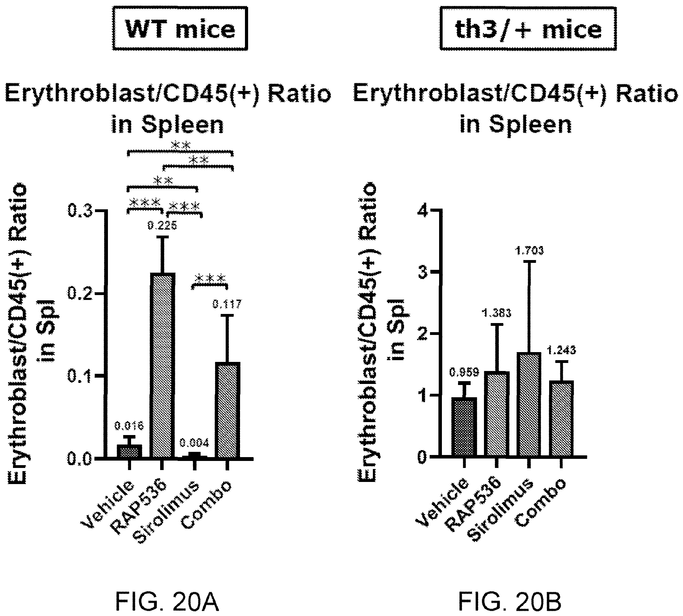

**FIGs. 20A** and **20B** demonstrate changes to the erythroblast/CD45(+) ratio in spleen in wild-type mice and th3/+ mice upon rapamycin and RAP-536 co-dosing. In wild-type mice, RAP-536 dosing increased erythroblast/CD45(+) ratio in spleen, and rapamycin decreased erythroblast/CD45(+) ratio in spleen.

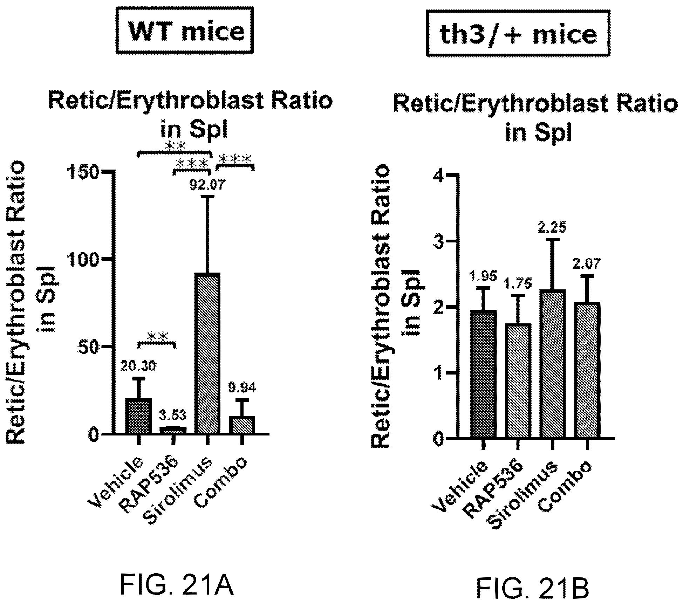

**FIGs. 21A** and **21B** demonstrate changes to the reticulocyte/erythroblast ratio in spleen in wild-type mice and th3/+ mice. The rapamycin and RAP-536 co-dosing had milder direct effects on erythropoiesis in spleen in th3/+ mice than in wild-type mice. The reticulocyte/erythroblast ratio in wild-type mice spleen was higher than in th3/+ mice spleen.

### 6. DETAILED DESCRIPTION

### 6.1 Abbreviations and Terminology

"β⁰" refers to an allele associated with a lack of beta globin subunit synthesis.

"β⁺" refers to an allele associated with reduced beta globin subunit synthesis.

As used herein, the term "about" when used in conjunction with a number refers to any number within 1%, 2%, 3%, 4%, 5%, 6%, 7%, 8%, 9%, 10%, 11%, 12%, 13%, 14%, or 15% of the referenced number. In certain embodiments, the term "about" encompasses the exact number recited.

As used herein, "ActRII" refers to activin receptor type II. As used herein, "ActRIIA" refers to activin receptor type IIA.

As used herein, "ActRIIB" refers to activin receptor type IIB. *See,* for example, Attisano et al., 1992, Cell 68: 97-108. GenBank^{™} accession number NM_001106.3 provides an exemplary human ActRIIB nucleic acid sequence. GenBank^{™} accession number NP_001097.2 provides an exemplary human ActRIIB amino acid sequence.

As used herein, "BL" refers to baseline.

As used herein, "CHC" refers to corpuscular hemoglobin concentration-mean.

As used herein, "ECD" refers to extracellular domain.

As used herein, "EPO" refers to erythropoietin.

As used herein, "sEPO" refers to serum erythropoietin.

As used herein, "ESA" refers to erythropoiesis-stimulating agent.

As used herein, "G-CSF" refers to granulocyte colony-stimulating factor.

As used herein, "GM-CSG" refers to granulocyte macrophage colony-stimulating factor.

As used herein, "Hb" and "HGB" both refer to hemoglobin.

As used herein, "HI-E" refers to erythroid hematological improvement. In certain embodiments, the HI-E is as defined by IWG. In certain embodiments, the HI-E is as defined by the modified 2006 IWG. In certain embodiments, the HI-E for a low transfusion burden patient is an increase in hemoglobin concentration in the patient of at least 1.5 g/dL for at least 8 weeks. In certain embodiments, the HI-E for a high transfusion burden patient is an at least 4 unit reduction in RBC transfusion over 8 weeks.

As used herein, "HTB" refers to high transfusion burden. In certain embodiments, a HTB subject receives greater than or equal to 4 RBC units over the course of 8 weeks.

As used herein, "IgG" refers to immunoglobulin G.

As used herein, "IPSS-R" refers to International Prognostic Scoring System - Revised. *See* Section 6.5.

As used herein, "IWG" refers to International Working Group. *See, e.g.,* Cheson et al. Blood. 2000 96:3671-3674. In certain embodiments, IWG refers to the modified 2006 criteria. *See, e.g.,* Cheson et al., 2006, Blood 108(2):419-425.

As used herein, "LTB" refers to low transfusion burden. In certain embodiments, a LTB subject receives less than 4 RBC units over the course of 8 weeks.

As used herein, "ITT" refers to intent-to-treat.

As used herein, "MedDRA" refers to Medical Dictionary for Regulatory Activities.

As used herein, "MCV" refers to mean corpuscular volume.

As used herein, "MDS" refers to myelodysplastic syndromes.

As used herein, "mTOR" refers to the mammalian target of rapamycin.

As used herein, "PD" refers to pharmacodynamic.

As used herein, "PK" refers to pharmacokinetic.

As used herein, "RA" refers to refractory anemia.

As used herein, "RAEB" refers to refractory anemia with an excess of blasts.

As used herein, "rapamycin" refers to Sirolimus. "Rapamycin" and "Sirolimus" are used interchangeably herein.

As used herein, "RBC" refers to red blood cells.

As used herein, "RBC-TI" refers to red blood cell transfusion independent.

As used herein, "RCMD-RS" refers to refractory cytopenia with multilineage dysplasia with ring sideroblasts.

As used herein, "RDW" refers to red blood cell distribution width.

As used herein, "RS" refers to ring sideroblast.

As used herein, "SC" refers to subcutaneous.

As used herein, "SF3B1" refers to splicing factor 3B1. GenBank^{™} accession numbers NM_012433.3, NM_001005523.2, and NM_001308824.1 provide exemplary nucleic acid sequences for human SF3B1. GenBank^{™} accession numbers NP_001295753.1, NP_001005526.1, and NP_036565.2 provide exemplary amino acid sequences for human SF3B1.

As used herein, "WPSS" refers to World Health Organization (WHO) Prognostic Scoring System.

As used herein, "luspatercept" refers to a product resulting from expression from an opening reading frame with the nucleotide sequence of SEQ ID NO:34 or a degenerate version of SEQ ID NO:34 that encodes SEQ ID NO:11, and subsequent protein purification procedures.

In certain embodiments, "treat", "treating", treatment" and the like refer to an action (such as administering an ActRIIB ligand trap and an mTOR inhibitor) to eliminate, reduce, suppress, mitigate, or ameliorate, either temporarily or permanently, at least one of the underlying causes of a disease, disorder, or condition afflicting a subject, or at least one of the symptoms associated with a disease, disorder, condition afflicting a subject.

In certain embodiments, "treat," "treatment," or "treating," in the context of anemia, includes amelioration of at least one symptom of anemia. Non-limiting examples of symptoms of anemia include fatigue, loss of energy, rapid heartbeat, shortness of breath, headaches, difficulty concentrating, dizziness, pale skin, leg cramps, and insomnia.

In certain embodiments, "treat," "treatment," or "treating," in the context of beta-thalassemia, includes amelioration of at least one symptom of beta-thalassemia. Nonlimiting examples of symptoms of beta-thalassemia include defective red blood cell production in the marrow, ineffective erythropoiesis, deficient hemoglobin levels, multiple organ dysfunction, iron overload, paleness, fatigue, jaundice, and splenomegaly.

In certain embodiments, "treat," "treatment," or "treating," in the context of MDS, includes amelioration of at least one symptom of MDS. Nonlimiting examples of symptoms of MDS include fatigue, weakness, easy bruising or bleeding, fever, bone pain, shortness of breath, and frequent infections.

In certain embodiments, "treat," "treatment," or "treating," in the context of MDS, includes amelioration of at least one symptom of MDS. Nonlimiting examples of symptoms of MDS include fatigue, frequent infections, bruising or bleeding easily, abdominal discomfort from a swollen spleen, and loss of appetite.

### 6.2 Overview

Provided herein are methods of treating anemia or for enhancing late stage erythropoiesis in a subject comprising: administering to the subject an ActRIIB ligand trap (*e.g.,* Luspatercept, a product resulting from expression from an opening reading frame with the nucleotide sequence of SEQ ID NO:34 or a degenerate version of SEQ ID NO:34 that encodes SEQ ID NO:11, and subsequent protein purification procedures; *see* Section 6.7), and administering to the subject an mTOR inhibitor (*e.g.* rapamycin; *see* Section 6.8). Dosing regimens are described in Section 6.4. Patient populations that can be treated using the methods provided herein are described in Section 6.5. Clinical outcomes of the methods provided herein are described in Section 6.3.

### 6.3 Methods of Treatment

Provided herein are methods of treating anemia or for enhancing late stage erythropoiesis in a subject comprising: administering to the subject an ActRIIB ligand trap (such as the ActRIIB ligand traps described in Section 6.7); and administering to the subject an mTOR inhibitor (such as the mTOR inhibitors described in Section 6.8).

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases red blood cell (RBC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than RBC levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases HCT levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HCT levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases hemoglobin (HGB) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces reticulocyte hemoglobin levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces H reticulocyte percentage in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than the H reticulocyte percentage in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases mean platelet volume (MPV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than MPV levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases the percentage of Ter119(+) late stage erythroid cells in the bone marrow in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than the percentage of Ter119(+) late stage erythroid cells in the bone marrow in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces erythroblast frequency in the bone marrow in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than the erythroblast frequency in the bone marrow in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia A method for treating anemia or for enhancing late stage erythropoiesis in a subject in need thereof, comprising: administering to the subject an ActRIIB ligand trap; and administering to the subject an mTOR inhibitor, wherein the levels of reticulocytes in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the levels of white blood cells in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, the anemia is an anemia associated with ineffective erythropoiesis, thalassemia, alpha-thalassemia, beta-thalassemia, myelodysplastic syndromes (MDS), or non-proliferative chronic myelomonocytic leukemia (CMML).

In certain embodiments, the mTOR inhibitor is rapamycin. In certain embodiments, the mTOR inhibitor is a pharmaceutically acceptable salt or hydrate of rapamycin.

In certain embodiments, the ActRIIB ligand trap is a polypeptide comprising an amino acid sequence that is at least: (a) 90% identical to SEQ ID NO:3; (b) 95% identical to SEQ ID NO:3; (c) 98% identical to SEQ ID NO:3; (d) SEQ ID NO:3; (e) 90% identical to SEQ ID NO:6; (f) 95% identical to SEQ ID NO:6; (g) 98% identical to SEQ ID NO:6; (h) SEQ ID NO:6; (i) 90% identical to SEQ ID NO:7; (j) 95% identical to SEQ ID NO:7; (k) 98% identical to SEQ ID NO:7; (l) SEQ ID NO:7; (m) 90% identical to SEQ ID NO:11; (n) 95% identical to SEQ ID NO:11; (o) 98% identical to SEQ ID NO:11; or (p) SEQ ID NO:11. In certain embodiments, the ActRIIB ligand trap is a product resulting from expression from an opening reading frame with the nucleotide sequence of SEQ ID NO:34 or a degenerate version of SEQ ID NO:34 that encodes SEQ ID NO:11. In certain embodiments, the ActRIIB ligand trap is a humanized fusion-protein consisting of the extracellular domain of ActRIIB and the human IgG1 Fc domain.

In certain embodiments, the method increases hemoglobin (HGB) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject prior to said treating.

In certain embodiments, the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HCT levels in the subject prior to said treating.

In certain embodiments, the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than MCV levels in the subject prior to said treating.

In certain embodiments, the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than CHC levels in the subject prior to said treating.

In certain embodiments, the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than the RDW levels in the subject prior to said treating.

In certain embodiments, the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in the subject prior to said treating.

In certain embodiments, the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in a reference population. In a particular embodiment, the reference population consists of 1, 5, 10, 25, 50, 75, 100, 200, 250, 300, 400, 500, or 1000 individuals. In a particular embodiment, the reference population consists of healthy individuals. In a particular embodiment, the reference population consists of people of the same age, weight, and/or gender as the subject.

In certain embodiments, levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in the subject prior to said treating.

In certain embodiments, the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in a reference population. In a particular embodiment, the reference population consists of 1, 5, 10, 25, 50, 75, 100, 200, 250, 300, 400, 500, or 1000 individuals. In a particular embodiment, the reference population consists of healthy individuals. In a particular embodiment, the reference population consists of people of the same age, weight, and/or gender as the subject.

In certain embodiments, the mTOR inhibitor is administered before or concurrently with the administration of ActRIIB ligand trap. In certain embodiments, the subject has been previously treated with the mTOR inhibitor prior the administration of ActRIIB ligand trap. In certain embodiments, the subject has been previously treated with the ActRIIB ligand trap prior the administration of mTOR inhibitor.

In certain embodiments, the subject is red blood cell non-transfusion-dependent. In certain embodiments, the subject is red blood cell transfusion-dependent.

In certain embodiment, the ActRIIB ligand trap is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg. In certain embodiments, the ActRIIB ligand trap is administered at a dose of 0.6 mg/kg, 0.8 mg/kg, 1 mg/kg, 1.33 mg/kg, or 1.75 mg/kg. In certain embodiments, the ActRIIB ligand trap is administered to the subject once every 21 days. In certain embodiments, the ActRIIB ligand trap is administered twice per week. In certain embodiments, the ActRIIB ligand trap is administered once per week. In certain embodiments, the ActRIIB ligand trap is administered to the subject subcutaneously. In certain embodiments, the subject is a human.

In certain embodiments, the mTOR inhibitor is administered orally. In certain embodiments, the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 15 mg/kg, 20 mg/kg, 25 mg/kg, 30 mg/kg, or 35 mg/kg. In certain embodiments, the mTOR inhibitor is administered daily.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases hemoglobin (HGB) levels in a reference population to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In certain embodiments, provide herein are methods for treating anemia or for enhancing late stage erythropoiesis in a subject in need thereof, comprising: administering to the subject an ActRIIB ligand trap; and administering to the subject an mTOR inhibitor, wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than HGB levels in a reference population who received either an ActRIIB ligand trap or an mTOR inhibitor, but not together. In certain embodiments, the subject is a human. In certain embodiments, the reference population consists of 1, 5, 10, 25, 50, 75, 100, 200, 250, 300, 400, 500, or 1000 individuals. In certain embodiments, the reference population consists of people of the same age, weight, and/or gender as the subject. In certain embodiments, the reference population consists of individuals with anemia. In certain embodiments, the reference population consists of healthy individuals.

In another aspect, provided herein are methods of treating anemia or for enhancing late stage erythropoiesis in a subject comprising: administering to the subject an ActRIIB ligand trap (e.g., Luspatercept, a product resulting from expression from an opening reading frame with the nucleotide sequence of SEQ ID NO:34 or a degenerate version of SEQ ID NO:34 that encodes SEQ ID NO:11; *see* Section 6.7), and administering to the subject an mTOR inhibitor (*e*.*g*. rapamycin; *see* Section 6.8).

In certain embodiments, the ActRIIB signaling inhibitor is an ActRIIB ligand trap (e.g., Luspatercept, a product resulting from expression from an opening reading frame with the nucleotide sequence of SEQ ID NO:34 or a degenerate version of SEQ ID NO:34 that encodes SEQ ID NO:11; *see* Section 6.7). In certain embodiments, the ActRIIB signaling inhibitor is administered to the subject in combination with a second pharmaceutically active agent or therapy as described in Section 6.3.1.

In certain embodiments, the mTOR inhibitor is, but not limited to, an mTOR inhibitor described in Section 6.8. In certain embodiments, the mTOR inhibitor is rapamycin. In certain embodiments, the mTOR inhibitor (*e.g.* rapamycin; *see* Section 6.8) is administered to the subject in combination with a second pharmaceutically active agent or therapy as described in Section 6.3.1.

### 6.3.1 Combination Therapy

In certain embodiments, the pharmaceutical composition comprising the mTOR inhibitor is administered before or concurrently with the administration of the ActRIIB ligand trap.

In certain embodiments, the pharmaceutical composition comprising the mTOR inhibitor administered after the administration of the ActRIIB ligand trap.

In certain embodiments, the subject has been previously treated with the mTOR inhibitor prior to the administration of the ActRIIB ligand trap.

In certain embodiments, the subject has been previously treated with an ActRIIB ligand trap prior to the administration of the mTOR inhibitor.

In certain embodiments, the mTOR inhibitor is administered concomitantly with the ActRIIB ligand trap.

### 6.4 Dose Regimen and Dose Adjustment

Provided herein are methods for treating anemia or for enhancing late stage erythropoiesis in a subject comprising administering to the subject an ActRIIB ligand trap and administering to the subject an mTOR inhibitor. In certain embodiments, the method further comprising: (a) taking a measurement of hemoglobin concentration, hematocrit, MCH, MCV, MCV, CHC, RDW, reticulocytes, spleen size, or spleen volume; and (b) administering a subsequent dose of the ActRIIB ligand trap based on the difference between the measurement of hemoglobin concentration, hematocrit, MCH, MCV, MCV, CHC, RDW, reticulocytes, spleen size, or spleen volume in a reference population.

In certain embodiments, the method further comprising: (a) taking a measurement of hemoglobin concentration, hematocrit, MCH, MCV, MCV, CHC, RDW, reticulocytes, spleen size, or spleen volume; and (b) administering a subsequent dose of a pharmaceutical composition comprising the mTOR inhibitor or pharmaceutically acceptable salt or hydrate thereof, based on the difference between the measurement of hemoglobin concentration hematocrit, MCH, MCV, MCV, CHC, RDW, reticulocytes, spleen size, or spleen volume in step (a) and in a reference population.

In certain embodiments, the spleen size or spleen volume is measured by magnetic resonance imaging (MRI). In certain embodiments, the spleen size or spleen volume is measured by computed tomography (CT). In certain embodiments, the spleen size or spleen volume is measured by palpation.

In yet another aspect, provided herein are methods for treating anemia, ineffective erythropoiesis, or beta-thalassemia, or for enhancing late stage erythropoiesis in a subject in need thereof, comprising: (a) taking a first measurement of hemoglobin concentration, hematocrit, MCH, MCV, MCV, CHC, RDW, reticulocytes, spleen size, or spleen volume in the subject; (b) administering to the subject an initial dose of an ActRIIB ligand trap; (c) administering to the subject an initial dose of a pharmaceutical composition comprising an mTOR inhibitor, or a pharmaceutically acceptable salt or hydrate thereof; (d) after a first period of time taking a second measurement of hemoglobin concentration, hematocrit, MCH, MCV, MCV, CHC, RDW, reticulocytes, spleen size, or spleen volume in the subject; and (e) administering a subsequent dose of the pharmaceutical composition comprising the mTOR inhibitor, or pharmaceutically acceptable salt or hydrate thereof, based on the difference between the second measurement of hemoglobin concentration, hematocrit, MCH, MCV, MCV, CHC, RDW, reticulocytes, spleen size, or spleen volume and the first measurement of hemoglobin concentration, hematocrit, MCH, MCV, MCV, CHC, RDW, reticulocytes, spleen size, or spleen volume.

In yet another aspect, provided herein are methods for treating anemia, ineffective erythropoiesis, or beta-thalassemia, or for enhancing late stage erythropoiesis in a subject in need thereof, comprising: (a) taking a first measurement of hemoglobin concentration, hematocrit, MCH, MCV, MCV, CHC, RDW, reticulocytes, spleen size, or spleen volume in the subject; (b) administering to the subject an initial dose of an ActRIIB ligand trap; (c) administering to the subject an mTOR inhibitor, or a pharmaceutically acceptable salt or hydrate thereof; (d) after a first period of time taking a second measurement of hemoglobin concentration, hematocrit, MCH, MCV, MCV, CHC, RDW, reticulocytes, spleen size, or spleen volume in the subject; and (e) administering a subsequent dose of the ActRIIB ligand trap based on the difference between the second measurement of hemoglobin concentration, hematocrit, MCH, MCV, MCV, CHC, RDW, reticulocytes, spleen size, or spleen volume and the first measurement of hemoglobin concentration, hematocrit, MCH, MCV, MCV, CHC, RDW, reticulocytes, spleen size, or spleen volume.

In certain embodiments, the first measurement of hemoglobin concentration, hematocrit, MCH, MCV, MCV, CHC, RDW, reticulocytes, spleen size, or spleen volume is taken prior to administering the mTOR inhibitor.

In certain embodiments, the first measurement of hemoglobin concentration, hematocrit, MCH, MCV, MCV, CHC, RDW, reticulocytes, spleen size, or spleen volume is taken prior to administering to the subject of the ActRIIB ligand trap.

In certain embodiments, the second measurement of hemoglobin concentration, hematocrit, spleen size, or spleen volume is taken approximately 1 week, 2 weeks, 3 weeks, 1 month, 2 months, 3 months, 4 months, 5 months, 6 months, 7 months, 8 months, 9 months, 10 months, 11 months, or 12 months after the administration of the initial dose of the mTOR inhibitor.In certain embodiments, the second measurement of hemoglobin concentration, hematocrit, spleen size, or spleen volume is taken approximately 1 week, 2 weeks, 3 weeks, 1 month, 2 months, 3 months, 4 months, 5 months, 6 months, 7 months, 8 months, 9 months, 10 months, 11 months, or 12 months after the administration of the initial dose of the ActRIIB ligand trap.

In certain embodiments, the first period of time is about 1 day, 2 days, 3 days, 4 days, 5 days, 6 days, 1 week, 2 weeks, 3 weeks, 4 weeks, 5 weeks, 6 weeks, 7 weeks, 8 weeks, 9 weeks, 10 weeks, 11 weeks, or 12 weeks.

In certain embodiments, the subsequent dose of the mTOR inhibitor is the same as the initial dose of the mTOR inhibitor.

In certain embodiments, the subsequent dose of the ActRIIB ligand trap is the same as the initial dose.

In certain embodiments, the pharmaceutical composition comprising the mTOR inhibitor or pharmaceutically acceptable salt or hydrate thereof is administered before or concurrently with the administration of the ActRIIB ligand trap.

In certain embodiments, the subject has been previously treated with any mTOR inhibitor prior the administration of the administration of the ActRIIB ligand trap. In certain embodiments, the subject has been previously treated with the mTOR inhibitor prior the administration of the administration of the ActRIIB ligand trap.

In certain embodiments, the elevated levels of hemoglobin concentration, hematocrit, MCH, CHC, spleen size, or spleen volume are equal to or about 10%, 20%, 25%, 30%, 40%, 50%, 60%, 70%, 75%, 80%, 90%, 100%, 200%, or 500% greater than the levels of hemoglobin concentration, hematocrit, MCH, CHC, spleen size, or spleen volume in the top 10%, top 5%, top 4%, top 3%, top 2%, or top 1% in the reference population.

In certain embodiments, the decreased levels of MCV or RDW are equal to or about 10%, 20%, 25%, 30%, 40%, 50%, 60%, 70%, 75%, 80%, 90%, or 100% less than the levels of MCV or RDW in the bottom 10%, bottom 5%, bottom 4%, bottom 3%, bottom 2%, or bottom 1% in the reference population.

In certain embodiments, the subsequent dose of the mTOR inhibitor is about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg less than the initial dose of the mTOR inhibitor, or is about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.5 mg/kg, about 2.6 mg/kg, about 2.7 mg/kg, about 2.8 mg/kg, about 2.9 mg/kg, about 3.0 mg/kg, about 3.5 mg/kg, about 4.0 mg/kg, about 4.5 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, or about 15 mg/kg less than the initial dose of the mTOR inhibitor. In certain embodiment, the subsequent dose of the mTOR inhibitor is the same as the initial dose of the mTOR inhibitor.

In certain embodiments, the subsequent dose of the ActRIIB ligand trap is about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg less than the initial dose of the ActRIIB ligand trap, or is about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.5 mg/kg, about 2.6 mg/kg, about 2.7 mg/kg, about 2.8 mg/kg, about 2.9 mg/kg, about 3.0 mg/kg, about 3.5 mg/kg, about 4.0 mg/kg, about 4.5 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, or about 35 mg/kg less than the initial dose of the ActRIIB ligand trap. In certain embodiment, the subsequent dose of the ActRIIB ligand trap is the same as the initial dose of the ActRIIB ligand trap.

In certain embodiments, the subsequent dose of the mTOR inhibitor is about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg greater than the initial dose of the mTOR inhibitor, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.5 mg/kg, about 2.6 mg/kg, about 2.7 mg/kg, about 2.8 mg/kg, about 2.9 mg/kg, about 3.0 mg/kg, about 3.5 mg/kg, about 4.0 mg/kg, about 4.5 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, or about 35 mg/kg greater than the initial dose of the mTOR inhibitor.

In certain embodiments, the subsequent dose of the ActRIIB ligand trap is about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg greater than the initial dose of the ActRIIB ligand trap, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, about 1.9 mg/kg, about 2.0 mg/kg, about 2.1 mg/kg, about 2.2 mg/kg, about 2.3 mg/kg, about 2.4 mg/kg, about 2.5 mg/kg, about 2.5 mg/kg, about 2.6 mg/kg, about 2.7 mg/kg, about 2.8 mg/kg, about 2.9 mg/kg, about 3.0 mg/kg, about 3.5 mg/kg, about 4.0 mg/kg, about 4.5 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, or about 35 mg/kg greater than the initial dose of the ActRIIB ligand trap.

### 6.4.1 ActRIIB Ligand Trap Dosing Regimens

In certain embodiments, the ActRIIB ligand trap administered to a subject according to the methods provided herein (*see* Section 6.3) is a product resulting from expression from an opening reading frame with the nucleotide sequence of SEQ ID NO:34 or a degenerate version of SEQ ID NO:34 that encodes SEQ ID NO:11. In certain embodiments, the dose of the ActRIIB ligand trap (*e.g.* a product resulting from expression from an opening reading frame with the nucleotide sequence of SEQ ID NO:34 or a degenerate version of SEQ ID NO:34 that encodes SEQ ID NO:11) is about 0.3 mg/kg, is about 0.35 mg/kg, is about 0.4 mg/kg, about 0.45 mg/kg, about 0.5 mg/kg, about 0.6 mg/kg, about 0.7 mg/kg, about 0.8 mg/kg, about 0.9 mg/kg, about 1.0 mg/kg, about 1.25 mg/kg, about 1.33 mg/kg, about 1.50 mg/kg, about 1.75 mg/kg, or about 2,00 mg/kg. In certain embodiments, the dose of the ActRIIB ligand trap is about 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg. In certain embodiments, the dose of the ActRIIB ligand trap is about 0.3 mg/kg. In certain embodiments, the dose of the ActRIIB ligand trap is about 0.45 mg/kg. In certain embodiments, the dose of the ActRIIB ligand trap is about 0.6 mg/kg. In certain embodiments, the dose of the ActRIIB ligand trap is about 0.8 mg/kg. In certain embodiments, the dose of the ActRIIB ligand trap is about 1.0 mg/kg. In certain embodiments, the dose of the ActRIIB ligand trap is about 1.25 mg/kg. In certain embodiments, the dose of the ActRIIB ligand trap is about 1.33 mg/kg. In certain embodiments, the dose of the ActRIIB ligand trap is about 1.50 mg/kg. In certain embodiments, the dose of the ActRIIB ligand trap is about 1.75 mg/kg.

In certain embodiments, the dose of the ActRIIB ligand trap is an initial dose. In certain embodiments, the initial dose of the ActRIIB ligand trap is about 0.1 mg/kg, about 0.3 mg/kg, about 0.5 mg/kg, about 0.6 mg/kg, about 0.75 mg/kg, about 0.8 mg/kg, about 1.0 mg/kg, about 1.33 mg/kg, about 1.5 mg/kg, or about 1.75 mg/kg. In certain embodiments, the initial dose of the ActRIIB ligand trap is about 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg. In certain embodiments, the initial dose of the ActRIIB ligand trap is about 0.1 mg/kg. In certain embodiments, the initial dose of the ActRIIB ligand trap is about 0.3 mg/kg. In certain embodiments, the initial dose of the ActRIIB ligand trap is about 0.5 mg/kg. In certain embodiments, the initial dose of the ActRIIB ligand trap is about 0.6 mg/kg. In certain embodiments, the initial dose of the ActRIIB ligand trap is about 0.75 mg/kg. In certain embodiments, the initial dose of the ActRIIB ligand trap is about 0.8 mg/kg. In certain embodiments, the initial dose of the ActRIIB ligand trap is about 1.0 mg/kg. In certain embodiments, the initial dose of the ActRIIB ligand trap is about 0.8 mg/kg. In certain embodiments, the initial dose of the ActRIIB ligand trap is about 1.5 mg/kg. In certain embodiments, the initial dose of the ActRIIB ligand trap is about 1.75 mg/kg. In certain embodiments, the initial dose of the ActRIIB ligand trap is about 2 mg/kg. In certain embodiments, the initial dose of the ActRIIB ligand trap is about 5 mg/kg. In certain embodiments, the initial dose of the ActRIIB ligand trap is about 10 mg/kg. In certain embodiments, the initial dose of the ActRIIB ligand trap is about 15mg/kg.In certain embodiments, the dose of the ActRIIB ligand trap is a subsequent dose. In certain embodiments, the subsequent dose of the ActRIIB ligand trap is determined herein in this Section. In certain embodiments, the subsequent dose of the ActRIIB ligand trap is determined based on the difference between the second measurement of hemoglobin concentration, hematocrit, MCH, MCV, MCV, CHC, RDW, reticulocytes, spleen size, or spleen volume and the first measurement of hemoglobin concentration, hematocrit, MCH, MCV, MCV, CHC, RDW, reticulocytes, spleen size, or spleen volume. In certain embodiments, the subsequent dose of the ActRIIB ligand trap is about 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg. In certain embodiments, the subsequent dose of the ActRIIB ligand trap is about 0.1 mg/kg, about 0.3 mg/kg, about 0.5 mg/kg, about 0.6 mg/kg, about 0.75 mg/kg, about 0.8 mg/kg, about 1.0 mg/kg, about 1.33 mg/kg, or about 1.5 mg/kg, or about 1.75 mg/kg. In certain embodiments, the subsequent dose of the ActRIIB ligand trap is about 0.1 mg/kg. In certain embodiments, the subsequent dose of the ActRIIB ligand trap is about 0.3 mg/kg. In certain embodiments, the subsequent dose of the ActRIIB ligand trap is about 0.5 mg/kg. In certain embodiments, the subsequent dose of the ActRIIB ligand trap is about 0.6 mg/kg. In certain embodiments, the subsequent dose of ActRIIB ligand trap is about 0.75 mg/kg. In certain embodiments, the subsequent dose of the ActRIIB ligand trap is about 0.8 mg/kg. In certain embodiments, the subsequent dose of ActRIIB ligand trap is about 1.0 mg/kg. In certain embodiments, the subsequent dose of the ActRIIB ligand trap is about 1.33 mg/kg. In certain embodiments, the subsequent dose of ActRIIB ligand trap is about 1.5 mg/kg. In certain embodiments, the subsequent dose of the ActRIIB ligand trap is about 1.75 mg/kg. In certain embodiments, the subsequent dose of the ActRIIB ligand trap is about 2 mg/kg. In certain embodiments, the subsequent dose of the ActRIIB ligand trap is about 5 mg/kg. In certain embodiments, the subsequent dose of the ActRIIB ligand trap is about 10 mg/kg. In certain embodiments, the subsequent dose of the ActRIIB ligand trap is about 15 mg/kg.

In certain embodiments, the subsequent dose of the ActIIB ligand trap is about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg greater than the initial dose, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, or about 0.5 mg/kg greater than the initial dose of the ActRIIB ligand trap.

In certain embodiments, the subsequent dose of the ActIIB ligand trap is about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg greater than the initial dose, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, or about 0.5 mg/kg less than the initial dose of the ActIIB ligand trap.

In certain embodiments, the subsequent dose of the ActIIB ligand trap is administered more frequently than the initial dose of the ActIIB ligand trap. In certain embodiments, the subsequent dose of the ActIIB ligand trap is administered less frequently than the initial dose of the ActIIB ligand trap. In certain embodiments, the subsequent dose of the ActIIB ligand trap is administered at the same frequency as the initial dose of the ActIIB ligand trap. In certain embodiments, the subsequent dose of the ActIIB ligand trap is administered every 14, 15, 16, 17, 18, 19, 20, 21, 22, 23, 24, 25, 26, 27, or 28 days. In certain embodiments, the subsequent dose is administered every 21 days. In certain embodiments, the subsequent dose is administered twice per week. In certain embodiments, the subsequent dose is administered once per week. In certain embodiments, the subsequent dose of the ActIIB ligand trap is administered continuously and/or indefinitely.

In certain embodiments, the ActRIIB ligand trap is administered to the subject subcutaneously. In certain embodiments, the ActRIIB ligand trap is administered to the subject subcutaneously in the upper arm, abdomen, or thigh of the subject. In certain embodiments, the ActRIIB ligand trap is administered to the subject every 21 days. In certain embodiments, the ActRIIB ligand trap is administered twice per week. In certain embodiments, the ActRIIB ligand trap is administered once per week. In certain embodiments, the ActRIIB ligand trap is administered to the subject every 14, 15, 16, 17, 18, 19, 20, 21, 22, 23, 24, 25, 26, 27, or 28 days. In certain embodiments, the ActRIIB ligand trap is administered to the subject every 21 days, subcutaneously in the upper arm, abdomen, or thigh of the subject.

In certain embodiments, the ActRIIB ligand trap is part of a composition as described in Section 6.6. In certain embodiments, the ActRIIB ligand trap is a sterile, preservative-free, lyophilized powder reconstituted in water for injection. In certain embodiments, a single dose of the ActRIIB ligand trap is reconstituted in a volume of water for injection of greater than 1 mL. In such embodiments, the single dose of the ActRIIB ligand trap is administered to the subject via two injections of equal volume of reconstituted ActRIIB ligand trap. In certain embodiments, the two injections are administered to the subject at separate sites, *e.g.,* one injection in the right thigh and one injection in the left thigh.

### 6.4.2 mTOR inhibitor Dosing Regimens

In certain embodiments, the mTOR inhibitor is rapamycin. In certain embodiments, the mTOR inhibitor is a pharmaceutically acceptable salt or hydrate of rapamycin. In certain embodiments, the mTOR inhibitor is rapamycin. In certain embodiments, the mTOR inhibitor is a pharmaceutically acceptable salt or hydrate of rapamycin. In certain embodiments, the mTOR inhibitor is deforolimus. In certain embodiments, the mTOR inhibitor is a pharmaceutically acceptable salt or hydrate of deforolimus. In certain embodiments, the mTOR inhibitor is everolimus. In certain embodiments, the mTOR inhibitor is a pharmaceutically acceptable salt or hydrate of everolimus. In certain embodiments, the mTOR inhibitor is temsirolimus. In certain embodiments, the mTOR inhibitor is a pharmaceutically acceptable salt or hydrate of temsirolimus. In certain embodiments, the mTOR inhibitor is ridaforolimus. In certain embodiments, the mTOR inhibitor is a pharmaceutically acceptable salt or hydrate of ridaforolimus. In certain embodiments, the mTOR inhibitor is tacrolimus (FK-506). In certain embodiments, the mTOR inhibitor is a pharmaceutically acceptable salt or hydrate of tacrolimus (FK-506). In certain embodiments, the mTOR inhibitor is zotarolimus (ABT-578). In certain embodiments, the mTOR inhibitor is a pharmaceutically acceptable salt or hydrate of zotarolimus (ABT-578).

In certain embodiments, the mTOR inhibitor is a non-rapamycin analog mTOR inhibiting compound. In certain embodiments, the mTOR inhibitor is a pharmaceutically acceptable salt or hydrate of a non-rapamycin analog mTOR inhibiting compound. In certain embodiments, the non-rapamycin analog mTOR inhibiting compounds including, but not limited to, 3,3-Diindolylmethane (DIM), 32 deoxy-rapamycin (SAR943), 3-Methyladenine, Arenobufagin, AZD 3147, AZD-2014 (Vistusertib), AZD8055, BC-LI-0186, BEZ235 (Dactolisib), Bimiralisib (PQR309), Caffeine, CC-115, CC-223 (Onatasertib), Chrysophanic acid (Chrysophanol), Ciclopirox Olamine, CID3528206, CIDD 0067106, Compound 401 (*see, e.g.,* Griffen et al., 2005, J. Med. Chem. 48:569), Curcumin, CZ415, eCF 309, epigallocatechin gallate (EGCG), ETP 45658, ETP-46464, GDC-0084, GDC-0349, GDC-0980 (Apitolisib, RG7422), Genistein, GNE-477, GNE-493, GSK1059615, GSK-2126458 (Omipalisib), ICSN3250, INK-128 (MLN0128, Sapanisertib), KU-0063794, LY3023414 (Samotolisib), ME-344 (NV-344), Metformin, MHY1485, MTI-31 (LXI-15029), mTOR inhibitor 10 (CAS No.: 1222999-54-3; *see, e.g.,* Liu et al., 2011, Bioorg. Med. Chem. Lett. 21(13):4036-4040), mTOR/HDAC1-IN-12l ((R)-N-(4-(1-(7-(hydroxyamino)-7-oxoheptyl)-4-morpholino-1H-pyrazolo[3,4-d]pyrimidin-6-yl)phenyl)-2-methylmorpholine-4-carboxamide), mTORC1-IN-1 (2-(4-((2,4,6-Trimethyl-3-(4-(3-(methylsulfonyl)pyridin-2-yl)piperazine-1-carbonyl)phenyl)amino)piperidin-1-yl)benzonitrile), mTOR-IN-1 (CAS No.: 1207358-59-5), mTOR-IN-17 ((E)-6-(Phenyldiazenyl)-1H-dipyrazolo[1,5-a:4',3'-e]pyrimidine-3,4,7-triamine), Niclosamide, NV-128 (LY-303511), NVP-BGT226, Omipalisib, OSI-027, OSU-53, Palomid 529 (P529), Perifosine, PF 05212384, PF-04691502, PF-04979064, PI 103 hydrochloride, PKI-402, PKI-587 (Gedatolisib), PP 121, PP-242 (Torkinib), PP30, PQR-620, QL-IX-55, Rapalink, Resveratrol, SAR245409, LY294002, wortmannin, quercetin, myricetin, staurosporine, SF1126, SF2523, STK16-IN-1, Sunitinib, Torin 1, Torin 2, Triacetyl aloe-emodin, VS-5584, WAY-600, WJD008, WYE-125132 (WYE-132), WYE-354, WYE-687, XL388, XL-765 (Voxtalisib, SAR245409), (+)-Usnic acid, Arnicolide D, BGT226 maleate (NVP-BGT226 maleate), Cbz-B3A, CC-115 hydrochloride, Cyclovirobuxine D, Dihydromyricetin (Ampelopsin or Ampeloptin), FT-1518, GNE-317, Hederacolchiside A1, JR-AB2-011, KU-0060648, NSC781406, PI-103, Polyphyllin I, Pomiferin (NSC 5113), PQR-530, Rheb inhibitor NR1, Rotundic acid, or WYE-687 dihydrochloride.

In certain embodiments, the mTOR inhibitor is selected from the group consisting of rapamycin, deforolimus, everolimus, temsirolimus, ridaforolimus, tacrolimus (FK-506), zotarolimus (ABT-578), 3,3-Diindolylmethane (DIM), 32 deoxy-rapamycin (SAR943), 3-Methyladenine, Arenobufagin, AZD 3147, AZD-2014 (Vistusertib), AZD8055, BC-LI-0186, BEZ235 (Dactolisib), Bimiralisib (PQR309), Caffeine, CC-115, CC-223 (Onatasertib), Chrysophanic acid (Chrysophanol), Ciclopirox Olamine, CID3528206, CIDD 0067106, Compound 401, Curcumin, CZ415, eCF 309, epigallocatechin gallate (EGCG), ETP 45658, ETP-46464, GDC-0084, GDC-0349, GDC-0980 (Apitolisib, RG7422), Genistein, GNE-477, GNE-493, GSK1059615, GSK-2126458 (Omipalisib), ICSN3250, INK-128 (MLN0128, Sapanisertib), KU-0063794, LY3023414 (Samotolisib), ME-344 (NV-344), Metformin, MHY1485, MTI-31 (LXI-15029), mTOR inhibitor 10 (CAS No.: 1222999-54-3), mTOR/HDAC1-IN-12l, mTORC1-IN-1, mTOR-IN-1, mTOR-IN-17, Niclosamide, NV-128 (LY-303511), NVP-BGT226, Omipalisib, OSI-027, OSU-53, Palomid 529 (P529), Perifosine, PF 05212384, PF-04691502, PF-04979064, PI 103 hydrochloride, PKI-402, PKI-587 (Gedatolisib), PP 121, PP-242 (Torkinib), PP30, PQR-620, QL-IX-55, Rapalink, Resveratrol, SAR245409, LY294002, wortmannin, quercetin, myricetin, staurosporine, SF1126, SF2523, STK16-IN-1, Sunitinib, Torin 1, Torin 2, Triacetyl aloe-emodin, VS-5584, WAY-600, WJD008, WYE-125132 (WYE-132), WYE-354, WYE-687, XL388, XL-765 (Voxtalisib, SAR245409), (+)-Usnic acid, Arnicolide D, BGT226 maleate (NVP-BGT226 maleate), Cbz-B3A, CC-115 hydrochloride, Cyclovirobuxine D, Dihydromyricetin (Ampelopsin or Ampeloptin), FT-1518, GNE-317, Hederacolchiside A1, JR-AB2-011, KU-0060648, NSC781406, PI-103, Polyphyllin I, Pomiferin (NSC 5113), PQR-530, Rheb inhibitor NR1, Rotundic acid, and WYE-687 dihydrochloride. In certain embodiments, the mTOR inhibitor is selected from the group consisting of rapamycin, deforolimus, everolimus, temsirolimus, ridaforolimus, tacrolimus (FK-506), and zotarolimus (ABT-578).

In certain embodiments, the mTOR inhibitor is an ATP competitive inhibitor.

In certain embodiments, the mTOR inhibitor (*e*.*g*. rapamycin) or pharmaceutically acceptable salt or hydrate thereof is administered to the subject orally. In certain embodiments, the mTOR inhibitor (*e*.*g*. rapamycin) or pharmaceutically acceptable salt or hydrate thereof is administered to the subject daily. In certain embodiments, the mTOR inhibitor (*e*.*g*. rapamycin) or pharmaceutically acceptable salt or hydrate thereof is administered to the subject every 1, 2, 3, 4, 5, 6, 7, 8, 9, 10, 11, 12, 13, 14, 15, 16, 17, 18, 19, 20, 21, 22, 23, 24, 25, 26, 27, or 28 days.

In certain embodiments, the dose of the mTOR inhibitor (e.g. rapamycin) or pharmaceutically acceptable salt or hydrate thereof is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, or about 15 mg/kg. In certain embodiments, the dose of mTOR inhibitor (*e*.*g*. rapamycin) or pharmaceutically acceptable salt or hydrate thereof is about 0.1 mg to about 0.5 mg, about 0.5 mg to about 1.0 mg, about 1 mg to about 5 mg, about 5 mg to about 10mg, or about 10 mg to about 15 mg.

In certain embodiments, the dose of the mTOR inhibitor (*e*.*g*. rapamycin) or pharmaceutically acceptable salt or hydrate thereof is an initial dose. In certain embodiments, the initial dose of the mTOR inhibitor (e.g. rapamycin) is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, or about 15 mg/kg. In certain embodiments, the initial dose of the mTOR inhibitor (*e*.*g*. rapamycin) or pharmaceutically acceptable salt or hydrate thereof is about 0.1 mg/kg to about 0.5 mg/kg, about 0.5 mg/kg to about 1.0 mg/kg, about 1 mg/kg to about 5 mg/kg, about 5 mg/kg to about 10 mg/kg, about 10 mg/kg to about 15 mg/kg, or about 15 mg/kg to about 50 mg/kg.

In certain embodiments, the dose of the mTOR inhibitor (*e*.*g*. rapamycin)or pharmaceutically acceptable salt or hydrate thereof is a subsequent dose. In certain embodiments, the subsequent dose of the mTOR inhibitor (*e*.*g*. rapamycin) or pharmaceutically acceptable salt or hydrate thereof is determined according to the methods provided herein in this Section. In certain embodiments, the subsequent of the mTOR inhibitor or pharmaceutically acceptable salt or hydrate thereof is determined based on the difference between the second measurement of hemoglobin concentration, hematocrit, MCH, MCV, MCV, CHC, RDW, reticulocytes, spleen size, or spleen volume and the first measurement of hemoglobin concentration, hematocrit, MCH, MCV, MCV, CHC, RDW, reticulocytes, spleen size, or spleen volume. In certain embodiments, the subsequent dose of the mTOR inhibitor (*e*.*g*. rapamycin) or pharmaceutically acceptable salt or hydrate thereof is about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, or about 15 mg/kg. In certain embodiments, the initial dose of the mTOR inhibitor (*e*.*g*. rapamycin) or pharmaceutically acceptable salt or hydrate thereof is about 0.1 mg/kg to about 0.5 mg/kg, about 0.5 mg/kg to about 1.0 mg/kg, about 1 mg/kg to about 5 mg/kg, about 5 mg/kg to about 10 mg/kg, about 10 mg/kg to about 15 mg/kg, or about 15 mg/kg to about 50 mg/kg.

In certain embodiments, the subsequent dose of the mTOR inhibitor (*e*.*g*. rapamycin)or pharmaceutically acceptable salt or hydrate thereof is about 0.5 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg greater than the initial dose, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, or about 0.5 mg/kg greater than the initial dose of the mTOR inhibitor (*e*.*g*. rapamycin)or pharmaceutically acceptable salt or hydrate thereof.

In certain embodiments, the subsequent dose of the mTOR inhibitor (*e*.*g*. rapamycin)or pharmaceutically acceptable salt or hydrate thereof is about 0.5 mg, about 0.5 mg, about 0.6 mg, about 0.7 mg, about 0.8 mg, about 0.9 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2.0 mg, about 2.1 mg, about 2.2 mg, about 2.3 mg, about 2.4 mg, about 2.5 mg, about 2.5 mg, about 2.6 mg, about 2.7 mg, about 2.8 mg, about 2.9 mg, about 3.0 mg, about 3.5 mg, about 4.0 mg, about 4.5 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, or about 35 mg less than the initial dose, or about 0.05 mg/kg, about 0.06 mg/kg, about 0.07 mg/kg, about 0.08 mg/kg, about 0.09 mg/kg, about 0.1 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, about 0.25 mg/kg, about 0.26 mg/kg, about 0.27 mg/kg, about 0.28 mg/kg, about 0.29 mg/kg, about 0.3 mg/kg, about 0.35 mg/kg, about 0.4 mg/kg, about 0.45 mg/kg, or about 0.5 mg/kg less than the initial dose of the mTOR inhibitor (*e*.*g*. rapamycin) or pharmaceutically acceptable salt or hydrate thereof.

### 6.5 Patient Population

The subjects treated in accordance with the methods described herein can be any mammals such as rodents and primates, and in a preferred embodiment, human.

In certain embodiments, the subject is transfusion-dependent.

In certain embodiments, the subject is transfusion-independent.

In certain embodiments, the methods described herein can be used to treat anemia, such as, an anemia associated with ineffective erythropoiesis, or thalassemia in the subject described herein.

In certain embodiments, the methods described herein can be used for enhancing late stage erythropoiesis in the subject described herein.

In certain embodiments, the subject treated in accordance with the methods described herein (*see* Section 6.3) has beta-thalassemia. In certain embodiments, the beta-thalassemia is transfusion-dependent beta-thalassemia. Transfusion-dependent beta-thalassemia is also known as "Cooley's anemia". In certain embodiments, the beta-thalassemia is beta-thalassemia major. In certain embodiments, the transfusion-dependent beta-thalassemia is beta-thalassemia major. In certain embodiments, the beta-thalassemia is non-transfusion-dependent beta-thalassemia. In certain embodiments, the beta-thalassemia is beta-thalassemia intermediate. In certain embodiments, the transfusion-dependent beta-thalassemia is non-beta-thalassemia intermediate. In certain embodiments, the subject has HbE/beta thalassemia. In certain embodiments, the subject (i) has beta-thalassemia major; (ii) has severe HbE/beta-thalassemia; and (iii) is transfusion-dependent. In certain embodiments, the subject (i) has beta-thalassemia intermedia; (ii) has mild/moderate HbE/beta-thalassemia; and (iii) is non-transfusion-dependent.

In certain embodiments, the subject treated in accordance with the methods described herein (*see* Section 6.3), has transfusion-dependent beta-thalassemia. In certain embodiments, the subject has been diagnosed with transfusion-dependent beta-thalassemia. In certain embodiments, the subject has been diagnosed with beta-thalassemia and hemoglobin E. In certain embodiments, the diagnosis has been confirmed by genetic analysis. In certain embodiments, the transfusion-dependent beta-thalassemia is beta-thalassemia major. In certain embodiments, the transfusion-dependent beta-thalassemia is beta-thalassemia major. In certain embodiments, the subject comprises a genotype comprising homozygosity or compound heterozygosity for a mutant beta globin allele. In certain embodiments, the homozygosity comprises β⁰/β⁰, wherein β⁰ refers to an allele associated with lack of beta globin chain synthesis. In certain embodiments, the homozygosity comprises β⁺/β⁺, wherein β⁺ refers to an allele associated with reduced beta globin chain synthesis. In certain embodiments, the compound heterozygosity comprises β⁰/β⁺, wherein β⁰ refers to an allele associated with lack of beta globin chain synthesis, and wherein β⁺ refers to an allele associated with reduced beta globin chain synthesis. In certain embodiments, the compound heterozygosity comprises β⁰/HbE, wherein β⁰ refers to an allele associated with lack of beta globin chain synthesis, and wherein HbE refers to hemoglobin E. In certain embodiments, the compound heterozygosity comprises β⁺/HbE, wherein β⁺ refers to an allele associated with reduced beta globin chain synthesis, and wherein HbE refers to hemoglobin E. In certain embodiments, the subject has symptomatic thalassemia. In certain embodiments, the subject has co-inherited duplication of the alpha-globin gene. In certain embodiments, the subject has been diagnosed with transfusion-dependent beta-thalassemia. In certain embodiments, the diagnosis has been confirmed by genetic analysis. In certain embodiments, the subject is a human infant subject. In certain embodiments, the subject has hereditary persistence of fetal hemoglobin.

In certain embodiments, the methods described herein can be used to treat MDS and/or non-proliferative CMML in the subject described herein.

In certain embodiments, the anemia treated by the methods described herein is an anemia due to protein precipitation, protein aggregation, and protein conformation change.

In certain embodiment, the methods described herein can be used to treat other hemoglobinopathies, such as sickle cell anemia.

In certain embodiment, the methods described herein can be used to treat any anemia that is resistant to any erythropoietic stimulating agent or erythropoietin (EPO).

In certain embodiments, the percentage of erythroblasts in a subject treated in accordance with the methods provided herein that are ring sideroblasts is at least 10%, 11%, 12%, 13%, 14%, 15%, 16%, 17%, 18%, 19%, or at least 20%. In certain embodiments, the percentage of erythroblasts in a subject treated in accordance with the methods provided herein that are ring sideroblasts is at least 15%. In certain embodiments, the percentage of erythroblasts in a subject treated in accordance with the methods provided herein that are ring sideroblasts is about 15%. In certain embodiments, the percentage of erythroblasts in a subject treated in accordance with the methods provided herein that are ring sideroblasts is between about 10% and about 20%. In certain embodiments, the percentage of erythroblasts in a subject treated in accordance with the methods provided herein that are ring sideroblasts is between about 12% and 17%. In certain embodiments, a subject treated in accordance with the methods provided herein has a ringed sideroblast to normal erythroblast ratio of at least 1:10, at least 1:7, or at least 1:5.

In certain embodiments, the subject treated according to the methods provided herein has a blood-related disorder. In certain embodiments, the blood-related disorder is anemia. In certain embodiments, the blood-related disorder is anemia requiring transfusion. In certain embodiments, the blood-related disorder is MDS. In certain embodiments, the blood-related disorder is non-proliferative CMML.

In certain embodiments, the subject treated in accordance with the methods described here can be of any age. In certain embodiments, the subject treated in accordance with the methods described herein is less than 18 years old. In a specific embodiment, the subject treated in accordance with the methods described herein is less than 13 years old. In another specific embodiment, the subject treated in accordance with the methods described herein is less than 12, less than 11, less than 10, less than 9, less than 8, less than 7, less than 6, or less than 5 years old. In another specific embodiment, the subject treated in accordance with the methods described herein is 1-3 years old, 3-5 years old, 5-7 years old, 7-9 years old, 9-11 years old, 11-13 years old, 13-15 years old, 15-20 years old, 20-25 years old, 25-30 years old, or greater than 30 years old. In another specific embodiment, the subject treated in accordance with the methods described herein is 30-35 years old, 35-40 years old, 40-45 years old, 45-50 years old, 50-55 years old, 55-60 years old, or greater than 60 years old. In another specific embodiment, the subject treated in accordance with the methods described herein is 60-65 years old, 65-70 years old, 70-75 years old, 75-80 years old, or greater than 80 years old.

In certain embodiments, the subject requires regular, lifelong red blood cell transfusions. In certain embodiments, the subject has a high transfusion burden. In certain embodiments, high transfusion burden is 12 or more red blood cell units over 24 weeks prior to treatment according to the methods provided herein. In certain embodiments, the subject has a low transfusion burden. In certain embodiments, low transfusion burden is 7-12 red blood cell units over 24 weeks prior to treatment according to the methods provided herein.

In certain embodiments, the subject has one or more transfusion-dependent beta-thalassemia clinical complications. Non-limiting examples of transfusion-dependent beta-thalassemia clinical complications include growth retardation, pallor, jaundice, poor musculature, genu valgum, hepatosplenomegaly, leg ulcers, development of masses from extramedullary hematopoiesis, and skeletal changes resulting from expansion of the bone marrow. In certain embodiments, the subject has one or more complications of chronic red blood cell transfusions. Non-limiting examples of complications of chronic red blood cell transfusions include transfusion-associated infections, such as, for example, hepatitis B virus infection, hepatitis C virus infection, and human immunodeficiency virus infection, alloimmunization, and organ damage due to iron overload, such as, for example, liver damage, heart damage, and endocrine gland damage.

In certain embodiments, the subject treated in accordance with the methods described herein (*see* Section 6.3), has non-transfusion-dependent beta-thalassemia. In certain embodiments, the subject has been diagnosed with beta-thalassemia. In certain embodiments, the subject has been diagnosed with beta-thalassemia and hemoglobin E. In certain embodiments, the beta-thalassemia has been confirmed by genetic analysis. In certain embodiments, the non-transfusion-dependent beta-thalassemia is beta-thalassemia intermedia. In certain embodiments, the non-transfusion-dependent beta thalassemia is mild-moderate hemoglobin E/beta-thalassemia. In certain embodiments, the non-transfusion-dependent beta-thalassemia does not require regular red blood cell transfusion. In certain embodiments, the subject seldom requires red blood cell transfusions. In certain embodiments, the non-transfusion-dependent beta-thalassemia requires regular red blood cell transfusion later in life. In certain embodiments, the subject has received 0 to 6 red blood cell units during the 24-week period prior to treatment according to the methods provided herein. In certain embodiments, the subject has a mean baseline hemoglobin level of less than 10.0 g/dL.

In certain embodiments, the beta-thalassemia is non-transfusion-dependent beta-thalassemia. In certain embodiments, the beta-thalassemia is beta-thalassemia intermediate. In certain embodiments, the transfusion-dependent beta-thalassemia is non-beta-thalassemia intermediate. In certain embodiments, the subject comprises a genotype comprising compound heterozygosity. In certain embodiments, the compound heterozygosity comprises a β⁰ allele, wherein β⁰ refers to an allele associated with lack of beta globin chain synthesis. In certain embodiments, the compound heterozygosity comprises a β⁺ allele, wherein β⁺ refers to an allele associated with reduced beta globin chain synthesis. In certain embodiments, the compound heterozygosity comprises β⁰/β⁺, wherein β⁰ refers to an allele associated with lack of beta globin chain synthesis, and wherein β⁺ refers to an allele associated with reduced beta globin chain synthesis. In certain embodiments, the compound heterozygosity comprises one or more hemoglobin variants. In certain embodiments, the hemoglobin variant is hemoglobin E. In certain embodiments, the subject (i) comprises a genotype comprising coinheritance of two severe beta globin chain mutations, and (ii) has alpha-thalassemia. In certain embodiments, the subject (i) comprises a genotype comprising coinheritance of two severe beta globin chain mutations, and (ii) has hereditary persistence of fetal hemoglobin. In certain embodiments, the subject has symptomatic thalassemia. In certain embodiments, the subject has co-inherited duplication of the alpha-globin gene. In certain embodiments, the subject has been diagnosed with beta-thalassemia. In certain embodiments, the diagnosis has been confirmed by genetic analysis.

In certain embodiments, the subject displays one or more non-transfusion-dependent beta-thalassemia clinical complications. Non-limiting examples of non-transfusion-dependent beta-thalassemia clinical complications include endocrine abnormalities, such as, for example, diabetes mellitus, hypothyroidism, hypogonadism, thrombotic events, pulmonary hypertension, hypercoagulability, the development of transfusion-dependency later in life, ineffective erythropoiesis, expansion of the hematopoietic tissue outside of the marrow medulla, formation of extramedullary hematopoiesis masses, skeletal deformities, osteopenia, osteoporosis, bone pain, gallstones, and leg ulcers. In certain embodiments, the subject exhibits alloimmunization.

In certain embodiments, the subject displays mild symptoms beta-thalassemia symptoms. In certain embodiments, the subject has near normal growth.

In certain embodiments, the non-transfusion-dependent beta-thalassemic subject displays severe symptoms. Non-limiting examples of severe symptoms include growth retardation, development retardation, and skeletal deformities.

In certain embodiments, the subject has splenomegaly. In certain embodiments, the splenomegaly develops in the first 6-12 months of the subject's life.

In certain embodiments, the subject has impaired growth during the first 10 years of the subject's life.

In certain embodiments, the subject exhibits microcytic, hypochromic anemia. In certain embodiments, the hemoglobin A2 levels in the subject prior to treatment of the subject according to the methods provided herein are elevated as compared to the hemoglobin A2 levels in a reference population (*e.g.,* a reference population as described in Section 6.9). In certain embodiments, the fetal hemoglobin levels in the subject prior to treatment of the subject according to the methods provided herein is elevated as compared to the fetal hemoglobin levels in a reference population (*e.g.,* a reference population as described in Section 6.9).

In certain embodiments, the subject does not express hemoglobin S.

In certain embodiments, the subject does not express hemoglobin S. In certain embodiments, the subject has not received red blood cell transfusions within 12 weeks prior to treatment according to the methods provided herein (*see* Section 6.3), wherein the subject has non-transfusion-dependent beta-thalassemia. In certain embodiments, the subject does not have active hepatitis C infection. In certain embodiments, the subject does not have active hepatitis B infection. In certain embodiments, the subject is not positive for human immunodeficiency virus. In certain embodiments, the subject does not have insulin-dependent diabetes. In certain embodiments, the subject has not been administered an erythropoiesis stimulating agent within 3 months prior to treatment according to the methods provided herein. In certain embodiments, the subject has not undergone iron chelation therapy within 168 days prior to treatment according to the methods provided herein. In certain embodiments, the subject has not undergone hydroxyurea treatment within 168 days prior to treatment according to the methods provided herein. In certain embodiments, the subject has not been administered biphosphonates within the 168 days prior to treatment according to the methods provided herein. In certain embodiments, the subject does not have uncontrolled hypertension. Uncontrolled hypertension refers to > Grade 1 according to NCI CTCAE version 4.0. In certain embodiments, the subject does not have liver disease with ALT greater than 3 times the upper limit of normal. In certain embodiments, the subject does not have liver disease with histopathological evidence of liver cirrhosis/fibrosis as determined by liver biopsy. In certain embodiments, the subject does not have heart disease. Heart disease or heart failure can be classified by the New York Heart Association as classification 3 or higher. In certain embodiments, the subject does not have arrhythmia requiring treatment. In certain embodiments, the subject does not have lung disease. Non-limiting examples of lung disease include pulmonary fibrosis and pulmonary hypertension. In certain embodiments, the subject does not have a creatinine clearance rate of less than 60 mL/min as determined by the Cockroff-Gault method. In certain embodiments, the subject does not have folate deficiency. In certain embodiments, the subject does not have proteinuria of Grade 3 or higher. In certain embodiments, the subject does not have adrenal insufficiency. In certain embodiments, the subject has not undergone a major surgery within 30 days prior to treatment according to the methods provided herein, except for wherein the major surgery is splenectomy. In certain embodiments, the subject does not have a history of severe allergic or anaphylactic reactions or hypersensitivity to recombinant proteins. In certain embodiments, the subject has not undergone long-term anticoagulant therapy. Nonlimiting examples of anticoagulant therapy includes heparin and warfarin. In certain embodiments, the subject is not undergoing treatment with cytotoxic agents, systemic corticosteroids, immunosuppressants, or anticoagulant therapy within 28 days prior to treatment according to the methods provided herein.

In certain embodiments, the subject is undergoing other treatment interventions. Non-limiting examples of other treatment interventions include splenectomy, transfusion therapy, iron chelation therapy, and fetal hemoglobin-inducing agents. In certain embodiments, the subject requires iron chelation therapy. *See* Section 6.3.1 for a description of combination therapies.

In certain embodiments, the subject treated in accordance with the methods provided herein (*see* Section 6.3) has been diagnosed with IPSS-R-defined MDS. In certain embodiments, the subject treated in accordance with the methods provided herein has been diagnosed with IPSS-R-defined MDS and at least 10%, 11%, 12%, 13%, 14%, 15%, 16%, 17%, 18%, 19%, or at least 20% of erythroblasts in the subject are ring sideroblasts. In certain embodiments, at least 15% of erythroblasts in the subject are ring sideroblasts.

IPSS-R refers to the International Prognostic Scoring System-Revised, which is utilized in the evaluation of prognosis in myelodysplastic syndromes. *See, e.g.,* Greenberg et al., 2012, Blood 120(12):2454-2465, and Erratum in Blood, 1998; 91:1100. The IPSS-R utilizes a criteria point system to characterize myelodysplastic syndrome patient outcomes as very low risk (less than or equal to 1.5 points; median survival of 8.8 years), low risk (greater than 1.5 points, less than or equal to 3 points; median survival of 5.3 years); intermediate risk (greater than 3 points, less than or equal to 4.5 points; median survival of 3 years); high risk (greater than 4.5 points, less than or equal to 6 points; median survival of 1.6 years); or very high (greater than 6 points; median survival of 0.8 years). The point system evaluates, *inter alia,* (i) the percentage of bone marrow blasts in the subject; (ii) the karyotype of the subject; and (iii) and cytopenias in the subject (defined as hemoglobin concentration of less than 10 g/dL, absolute neutrophil count of less than 1,800/µL, and platelet count of less than 100,000/ µL).

In certain embodiments, the subject treated in accordance with the methods provided herein (*see* Section 6.3) has been diagnosed with non-proliferative CMML. In certain embodiments, the subject treated in accordance with the methods provided herein has been diagnosed with non-proliferative CMML and at least 10%, 11%, 12%, 13%, 14%, 15%, 16%, 17%, 18%, 19%, or at least 20% of erythroblasts in the subject are ring sideroblasts.

In certain embodiments, the subject treated in accordance with the methods provided herein (*see* Section 6.3) has MDS. In certain embodiments, the MDS is IPSS-defined low risk MDS. In certain embodiments, the MDS is IPSS-defined intermediate-1 risk MDS. In certain embodiments, the MDS is IPSS-defined intermediate-2 risk MDS. In certain embodiments, the MDS is IPSS-defined high risk MDS. In certain embodiments, the MDS is IPSS-R-defined very low risk MDS. In certain embodiments, the MDS is IPSS-R-defined low risk MDS. In certain embodiments, the MDS is IPSS-R-defined intermediate risk MDS. In certain embodiments, the MDS is IPSS-R-defined high risk MDS. In certain embodiments, the MDS is IPSS-R-defined very high risk MDS. In certain embodiments, the subject treated in accordance with the methods provided herein (i) has MDS and (ii) has RARS. In certain embodiments, the subject treated in accordance with the methods provided herein (i) has MDS and (ii) has RCMD-RS. In certain embodiments, the subject treated in accordance with the methods provided herein (i) has MDS, (ii) has RARS, and (iii) has RCMD-RS. In certain embodiments, the subject treated in accordance with the methods provided herein (i) has MDS, and (ii) at least 15% of erythroblasts in the subject are ring sideroblasts. In certain embodiments, the subject treated in accordance with the methods provided herein (i) has MDS, (ii) has RARS, and (iii) at least 15% of erythroblasts in the subject are ring sideroblasts. In certain embodiments, the subject treated in accordance with the methods provided herein (i) has MDS, (ii) has RCMD-RS, and (iii) at least 15% of erythroblasts in the subject are ring sideroblasts. In certain embodiments, the subject treated in accordance with the methods provided herein (i) has MDS, (ii) has RARS, (iii) has RCMD-RS, and (iv) at least 15% of erythroblasts in the subject are ring sideroblasts. In certain embodiments, the subject treated in accordance with the methods provided herein (i) has MDS, (ii) has RARS, and (iii) expresses SF3B1 with one or more mutations. In certain embodiments, the subject treated in accordance with the methods provided herein (i) has MDS, (ii) has RCMD-RS, and (iii) expresses SF3B1 with one or more mutations. In certain embodiments, the subject treated in accordance with the methods provided herein (i) has MDS, (ii) has RARS, (iii) has RCMD-RS, and (iv) expresses SF3B1 with one or more mutations. In certain embodiments, the subject treated in accordance with the methods provided herein (i) has MDS, (ii) at least 15% of erythroblasts in the subject are ring sideroblasts, and (iii) expresses SF3B1 with one or more mutations. In certain embodiments, the subject treated in accordance with the methods provided herein (i) has MDS, (ii) has RARS, (iii) at least 15% of erythroblasts in the subject are ring sideroblasts, and (iv) expresses SF3B1 with one or more mutations. In certain embodiments, the subject treated in accordance with the methods provided herein (i) has MDS, (ii) has RCMD-RS, (iii) at least 15% of erythroblasts in the subject are ring sideroblasts, and (iv) expresses SF3B1 with one or more mutations. In certain embodiments, the subject treated in accordance with the methods provided herein (i) has MDS, (ii) has RARS, (iii) has RCMD-RS, (iv) at least 15% of erythroblasts in the subject are ring sideroblasts, and (v) expresses SF3B1 with one or more mutations.

In certain embodiments, the subject treated in accordance with the methods provided herein (*see* Section 6.3) has non-proliferative CMML. In certain embodiments, the subject treated in accordance with the methods provided herein (i) has non-proliferative CMML and (ii) has RARS. In certain embodiments, the subject treated in accordance with the methods provided herein (i) has non-proliferative CMML and (ii) has RCMD-RS. In certain embodiments, the subject treated in accordance with the methods provided herein (i) has non-proliferative CMML, (ii) has RARS, and (iii) has RCMD-RS. In certain embodiments, the subject treated in accordance with the methods provided herein (i) has non-proliferative CMML, and (ii) at least 15% of erythroblasts in the subject are ring sideroblasts. In certain embodiments, the subject treated in accordance with the methods provided herein (i) has non-proliferative CMML, (ii) has RARS, and (iii) at least 15% of erythroblasts in the subject are ring sideroblasts. In certain embodiments, the subject treated in accordance with the methods provided herein (i) has non-proliferative CMML, (ii) has RCMD-RS, and (iii) at least 15% of erythroblasts in the subject are ring sideroblasts. In certain embodiments, the subject treated in accordance with the methods provided herein (i) has non-proliferative CMML, (ii) has RARS, (iii) has RCMD-RS, and (iv) at least 15% of erythroblasts in the subject are ring sideroblasts. In certain embodiments, the subject treated in accordance with the methods provided herein (i) has non-proliferative CMML, and (ii) expresses SF3B1 with one or more mutations. In certain embodiments, the subject treated in accordance with the methods provided herein (i) has non-proliferative CMML, (ii) has RARS, and (iii) expresses SF3B1 with one or more mutations. In certain embodiments, the subject treated in accordance with the methods provided herein (i) has non-proliferative CMML, (ii) has RCMD-RS, and (iii) expresses SF3B1 with one or more mutations. In certain embodiments, the subject treated in accordance with the methods provided herein (i) has non-proliferative CMML, (ii) has RARS, (iii) has RCMD-RS, and (iv) expresses SF3B1 with one or more mutations. In certain embodiments, the subject treated in accordance with the methods provided herein (i) has non-proliferative CMML, (ii) at least 15% of erythroblasts in the subject are ring sideroblasts, and (iii) expresses SF3B1 with one or more mutations. In certain embodiments, the subject treated in accordance with the methods provided herein (i) has non-proliferative CMML, (ii) has RARS, (iii) at least 15% of erythroblasts in the subject are ring sideroblasts, and (iv) expresses SF3B1 with one or more mutations. In certain embodiments, the subject treated in accordance with the methods provided herein (i) has non-proliferative CMML, (ii) has RCMD-RS, (iii) at least 15% of erythroblasts in the subject are ring sideroblasts, and (iv) expresses SF3B1 with one or more mutations. In certain embodiments, the subject treated in accordance with the methods provided herein has (i) has non-proliferative CMML, (ii) has RARS, (iii) has RCMD-RS, (iv) at least 15% of erythroblasts in the subject are ring sideroblasts, and (v) expresses SF3B1 with one or more mutations.

In certain embodiments, the subject treated in accordance with the methods provided herein *(see* Section 6.3) expresses a gene with a mutation associated with ineffective erythropoiesis. In certain embodiments, the subject treated in accordance with the methods provided herein expresses one or more splicing factor gene comprising one or more mutation. In a specific embodiment, the subject treated in accordance with the methods provided herein expresses SF3B1 with one or more mutations. In certain embodiments, the one or more mutations is in a non-coding region. In certain embodiments, SF3B1 is the gene encoding SB3B 1. In certain embodiments, the one or more mutations is in a coding region. In certain embodiments, SF3B1 is SF3B1 protein. In certain embodiments, the one or more mutations in SF3B1 protein is selected from the group consisting of E622D, R625C, H662Q, H662D, K66N, K666T, K666Q, K666E, A672D, K700E, I704N. In certain embodiments, the subject treated in accordance with the methods provided herein expresses SF3B 1 protein with the mutation E622D. In certain embodiments, the subject treated in accordance with the methods provided herein expresses SF3B1 protein with the mutation R625C. In certain embodiments, the subject treated in accordance with the methods provided herein expresses SF3B1 protein with the mutation H662Q. In certain embodiments, the subject treated in accordance with the methods provided herein expresses SF3B1 protein with the mutation H662D. In certain embodiments, the subject treated in accordance with the methods provided herein expresses SF3B1 protein with the mutation K66N. In certain embodiments, the subject treated in accordance with the methods provided herein expresses SF3B1 protein with the mutation K666T. In certain embodiments, the subject treated in accordance with the methods provided herein expresses SF3B1 protein with the mutation K666Q. In certain embodiments, the subject treated in accordance with the methods provided herein expresses SF3B1 protein with the mutation K666E. In certain embodiments, the subject treated in accordance with the methods provided herein expresses SF3B1 protein with the mutation A672D. In certain embodiments, the subject treated in accordance with the methods provided herein expresses SF3B1 with the mutation K700E. In certain embodiments, the subject treated in accordance with the methods provided herein expresses SF3B1 protein with the mutation I704N. In a specific embodiment, the subject treated in accordance with the methods provided herein expresses SRSF2 with one or more mutations. In a specific embodiment, the subject treated in accordance with the methods provided herein expresses DNMT3A with one or more mutations. In a specific embodiment, the subject treated in accordance with the methods provided herein expresses TET2 with one or more mutations. In a specific embodiment, the subject treated in accordance with the methods provided herein expresses SETBP1 with one or more mutations.

In certain embodiments, the subject treated in accordance with the methods provided herein *(see* Section 6.3) has thrombocytopenia. In certain embodiments, the subject treated in accordance with the methods provided herein has less than 1 x 10¹¹ platelets per liter. In certain embodiments, the subject treated in accordance with the methods provided herein has neutropenia. In certain embodiments, the subject treated in accordance with the methods provided herein has an absolute neutrophil count of less than 1 x 10⁹ per liter.

In certain embodiments, the subject treated in accordance with the methods provided herein has less than 13,000 white blood cells per µL, less than 12,000 white blood cells per µL, less than 11,000 white blood cells per µL, less than 10,000 white blood cells per µL, less than 7,500 white blood cells per µL, or less than 500 white blood cells per µL.

In certain embodiments, hemoglobin levels in the subject treated in accordance with the methods provide herein are less than 10 g/dL, 9 g/dL, 8 g/dL, or 7 g/dL. In certain embodiments, hemoglobin levels in the subject treated in accordance with the methods provided herein are between 7 g/dL and 7.5 g/dL, between 7.5 g/dL and 8 g/dL, between 8 g/dL and 8.5 g/dL, between 8.5 g/dL and 9.0 g/dL, between 9.0 g/dL and 9.5 g/dL, or between 9.5 g/dL and 10.0 g/dL.

In certain embodiments, the subject treated in accordance with the methods provided herein (*see* Section 6.3) has a low transfusion burden. In certain embodiments, the subject with a low transfusion burden treated in accordance with the methods provided herein requires at most 0, 1, 2, or 3 units of red blood cells per 8 weeks. In certain embodiments, the subject treated in accordance with the methods provided herein (*see* Section 6.3) has a high transfusion burden. In certain embodiments, the subject with a high transfusion burden treated in accordance with the methods provided herein requires at least 4, 5, 6, 7, 8, 9, 10, 11, 12, or 13 units of red blood cells per 8 weeks.

In certain embodiments, the subject treated in accordance with the methods provided herein has no response, a loss of response, or low chance of response to one or more ESAs.

In certain embodiments, the subject treated in accordance with the methods provided herein (*see* Section 6.3) has undergone prior treatment with one or more ESAs or is currently undergoing treatment with one or more ESAs. In certain embodiments, the subject treated in accordance with the methods provided herein has undergone prior treatment with hypomethylating agents. In certain embodiments, the subject treated in accordance with the methods provided herein has undergone prior treatment with lenalidomine. In certain embodiments, the subject treated in accordance with the methods provided herein has not undergone treatment with azacitidine, decitabine, ESA, G-CSF, GM-CSG, or lenalidomide. In certain embodiments, the subject treated in accordance with the methods provided herein does not respond to treatment with one or more ESAs. In certain embodiments, the subject treated in accordance with the methods provided herein is refractory to treatment with one or more ESAs. In certain embodiments, the subject treated in accordance with the methods provided herein becomes refractory to treatment with one or more ESAs. In certain embodiments, the subject treated in accordance with the methods provided herein is refractory to prior ESA treatment. In certain embodiments, the subject treated in accordance with the methods provided herein is a subject who is refractory to prior ESA treatment has documented non-response or response that is no longer maintained to prior ESA-containing regimen, either as single agent or combination (*e.g.*, with G-CSF); the ESA regimen must have been either (a) recombinant human erythropoietin of greater than 40,000 IU/week for at least 8 doses or equivalent, or (b) darbepoetin alpha of greater than 500 µg once every three weeks for at least 4 doses or equivalent. In certain embodiments, the subject treated in accordance with the methods provided herein is intolerant to prior ESA-treatment. In certain embodiments, the subject treated in accordance with the methods provided herein is a subject who is intolerant to prior ESA-treatment has documented discontinuation of prior ESA-containing regimen, either as single agent or combination (e.g., with G-CSF), at any time after introduction due to intolerance or an adverse event. In certain embodiments, the subject treated in accordance with the methods provided herein is ESA-ineligible. In certain embodiments, the subject treated in accordance with the methods provided herein is a subject who is ESA-ineligible has a low chance of response to ESA based on an endogenous serum erythropoietin level of greater than 200 U/L for subjects not previously treated with ESAs.

In certain embodiments, the subject treated in accordance with the methods described herein *(see* Section 6.3) has MDS. In certain embodiments, the subject treated in accordance with the methods described herein has MDS and intact chromosome 5q. In certain embodiments, the subject treated in accordance with the methods provided herein has MDS, intact chromosome 5q, and does not have documented treatment failure with lenalidomide. In certain embodiments, the subject treated in accordance with the methods provided herein has MDS, intact chromosome 5q, and documented treatment failure with lenalidomide. In certain embodiments, the subject treated in accordance with the methods described herein has MDS with chromosome 5q deletion. MDS with chromosome 5q deletion comprises a deletion of the long arm of chromosome 5 and is characterized by, *inter alia*, macrocytic anemia with oval macrocytes, normal to slightly reduced white blood cell counts, normal to elevated platelet counts, and less than 5% blasts in the bone marrow and blood. In certain embodiments, the subject treated in accordance with the methods provided herein has MDS with chromosome 5q deletion and does not have documented treatment failure with lenalidomide. In certain embodiments, the subject treated in accordance with the methods provided herein has MDS with chromosome 5q deletion and documented treatment failure with lenalidomide. In certain embodiments, treatment failure with lenalidomide comprises loss of response to lenalidomide, no response to lenalidomide after 4 months of treatment with lenalidomide, intolerance to treatment with lenalidomide, or cytopenia precluding treatment with lenalidomide.

### 6.6 Pharmaceutical Compositions

In certain embodiments, the ActRIIB ligand traps and the mTOR inhibitors are formulated suitable for combination treatment described herein.

In certain embodiments, the ActRIIB ligand trap and the mTOR inhibitor are formulated together with a pharmaceutically acceptable carrier for use with the methods described herein.

In certain embodiments, the ActRIIB ligand traps and the mTOR inhibitors are formulated together for parenteral administration.

In certain embodiments, the ActRIIB ligand traps and the mTOR inhibitors are formulated together for subcutaneous administration.

In certain embodiments, the ActRIIB ligand traps are formulated with a pharmaceutically acceptable carrier for use with the methods described herein.

In a preferred embodiment, the ActRIIB ligand trap is formulated for subcutaneous administration.

In another preferred embodiment, the ActRIIB ligand trap is packaged in a container as a sterile, preservative-free lyophilized powder or cake. In certain embodiments, the container comprises 25 mg of the ActRIIB ligand trap. In certain embodiments, the container comprising 25 mg of the ActRIIB ligand trap comprises a total of 37.5 mg of protein. In certain embodiments, ActRIIB ligand trap in the container comprising 25 mg of the ActRIIB ligand trap is reconstituted with 0.68 mL of water for injection. In certain embodiments, the container comprises 75 mg of the ActRIIB ligand trap. In certain embodiments, the container comprising 75 mg of the ActRIIB ligand trap comprises a total of 87.5 mg of protein. In certain embodiments, ActRIIB ligand trap in the container comprising 75 mg of the ActRIIB ligand trap is reconstituted with 1.6 mL of water for injection. In certain embodiments, the ActRIIB ligand trap in the container is reconstituted with a volume of water for injection, such that the final concentration of the reconstituted ActRIIB ligand trap in the water for injection is 50 mg/mL with a pH of approximately 6.5. In certain embodiments, the container is stored at between 2°C and 8°C. In certain embodiments, the container is a 3 mL glass vial with a gray butyl coated stopper.

In certain embodiments, the therapeutic methods provided herein include administering the composition (comprising an ActRIIB ligand trap) systemically, or locally as an implant or device. When administered, the therapeutic composition for uses provided herein is in a pyrogen-free, physiologically acceptable form. Therapeutically useful agents other than the ActRIIB ligand trap which may also optionally be included in the composition as described above, may be administered simultaneously or sequentially with the subject compounds (*e.g*., ActRIIB ligand trap, *see* Section 6.7)).

In certain embodiments, the ActRIIB ligand trap is administered parenterally. In a preferred embodiment, the ActRIIB ligand trap will be administered subcutaneously. Pharmaceutical compositions suitable for parenteral administration may comprise one or more ActRIIB polypeptides in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions for use in the methods described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The compositions described herein may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

It is understood that the dosage regimen will be determined by the attending physician considering various factors which modify the action of the compounds described herein (*e.g.*, an ActRIIB ligand trap (see, Section 6.7)).

In certain embodiments, the ActRIIB ligand trap is substantially pure in a pharmaceutical composition. Specifically, at most 20%, 10%, 5%, 2.5%, 1%, 0.1%, or at most 0.05% of the compounds in the pharmaceutical composition are compounds other than the ActRIIB ligand trap and the pharmaceutical acceptable carrier.

In certain embodiments, the mTOR inhibitors (*e.g*. rapamycin) are formulated with a pharmaceutically acceptable carrier for use with the methods described herein.

In a preferred embodiment, the mTOR inhibitor (*e.g*. rapamycin) is formulated for oral administration.

In certain embodiments, the mTOR inhibitor (*e.g*. rapamycin) is formulated for parental administration. In another preferred embodiment, the mTOR inhibitor (*e.g*. rapamycin) is packaged in a container as a sterile, preservative-free lyophilized powder or cake.

In certain embodiments, the mTOR inhibitor (*e.g*. rapamycin) is in the form of a capsule or tablet. In certain embodiments, the mTOR inhibitor (*e.g*. rapamycin) is in the form of microtablets or micropellets, and wherein the microtablets or micropellets are enterically coated. In certain embodiments, the microtablets or micropellets are contained in a capsule.

### 6.7 ActRIIB Ligand Trap

In certain embodiments, the ActRIIB ligand traps described in this Section can be used in the methods provided herein (*see*, Section 6.3). In certain embodiments, the ActRIIB ligand trap for use with the present methods comprises an amino acid sequence of SEQ ID NO:11. In certain embodiments, the ActRIIB ligand trap for use with the present methods is a product resulting from expression from an opening reading frame with the nucleotide sequence of SEQ ID NO:34 or a degenerate version of SEQ ID NO:34 that encodes SEQ ID NO:11.

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms of the receptor. Members of the ActRIIB family are generally transmembrane proteins, composed of a ligand-binding extracellular domain with a cysteine-rich region, a transmembrane domain, and a cytoplasmic domain with predicted serine/threonine kinase activity.

ActRIIB ligand traps to be used in the compositions and methods described herein include, without limitation, activin-binding soluble ActRIIB polypeptides; antibodies that bind to activin (particularly the activin A or B subunits, also referred to as betaA or betaB) and disrupt ActRIIB binding; antibodies that bind to ActRIIB and disrupt activin binding; non-antibody proteins selected for activin or ActRIIB binding; and randomized peptides selected for activin or ActRIIB binding, which can be conjugated to an Fc domain.

In certain embodiments, two or more different proteins (or other moieties) with activin or ActRIIB binding activity, especially activin binders that block the type I (*e.g.*, a soluble type I activin receptor) and type II (*e.g.*, a soluble type II activin receptor) binding sites, respectively, may be linked together to create a bifunctional or multifunctional binding molecule that inhibits ActRIIB and thus can be used in the compositions and methods described herein include. In certain embodiments, Activin-ActRIIB signaling axis antagonists that inhibit ActRIIB include nucleic acid aptamers, small molecules and other agents are used in the compositions and methods described herein include.

Such ActRIIB ligand traps can be generated and modified as previously described in Section 5.5.2 of International Publication No. WO 2014/066486, which is incorporated herein in its entirety.

### (a) ActRIIB Ligand Traps Comprising ActRIIB Antibodies

In certain embodiments, the ActRIIB ligand traps to be used in the compositions and methods described herein include antibodies that bind to activin (particularly the activin A or B subunits) and disrupt ActRIIB binding.

### (b) ActRIIB Ligand Traps Comprising ActRIIB Polypeptides

As used herein, the term "ActRIIB polypeptide" refers to polypeptides comprising any naturally occurring polypeptide of an ActRIIB family member as well as any variants thereof (including mutants, fragments, fusions, and peptidomimetic forms) that retain a useful activity. For example, ActRIIB polypeptides include polypeptides derived from the sequence of any known ActRIIB receptor having a sequence at least about 80% identical to the sequence of an ActRIIB polypeptide, and optionally at least 85%, 90%, 95%, 96%, 97%, 98%, 99% or greater identity. For example, an ActRIIB polypeptide may bind to and inhibit the function of an ActRIIB protein and/or activin. An example of an ActRIIB polypeptide includes the human ActRIIB precursor polypeptide (SEQ ID NO:2 or SEQ ID NO:14). With respect to the ActRIIB precursor polypeptide whose amino acid sequence is depicted as SEQ ID NO:2 or SEQ ID NO:14 (i.e., the human ActRIIB precursor polypeptide), the signal peptide of the ActRIIB precursor polypeptide is located at amino acids 1 to 18; the extracellular domain is located at amino acids 19 to 134 and the potential N-linked glycosylation sites are located at amino acid positions 42 and 65. The nucleic acid sequence encoding the human ActRIIB precursor polypeptide of SEQ ID NO:2 is disclosed as SEQ ID NO:5 (SEQ ID NO:5 provides an alanine at the codon corresponding to amino acid position 64, but could be readily modified by one of skill in the art using methods known in the art to provide an arginine at the codon corresponding to amino acid position 64 instead). See Table 1 for a description of the sequences.

The numbering of amino acids for all of the ActRIIB-related polypeptides described herein is based on the amino acid numbering for SEQ ID NO:2 and SEQ ID NO: 14 (which only differ in the amino acid expressed at position 64), unless specifically designated otherwise. For example, if an ActRIIB polypeptide is described as having a substitution/mutation at amino acid position 79, then it is to be understood that position 79 refers to the 79th amino acid in SEQ ID NO:2 or SEQ ID NO:14, from which the ActRIIB polypeptide is derived. Likewise, if an ActRIIB polypeptide is described as having an alanine or an arginine at amino acid position 64, then it is to be understood that position 64 refers to the 64th amino acid in SEQ ID NO:2 or SEQ ID NO:14, from which the ActRIIB polypeptide is derived.

In certain embodiments, the ActRIIB ligand traps used in the compositions and methods described herein comprise polypeptides comprising an activin-binding domain of ActRIIB. In certain embodiments, the activin-binding domains of ActRIIB comprise the extracellular domain of ActRIIB, or a portion thereof. In specific embodiments, the extracellular domain or portion thereof of ActRIIB is soluble. Illustrative modified forms of ActRIIB polypeptides are disclosed in U.S. Patent Application Publication Nos. 20090005308 and 20100068215, the disclosures of which are incorporated herein by reference in their entireties.

In specific embodiments, the ActRIIB ligand traps used in the compositions and methods described herein are soluble ActRIIB polypeptides. The term "soluble ActRIIB polypeptide" generally refers to polypeptides comprising an extracellular domain of an ActRIIB protein, including any naturally occurring extracellular domain of an ActRIIB protein as well as any variants thereof (including mutants, fragments and peptidomimetic forms). Soluble ActRIIB polypeptides can bind to activin; however, the wild type ActRIIB protein does not exhibit significant selectivity in binding to activin versus GDF8/11. In certain embodiments, altered forms of ActRIIB with different binding properties can be used in the methods provided herein. Such altered forms are disclosed, *e.g.*, in international patent application publication Nos. WO 2006/012627 and WO 2010/019261, the disclosures of which are incorporated herein by reference in their entireties. Native or altered ActRIIB proteins may be given added specificity for activin by coupling them with a second, activin-selective binding agent. Exemplary soluble ActRIIB polypeptides include the extracellular domain of a human ActRIIB polypeptide (*e.g*., SEQ ID NOs: 3, 4, 9, 12, 13, 15, 16, 17, 18, 19, 22, 23, 28, and 29).

An Fc fusion protein having the ActRIIB extracellular sequence disclosed by Hilden et al. (Blood, 1994, 83(8):2163-70), which has an alanine at the position corresponding to amino acid 64 of the ActRIIB precursor amino acid sequence, i.e., SEQ ID NO:2 (herein referred to as "A64"), has been demonstrated to possess a relatively low affinity for activin and GDF-11. By contrast, an Fc fusion protein with an arginine at position 64 of the ActRIIB precursor amino acid sequence (herein referred to as "R64") has an affinity for activin and GDF-11 in the low nanomolar to high picomolar range (see, *e.g.*, U.S. Patent Application Publication No. 20100068215, the disclosure of which is herein incorporated in its entirety). An ActRIIB precursor amino acid sequence with an arginine at position 64 is presented in SEQ ID NO:14. As such, in certain embodiments, the ActRIIB polypeptides used in accordance with the compositions and methods described herein may comprise either (i) an alanine at the position corresponding to amino acid 64 of the ActRIIB precursor amino acid sequence, i.e., SEQ ID NO:2; or (ii) an arginine at position 64 of the ActRIIB precursor amino acid sequence, i.e., SEQ ID NO: 14. In other embodiments, the ActRIIB polypeptides used in accordance with the compositions and methods described herein may comprise an amino acid that is not alanine or arginine at the position corresponding to amino acid 64 of the ActRIIB precursor amino acid sequence, i.e., SEQ ID NO: 2 or SEQ ID NO:14.

It has been shown that a deletion of the proline knot at the C-terminus of the extracellular domain of ActRIIB reduces the affinity of the receptor for activin (see, *e.g.*, Attisano et al., Cell, 1992, 68(1):97-108). An ActRIIB-Fc fusion protein containing amino acids 20-119 of SEQ ID NO: 14 (i.e., SEQ ID NO:18), "ActRIIB(20-119)-Fc" has reduced binding to GDF-11 and activin relative to an ActRIIB-Fc fusion protein containing amino acids 20-134 of SEQ ID NO: 14 (i.e., SEQ ID NO:17), "ActRIIB(20-134)-Fc", which includes the proline knot region and the complete juxtamembrane domain. However, an ActRIIB-Fc fusion protein containing amino acids 20-129 of SEQ ID NO: 14, "ActRIIB(20-129)-Fc" retains similar but somewhat reduced activity relative to the non-truncated extracellular domain of ActRIIB, even though the proline knot region is disrupted. Thus, ActRIIB polypeptides comprising extracellular domains that stop at amino acid 134, 133, 132, 131, 130 and 129 of SEQ ID NO: 14 (or SEQ ID NO:2) are all expected to be active, but constructs stopping at amino acid 134 or 133 may be most active. Similarly, mutations at any of residues 129-134 are not expected to alter ligand binding affinity by large margins, as indicated by the fact that mutations of P129 and P130 of SEQ ID NO: 14 do not substantially decrease ligand binding. Therefore, the ActRIIB polypeptides used in accordance with the methods and compositions described herein may end as early as amino acid 109 (i.e., the final cysteine) of SEQ ID NO:14 (or SEQ ID NO:2), however, forms ending at or between amino acid positions 109 and 119 of SEQ ID NO:14 (or SEQ ID NO:2) are expected to have reduced ligand binding ability.

Amino acid 29 of SEQ ID NO:2 and SEQ ID NO:14 represents the initial cysteine in the ActRIIB precursor sequence. It is expected that an ActRIIB polypeptide beginning at amino acid 29 of the N-terminus of SEQ ID NO:2 or SEQ ID NO:14, or before these amino acid positions, will retain ligand binding activity. An alanine to asparagine mutation at position 24 of SEQ ID NO:2 or SEQ ID NO:14 introduces an N-linked glycosylation sequence without substantially affecting ligand binding. This confirms that mutations in the region between the signal cleavage peptide and the cysteine cross-linked region, corresponding to amino acids 20-29 of SEQ ID NO:2 or SEQ ID NO:14, are well tolerated. In particular, ActRIIB polypeptides beginning at amino acid position 20, 21, 22, 23 and 24 of SEQ ID NO:2 or SEQ ID NO:14 will retain activity, and ActRIIB polypeptides beginning at amino acid positions 25, 26, 27, 28 and 29 of SEQ ID NO:2 or SEQ ID NO:14 are also expected to retain activity. An ActRIIB polypeptide beginning at amino acid position 22, 23, 24 or 25 of SEQ ID NO:2 or SEQ ID NO:14 will have the most activity.

Taken together, the active portions (i.e., ActRIIB polypeptides) of the ActRIIB precursor protein (i.e., SEQ ID NO:2 or SEQ ID NO:14) to be used in accordance with the methods and compositions described herein will generally comprise amino acids 29-109 of SEQ ID NO:2 or SEQ ID NO:14, and such ActRIIB polypeptides may, for example, begin at a residue corresponding to any one of amino acids 19-29 of SEQ ID NO:2 or SEQ ID NO:14 and end at a position corresponding to any one of amino acids 109-134 of SEQ ID NO:2 or SEQ ID NO:14. Specific examples of ActRIIB polypeptides encompassed herein include those that begin at an amino acid position from 19-29, 20-29 or 21-29 of SEQ ID NO:2 or SEQ ID NO:14 and end at an amino acid position from 119-134, 119-133 or 129-134, 129-133 of SEQ ID NO:2 or SEQ ID NO:14. Other specific examples of ActRIIB polypeptides encompassed herein include those that begin at an amino acid position from 20-24 (or 21-24, or 22-25) of SEQ ID NO:2 or SEQ ID NO:14 and end at an amino acid position from 109-134 (or 109-133), 119-134 (or 119-133) or 129-134 (or 129-133) of SEQ ID NO:2 or SEQ ID NO:14. Variant ActRIIB polypeptides falling within these ranges are also contemplated, particularly those having at least 80%, 85%, 90%, 91%, 92%, 93%, 94%, 95%, 96%, 97%, 98%, or 99% sequence identity or sequence homology to the corresponding portion of SEQ ID NO:2 or SEQ ID NO:14.

In certain embodiments, the ActRIIB ligand traps used in the compositions and methods described herein comprise a truncated form of an extracellular domain of ActRIIB. The truncation can be at the carboxy terminus and/or the amino terminus of the ActRIIB polypeptide. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIB polypeptide extracellular domain. For example, truncated forms of ActRIIB include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:2 or SEQ ID NO:14.

Additional exemplary truncated forms of ActRIIB include (i) polypeptides beginning at amino acids at any of amino acids 21-29 of SEQ ID NO:2 or SEQ ID NO:14 (optionally beginning at 22-25 of SEQ ID NO:2 or SEQ ID NO:14) and ending at any of amino acids 109-134 of SEQ ID NO:2 or SEQ ID NO:14; (ii) polypeptides beginning at any of amino acids 20-29 of SEQ ID NO:2 or SEQ ID NO:14 (optionally beginning at 22-25 of SEQ ID NO:2 or SEQ ID NO:14) and ending at any of amino acids 109-133 of SEQ ID NO:2 or SEQ ID NO:14; (iii) polypeptides beginning at any of amino acids 20-24 of SEQ ID NO:2 or SEQ ID NO:14 (optionally beginning at 22-25 of SEQ ID NO:2 or SEQ ID NO:14) and ending at any of amino acids 109-133 of SEQ ID NO:2 or SEQ ID NO:14; (iv) polypeptides beginning at any of amino acids 21-24 of SEQ ID NO:2 or SEQ ID NO:14 and ending at any of amino acids 109-134 of SEQ ID NO:2 or SEQ ID NO:14; (v) polypeptides beginning at any of amino acids 20-24 of SEQ ID NO:2 or SEQ ID NO:14 and ending at any of amino acids 118-133 of SEQ ID NO:2 or SEQ ID NO:14; (vi) polypeptides beginning at any of amino acids 21-24 of SEQ ID NO:2 or SEQ ID NO:14 and ending at any of amino acids 118-134 of SEQ ID NO:2 or SEQ ID NO:14, (vii) polypeptides beginning at any of amino acids 20-24 of SEQ ID NO:2 or SEQ ID NO:14 and ending at any of amino acids 128-133 of SEQ ID NO:2 or SEQ ID NO:14; (viii) polypeptides beginning at any of amino acids 20-24 of SEQ ID NO:2 or SEQ ID NO:14 and ending at any of amino acids 128-133 of SEQ ID NO:2 or SEQ ID NO:14, (ix) polypeptides beginning at any of amino acids 21-29 of SEQ ID NO:2 or SEQ ID NO:14 and ending at any of amino acids 118-134 of SEQ ID NO:2 or SEQ ID NO:14; (x) polypeptides beginning at any of amino acids 20-29 of SEQ ID NO:2 or SEQ ID NO:14 and ending at any of amino acids 118-133 of SEQ ID NO:2 or SEQ ID NO:14; (xi) polypeptides beginning at any of amino acids 21-29 of SEQ ID NO:2 or SEQ ID NO:14 and ending at any of amino acids 128-134 of SEQ ID NO:2 or SEQ ID NO:14; and (xii) polypeptides beginning at any of amino acids 20-29 of SEQ ID NO:2 or SEQ ID NO:14 and ending at any of amino acids 128-133 of SEQ ID NO:2 or SEQ ID NO:14. In a specific embodiment, an ActRIIB polypeptides comprises, consists essentially of, or consists of, an amino acid sequence beginning at amino acid position 25 of SEQ ID NO:2 or SEQ ID NO:14 and ending at amino acid position 131 of SEQ ID NO:2 or SEQ ID NO:14. In another specific embodiment, an ActRIIB polypeptide consists of, or consists essentially of, the amino acid sequence of SEQ ID NO:3, 4, 9, 12, 13, 15, 16, 17, 18, 19, 22, 23, 28, or 29.

Any of the ActRIIB polypeptides used in the compositions and methods described herein may be produced as a homodimer. Any of the ActRIIB polypeptides used in the compositions and methods described herein may be formulated as a fusion protein having a heterologous portion that comprises a constant region from an IgG heavy chain, such as an Fc domain. Any of the ActRIIB polypeptides used in the compositions and methods described herein may comprise an acidic amino acid at the position corresponding to position 79 of SEQ ID NO:2 or SEQ ID NO:14, optionally in combination with one or more additional amino acid substitutions, deletions or insertions relative to SEQ ID NO:2 or SEQ ID NO:14.

In specific embodiments, the ActRIIB ligand traps used in the compositions and methods described herein comprise an extracellular domain of ActRIIB with one or more amino acid substitutions/mutations. Such an amino acid substitution/mutation can be, for example, an exchange from the leucine at amino acid position 79 of SEQ ID NO:2 or SEQ ID NO:14 to an acidic amino acid, such as aspartic acid or glutamic acid. For example, position L79 of SEQ ID NO:2 or SEQ ID NO:14 may be altered in ActRIIB extracellular domain polypeptides to confer altered activin-myostatin (GDF-11) binding properties. L79A and L79P mutations reduce GDF-11 binding to a greater extent than activin binding. L79E and L79D mutations retain GDF-11 binding, while demonstrating greatly reduced activin binding.

In certain embodiments, the ActRIIB ligand traps used in the compositions and methods described herein comprise a truncated form of an ActRIIB extracellular domain that also carries an amino acid substitution, *e.g*., an exchange from the leucine at amino acid position 79 of SEQ ID NO:2 or SEQ ID NO: 14 to an acidic amino acid, such as aspartic acid or glutamic acid. In a specific embodiment, the truncated form of an extracellular domain of ActRIIB polypeptide that also carries an amino acid substitution used in the compositions and methods described herein is SEQ ID NO:9. Forms of ActRIIB that are truncated and/or carry one or more amino acid substitutions can be linked to an Fc domain of an antibody as discussed above.

Functionally active fragments of ActRIIB polypeptides can be obtained, for example, by screening polypeptides recombinantly produced from the corresponding fragment of the nucleic acid encoding an ActRIIB polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIB protein or signaling mediated by activin.

In addition, functionally active variants of ActRIIB polypeptides can be obtained, for example, by screening libraries of modified polypeptides recombinantly produced from the corresponding mutagenized nucleic acids encoding an ActRIIB polypeptide. The variants can be produced and tested to identify those that can function as antagonists (inhibitors) of ActRIIB protein or signaling mediated by activin. In certain embodiments, a functional variant of the ActRIIB polypeptides comprises an amino acid sequence that is at least 75% identical to an amino acid sequence selected from SEQ ID NO:3, 4, 9, 12, 13, 15, 16, 17, 18, 19, 22, 23, 28, and 29. In certain embodiments, the functional variant has an amino acid sequence at least 80%, 85%, 90%, 95%, 96%, 97%, 98%, or 99% identical to an amino acid sequence selected from SEQ ID NO:3, 4, 9, 12, 13, 15, 16, 17, 18, 19, 22, 23, 28, and 29.

It has been demonstrated that the ligand binding pocket of ActRIIB is defined by residues Y31, N33, N35, L38 through T41, E47, E50, Q53 through K55, L57, H58, Y60, S62, K74, W78 through N83, Y85, R87, A92, and E94 through F101 of SEQ ID NO:2 or SEQ ID NO: 14. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB polypeptide for use in the methods and compositions described herein is one that comprises amino acids 29-109 of SEQ ID NO:2 or SEQ ID NO: 14, but optionally beginning at an amino acid position ranging from 20-24 or 22-25 of SEQ ID NO:2 or SEQ ID NO:14 and ending at an amino acid position ranging from 129-134 of SEQ ID NO:2 or SEQ ID NO: 14, and comprising no more than 1, 2, 5, or 15 conservative amino acid changes in the ligand binding pocket, and zero, one or more non-conservative alterations at amino acid positions 40, 53, 55, 74, 79 and/or 82 of SEQ ID NO:2 or SEQ ID NO:14 in the ligand binding pocket. Such an ActRIIB polypeptide may retain greater than 80%, 90%, 95% or 99% sequence identity or sequence homology to the sequence of amino acids 29-109 of SEQ ID NO:2 or SEQ ID NO: 14. Sites outside the binding pocket, at which variability may be particularly well tolerated, include the amino and carboxy termini of the extracellular domain of ActRIIB, and positions 42-46 and 65-73. An asparagine to alanine alteration at position 65 of SEQ ID NO:2 or SEQ ID NO:14 (N65A) actually improves ligand binding in the A64 background, and is thus expected to have no detrimental effect on ligand binding in the R64 background. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64.

In specific embodiments, the ActRIIB ligand traps used in the compositions and methods described herein comprise a conjugate/fusion protein comprising an extracellular domain (*e.g*., an activin-binding domain) of an ActRIIB receptor linked to an Fc portion of an antibody. Such conjugate/fusion proteins may comprise any of the ActRIIB polypeptides disclosed herein (e.g., any of SEQ ID NOs:3, 4, 9, 12, 13, 15, 16, 17, 18, 19, 22, 23, 28, and 29), any ActRIIB polypeptides known in the art, or any ActRIIB polypeptides generated using methods known in the art and/or provided herein.

In certain embodiments, the extracellular domain is linked to an Fc portion of an antibody via a linker, *e.g.*, a peptide linker. Exemplary linkers include short polypeptide sequences such as 2-10, 2-5, 2-4, 2-3 amino acid residues (*e.g*., glycine residues), such as, for example, a Gly-Gly-Gly linker. In a specific embodiment, the linker comprises the amino acid sequence Gly-Gly-Gly (GGG). In another specific embodiment, the linker comprises the amino acid sequence Thr-Gly-Gly-Gly (TGGG). Optionally, the Fc domain has one or more mutations at residues such as Asp-265, lysine 322, and Asn-434. In certain cases, the mutant Fc domain having one or more of these mutations (*e.g*., an Asp-265 mutation) has a reduced ability to bind to the Fcγ receptor relative to a wild-type Fc domain. In other cases, the mutant Fc domain having one or more of these mutations (*e.g*., an Asn-434 mutation) has an increased ability to bind to the MHC class I- related Fc-receptor (FcRN) relative to a wild-type Fc domain. Exemplary fusion proteins comprising a soluble extracellular domain of ActRIIB fused to an Fc domain are set forth in SEQ ID NOs:6, 7, 10, 11, 20, 21, 24, 25, 26, 27, 30, 32, and 33.

In a specific embodiment, the ActRIIB ligand traps used in the compositions and methods described herein comprise the extracellular domain of ActRIIB, or a portion thereof, linked to an Fc portion of an antibody, wherein said ActRIIB ligand trap comprises an amino acid sequence that is at least 75% identical to an amino acid sequence selected from SEQ ID NOs:6, 7, 10, 11, 20, 21, 24, 25, 26, 27, 30, 32, and 33. In another specific embodiment, the ActRIIB ligand traps used in the compositions and methods described herein comprise the extracellular domain of ActRIIB, or a portion thereof, linked to an Fc portion of an antibody, wherein said ActRIIB ligand trap comprises an amino acid sequence that is at least 80%, 85%, 90%, 95%, 96%, 97%, 98%, or 99% identical to an amino acid sequence selected from SEQ ID NOs: 6, 7, 10, 11, 20, 21, 24, 25, 26, 27, 30, 32, and 33.

In a specific embodiment, the ActRIIB ligand traps to be used in the compositions and methods described herein is a fusion protein between the extracellular domain of the human ActRIIB receptor and the Fc portion of IgG1. In another specific embodiment, the ActRIIB ligand trap to be used in the compositions and methods described herein is a fusion protein between a truncated extracellular domain of the human ActRIIB receptor and the Fc portion of IgG1. In another specific embodiment, the ActRIIB ligand trap to be used in the compositions and methods described herein is a fusion protein between a truncated extracellular domain of the human ActRIIB receptor and the Fc portion of IgG1, wherein the truncated extracellular domain of the human ActRIIB receptor possesses an amino acid substitution at the amino acid position corresponding to amino acid 79 of SEQ ID NO:2 or SEQ ID NO:14. In one embodiment, the amino acid substitution at the amino acid position corresponding to amino acid 79 of SEQ ID NO:2 or SEQ ID NO:14 is substitution of Leucine for Aspartic Acid (i.e., an L79D mutation).

In a specific embodiment, the ActRIIB ligand trap to be used in the compositions and methods described herein is SEQ ID NO:10 or 11, which represents a fusion protein between the extracellular domain of the human ActRIIB receptor and the Fc portion of IgG1, wherein said ActRIIB extracellular domain comprises amino acids 25-131 of SEQ ID NO:14 with an L79D mutation. The nucleic acid sequence encoding the ActRIIB-Fc fusion protein of SEQ ID NO:10 is presented in SEQ ID NO:31.

In another specific embodiment, the ActRII ligand trap to be used in the compositions and methods described herein is a polypeptide comprising: (i) a fragment of the extracellular domain of ActRIIB, wherein the fragment consists of the sequence of amino acid 25-131 of SEQ ID NO:14 and wherein the fragment carries the L79D amino acid substitution; (ii) a linker; and (iii) an Fc of an IgG.

In another specific embodiment, the ActRIIB ligand trap to be used in the compositions and methods described herein is SEQ ID NO:20 or 21, which represents a fusion protein between the extracellular domain of the human ActRIIB receptor and the Fc portion of IgG1, wherein said ActRIIB extracellular domain comprises amino acids 25-131 of SEQ ID NO:2 with an L79D mutation.

In specific embodiments, mutated ActRIIB polypeptides comprising the addition of a further N-linked glycosylation site (N-X-S/T) that increases the serum half-life of an ActRIIB-Fc fusion protein, relative to the ActRIIB(R64)-Fc form can be used in the methods and compositions described herein. In a specific embodiment, introduction of an asparagine at position 24 of SEQ ID NO:2 or SEQ ID NO:14 (A24N) results in the creation of an NXT sequence that confers a longer half-life. Other NX(T/S) sequences can be found at 42-44 (NQS) and 65-67 (NSS), although the latter may not be efficiently glycosylated with the R at position 64 (i.e., in R64 polypeptides). N-X-S/T sequences may be generally introduced at positions outside the ligand binding pocket of ActRIIB, which is detailed above. Particularly suitable sites for the introduction of non-endogenous N-X-S/T sequences include amino acids 20-29, 20-24, 22-25, 109-134, 120-134 or 129-134 of SEQ ID NO:2 or SEQ ID NO:14. N-X-S/T sequences may also be introduced into the linker between the ActRIIB sequence and the Fc or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E106N, R112N, G120N, E123N, P129N, A132N, R112S and R112T (with all amino acid positions corresponding to the positions they can be found in SEQ ID NO:2 or SEQ ID NO: 14). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T are encompassed herein. Likewise, in an A24N variant, an S26T alteration may be used. Accordingly, an ActRIIB polypeptide may include one or more additional, non-endogenous N-linked glycosylation consensus sequences.

In certain embodiments, the methods and compositions described herein use isolated or purified ActRIIB polypeptides, i.e., ActRIIB polypeptides which are isolated from, or otherwise substantially free of, other proteins can be used with the methods and compositions described herein. ActRIIB polypeptides will generally be produced by expression from recombinant nucleic acids.

In certain aspects, the ActRIIB polypeptides used in the methods and compositions described herein are encoded by isolated and/or recombinant nucleic acids, including fragments, functional variants and fusion proteins disclosed herein. For example, SEQ ID NO:5 encodes the naturally occurring human ActRIIB precursor polypeptide. The subject nucleic acids may be single-stranded or double stranded. Such nucleic acids may be DNA or RNA molecules. These nucleic acids may be used, for example, in methods for making ActRIIB polypeptides or as direct therapeutic agents (*e.g.*, in a gene therapy approach).

In certain aspects, the nucleic acids that can be used to produce ActRIIB polypeptides suitable for use in the methods and compositions described herein are further understood to include nucleic acids that are variants of SEQ ID NO:5as well as variants of those nucleic acid sequences that encode soluble ActRIIB polypeptides (*e.g*., nucleic acids that encode SEQ ID NOs: 3, 4, 9, 12, 13, 15, 16, 17, 18, 19, 22, 23, 28, and 29). Variant nucleotide sequences include sequences that differ by one or more nucleotide substitutions, additions or deletions, such as allelic variants.

In certain embodiments, the isolated or recombinant nucleic acid sequences that can be used to produce ActRIIB polypeptides suitable for use in the methods and compositions described herein are at least 80%, 85%, 90%, 95%, 97%, 98%, 99% or 100% identical to SEQ ID NO:5 or those nucleic acid sequences that encode soluble ActRIIB polypeptides (e.g., nucleic acids that encode SEQ ID NOs: 3, 4, 9, 12, 13, 15, 16, 17, 18, 19, 22, 23, 28, and 29). One of ordinary skill in the art will appreciate that nucleic acid sequences complementary to SEQ ID NO:5 or those nucleic acid sequences that encode soluble ActRIIB polypeptides (e.g., nucleic acids that encode SEQ ID NOs: 3, 4, 9, 12, 13, 15, 16, 17, 18, 19, 22, 23, 28, and 29), and variants of SEQ ID NO:5 or those nucleic acid sequences that encode soluble ActRIIB polypeptides (e.g., nucleic acids that encode SEQ ID NOs: 3, 4, 9, 12, 13, 15, 16, 17, 18, 19, 22, 23, 28, and 29) can be used with the methods and compositions described herein. In further embodiments, the nucleic acid sequences can be isolated, recombinant, and/or fused with a heterologous nucleotide sequence, or in a DNA library.

### 6.8 mTOR inhibitors

In certain embodiments, the mTOR inhibitor is rapamycin. In certain embodiments, the mTOR inhibitor is a pharmaceutically acceptable salt or hydrate of rapamycin. In certain embodiments, the mTOR inhibitor is deforolimus. In certain embodiments, the mTOR inhibitor is a pharmaceutically acceptable salt or hydrate of deforolimus. In certain embodiments, the mTOR inhibitor is everolimus. In certain embodiments, the mTOR inhibitor is a pharmaceutically acceptable salt or hydrate of everolimus. In certain embodiments, the mTOR inhibitor is temsirolimus. In certain embodiments, the mTOR inhibitor is a pharmaceutically acceptable salt or hydrate of temsirolimus. In certain embodiments, the mTOR inhibitor is ridaforolimus. In certain embodiments, the mTOR inhibitor is a pharmaceutically acceptable salt or hydrate of ridaforolimus. In certain embodiments, the mTOR inhibitor is tacrolimus (FK-506). In certain embodiments, the mTOR inhibitor is a pharmaceutically acceptable salt or hydrate of tacrolimus (FK-506). In certain embodiments, the mTOR inhibitor is zotarolimus (ABT-578). In certain embodiments, the mTOR inhibitor is a pharmaceutically acceptable salt or hydrate of zotarolimus (ABT-578).

In certain embodiments, the mTOR inhibitor is a non-rapamycin analog mTOR inhibiting compound. In certain embodiments, the mTOR inhibitor is a pharmaceutically acceptable salt or hydrate of a non-rapamycin analog mTOR inhibiting compound. In certain embodiments, the non-rapamycin analog mTOR inhibiting compounds including, but not limited to, 3,3-Diindolylmethane (DIM), 32 deoxy-rapamycin (SAR943), 3-Methyladenine, Arenobufagin, AZD 3147, AZD-2014 (Vistusertib), AZD8055, BC-LI-0186, BEZ235 (Dactolisib), Bimiralisib (PQR309), Caffeine, CC-115, CC-223 (Onatasertib), Chrysophanic acid (Chrysophanol), Ciclopirox Olamine, CID3528206, CIDD 0067106, Compound 401 (*see*, *e.g.*, Griffen et al., 2005, J. Med. Chem. 48:569), Curcumin, CZ415, eCF 309, epigallocatechin gallate (EGCG), ETP 45658, ETP-46464, GDC-0084, GDC-0349, GDC-0980 (Apitolisib, RG7422), Genistein, GNE-477, GNE-493, GSK1059615, GSK-2126458 (Omipalisib), ICSN3250, INK-128 (MLN0128, Sapanisertib), KU-0063794, LY3023414 (Samotolisib), ME-344 (NV-344), Metformin, MHY1485, MTI-31 (LXI-15029), mTOR inhibitor 10 (CAS No.: 1222999-54-3; *see*, *e.g.*, Liu et al., 2011, Bioorg. Med. Chem. Lett. 21(13):4036-4040), mTOR/HDAC1-IN-12l ((R)-N-(4-(1-(7-(hydroxyamino)-7-oxoheptyl)-4-morpholino-1H-pyrazolo[3,4-d]pyrimidin-6-yl)phenyl)-2-methylmorpholine-4-carboxamide), mTORC1-IN-1 (2-(4-((2,4,6-Trimethyl-3-(4-(3-(methylsulfonyl)pyridin-2-yl)piperazine-1-carbonyl)phenyl)amino)piperidin-1-yl)benzonitrile), mTOR-IN-1 (CAS No.: 1207358-59-5), mTOR-IN-17 ((E)-6-(Phenyldiazenyl)-1H-dipyrazolo[1,5-a:4',3'-e]pyrimidine-3,4,7-triamine), Niclosamide, NV-128 (LY-303511), NVP-BGT226, Omipalisib, OSI-027, OSU-53, Palomid 529 (P529), Perifosine, PF 05212384, PF-04691502, PF-04979064, PI 103 hydrochloride, PKI-402, PKI-587 (Gedatolisib), PP 121, PP-242 (Torkinib), PP30, PQR-620, QL-IX-55, Rapalink, Resveratrol, SAR245409, LY294002, wortmannin, quercetin, myricetin, staurosporine, SF1126, SF2523, STK16-IN-1, Sunitinib, Torin 1, Torin 2, Triacetyl aloe-emodin, VS-5584, WAY-600, WJD008, WYE-125132 (WYE-132), WYE-354, WYE-687, XL388, XL-765 (Voxtalisib, SAR245409), (+)-Usnic acid, Arnicolide D, BGT226 maleate (NVP-BGT226 maleate), Cbz-B3A, CC-115 hydrochloride, Cyclovirobuxine D, Dihydromyricetin (Ampelopsin or Ampeloptin), FT-1518, GNE-317, Hederacolchiside A1, JR-AB2-011, KU-0060648, NSC781406, PI-103, Polyphyllin I, Pomiferin (NSC 5113), PQR-530, Rheb inhibitor NR1, Rotundic acid, or WYE-687 dihydrochloride .

In certain embodiments, the mTOR inhibitor is selected from the group consisting of rapamycin, deforolimus, everolimus, temsirolimus, ridaforolimus, tacrolimus (FK-506), zotarolimus (ABT-578), 3,3-Diindolylmethane (DIM), 32 deoxy-rapamycin (SAR943), 3-Methyladenine, Arenobufagin, AZD 3147, AZD-2014 (Vistusertib), AZD8055, BC-LI-0186, BEZ235 (Dactolisib), Bimiralisib (PQR309), Caffeine, CC-115, CC-223 (Onatasertib), Chrysophanic acid (Chrysophanol), Ciclopirox Olamine, CID3528206, CIDD 0067106, Compound 401, Curcumin, CZ415, eCF 309, epigallocatechin gallate (EGCG), ETP 45658, ETP-46464, GDC-0084, GDC-0349, GDC-0980 (Apitolisib, RG7422), Genistein, GNE-477, GNE-493, GSK1059615, GSK-2126458 (Omipalisib), ICSN3250, INK-128 (MLN0128, Sapanisertib), KU-0063794, LY3023414 (Samotolisib), ME-344 (NV-344), Metformin, MHY1485, MTI-31 (LXI-15029), mTOR inhibitor 10 (CAS No.: 1222999-54-3), mTOR/HDAC1-IN-12l, mTORC1-IN-1, mTOR-IN-1, mTOR-IN-17, Niclosamide, NV-128 (LY-303511), NVP-BGT226, Omipalisib, OSI-027, OSU-53, Palomid 529 (P529), Perifosine, PF 05212384, PF-04691502, PF-04979064, PI 103 hydrochloride, PKI-402, PKI-587 (Gedatolisib), PP 121, PP-242 (Torkinib), PP30, PQR-620, QL-IX-55, Rapalink, Resveratrol, SAR245409, LY294002, wortmannin, quercetin, myricetin, staurosporine, SF1126, SF2523, STK16-IN-1, Sunitinib, Torin 1, Torin 2, Triacetyl aloe-emodin, VS-5584, WAY-600, WJD008, WYE-125132 (WYE-132), WYE-354, WYE-687, XL388, XL-765 (Voxtalisib, SAR245409), (+)-Usnic acid, Arnicolide D, BGT226 maleate (NVP-BGT226 maleate), Cbz-B3A, CC-115 hydrochloride, Cyclovirobuxine D, Dihydromyricetin (Ampelopsin or Ampeloptin), FT-1518, GNE-317, Hederacolchiside A1, JR-AB2-011, KU-0060648, NSC781406, PI-103, Polyphyllin I, Pomiferin (NSC 5113), PQR-530, Rheb inhibitor NR1, Rotundic acid, and WYE-687 dihydrochloride. In certain embodiments, the mTOR inhibitor is selected from the group consisting of rapamycin, deforolimus, everolimus, temsirolimus, ridaforolimus, tacrolimus (FK-506), and zotarolimus (ABT-578).

In certain embodiments, the mTOR inhibitor is an ATP competitive inhibitor.

### 6.9 Reference Population

In certain embodiments, the size of the reference population can be 1, 5, 10, 25, 50, 75, 100, 200, 250, 300, 400, 500, or 1000 individuals. In certain embodiments, the reference population consists of 1, 5, 10, 25, 50, 75, 100, 200, 250, 300, 400, 500, or 1000 individuals. In certain embodiments, the reference population consists of random volunteers. In certain embodiments, the reference population consists of healthy people. In certain embodiments, the reference population consists of people of the same age, weight, and/or gender as the patient population as described in Section 6.5. In certain embodiments, the reference population consists of people without thalassemia, alpha-thalassemia, beta-thalassemia, myelodysplastic syndromes (MDS), or non-proliferative chronic myelomonocytic leukemia (CMML). In certain embodiments, the reference population consists of people with thalassemia, alpha-thalassemia, beta-thalassemia, myelodysplastic syndromes (MDS), or non-proliferative chronic myelomonocytic leukemia (CMML).

### 7. EXAMPLES

### 7.1 Example 1. Rapamycin (Sirolimus) and Luspatercept Combination Experiments

### 7.1.1 Background

The two independent experiments were conducted to test whether the coadministration of rapamycin and luspatercept increases red blood cell and hemoglobin levels more than the administration of luspatercept alone. The two experiments were similar in design, except that wild-type female mice were used in Experiment 1 while beta-thalassemia model mice (9-18 week old female mice that are heterozygous mutant for the Hbb-b1 and Hbb-b2 genes) were used in Experiment 2. The experiment design is illustrated in FIG. 1.

### 7.1.2 Materials and Methods

### (a) Mice

All mice were purchased from the Jackson Laboratory. For experiment 1, 10-12 week old wild-type (C57BL/6J) female mice (Jackson Lab Stock# 000664) were used. For experiment 2, 9-18 week old *B6.129P2-Hbb-b1^{tmlUnc} Hbb-b2^{tmlUnc}* /*J* female mice on 000664 C57BL/6J background (Jackson Lab Stock# 002683, beta-thalassemia mouse model) were used.

### (b) Dosing

A murine version of luspatercept (RAP-536) in 1X Phosphate Buffered Saline (PBS) with a total volume of 150 µL and at 10 mg/kg dose was injected via sub-cutaneous (SC) route twice a week for two weeks. Rapamycin at 4 mg/kg dose in 1X PBS that contains 5% Tween 80, 5% PEG400, 4% Ethanol and in a total volume of 150 µL was injected via the intraperitoneal route everyday, except for Sundays, for two weeks. For control group, 150 µL 1X PBS (Vehicle 1) was injected via sub-cutaneous (SC) route twice a week for two weeks and 1X PBS that contains 5% Tween 80, 5% PEG400, 4% Ethanol (Vehicle 2) solutions in 150 µL volume were injected via the intraperitoneal route everyday, except for Sundays, for two weeks. In both experiments, mice were divided into 4 groups. Group 1 received Vehicle 1 (SC) + Vehicle 2 (IP) injections, group 2 received RAP-536 (Drug 1) (SC) + Vehicle 2 (IP) injections, group 3 received Vehicle 1 (SC) + rapamycin (Drug 2) (IP) injections and group 4 received RAP-536 (Drug 1) (SC) + rapamycin (Drug 2) (IP) injections.

### (c) Bone marrow and spleen suspensions and blood preparation

On day 15, one day after the final dosing, the mice were euthanized, and blood was collected via cardiac puncture and put immediately into K2EDTA blood collection tubes. Bone marrow was collected by flushing femurs and tibias from each mouse using a syringe with a needle and using 5 mL 1X HBSS (Ca and Mg free) that contains 2% heat inactivated bovine serum (Thermo Fisher Scientific). Spleens were removed and splenocytes were prepared by smashing spleens using a syringe plunger on a 70 µM cell strainer placed in a 50 mL conical tube. Both bone marrow and spleen suspensions were kept on ice until flow cytometry analysis was conducted.

### (d) Complete Blood Count (CBC) analysis

CBC, reticulocyte (retic) and CHr analyses were done on blood samples on the same day of blood collections using Siemens Advia 120 equipment.

### (e) Bone marrow, spleen and blood analysis by flow cytometry

2 million bone marrow and spleen cells from prepared cell suspensions and 5 million blood cells were stained for each flow cytometry analysis. Cells were first stained with thiazole orange (1:50,000 dilution of 10 mM stock) and Hoechst (1:1000 dilution of 5 mg/mL stock) in stain medium (HBSS with 2% heat inactivated bovine serum) for 45 minutes at room temperature. After a single wash with stain medium, cells were stained with the antibody cocktail for 20 minutes at 4 C°. The antibodies used were CD45 (clone 30-F11), Ter119, CD44 (clone IM7), CD71 (clone RI7217). All antibodies against surface antigens used in this study were purchased from Biolegend and Thermo Fisher Scientific. After antibody staining, cells were washed once with stain medium once. Sytox-AADvanced reagent (from Thermo Fisher Scientific) was used as viability dye by following the product protocol. LSR Fortessa Flow Cytometry Analyzer from BD Biosciences was used for flow cytometry analysis. FlowJo software from BD Biosciences was used to analyze the flow cytometry data. Statistical analysis was conducted by Microsoft Excel and GraphPad Prism software.

### 7.1.3 Detailed Protocol for Dosing

### (a) Experiment 1:

- 10-12 weeks old wild-type (C57BL/6J) mice (Jackson Lab Stock# 000664) were used.
- **Drug 1:** RAP-536 in 1X PBS: 10 mg/kg, Subcutaneous (SC), Twice a week starting from first day.
- **Vehicle 1:** 1X PBS
- **Drug 2:** Rapamycin in 1X PBS that contains 5% Tween 80, 5% PEG400, 4% Ethanol. 4 mg/kg dose, IP (4 mg/kg dose).
- **Vehicle 2:** 5% Tween 80, 5% PEG400, 4% Ethanol in 1X PBS
- **Treatment Groups:** C57BL/6J wild-type (wt) female mice (Jackson), 7 animals/group
   (1) Group 1: Vehicle 1 (SC) + Vehicle 2 (IP)
   (2) Group 2: Drug 1 (RAP-536) (SC) + Vehicle 2 (IP)
   (3) Group 3: Vehicle 1 (SC) + Drug 2 (rapamycin) (IP)
   (4) Group 4: Drug 1 (RAP-536) (SC) + Drug 2 (rapamycin) (IP)
- **Dosing and Harvest Schedule**
   (1) Day 1 (Tuesday):
      ▪ Measure body weights.
      ▪ Inject Vehicle 1 and Drug 1 (RAP-536) via SC to designated groups
      ▪ Inject Vehicle 2 and Drug 2 (rapamycin) via IP to designated groups
   (2) Days 2 and 3 (Wednesday and Thursday):
      ▪ Inject Vehicle-2 and Drug 2 (rapamycin) via IP to designated groups on each day
   (3) Day 4 (Friday):
      ▪ Inject Vehicle 1 and Drug 1 (RAP-536) via SC to designated groups
      ▪ Inject Vehicle 2 and Drug 2 (rapamycin) via IP to designated groups
   (4) Day 5 (Saturday):
      ▪ Inject Vehicle 2 and Drug 2 (rapamycin) via IP to designated groups
   (5) Day 7 (Monday):
      ▪ Inject Vehicle 2 and Drug 2 (rapamycin) via IP to designated groups
   (6) Day 8 (Tuesday):
      ▪ Inject Vehicle 1 and Drug 1 (RAP-536) via SC to designated groups
      ▪ Inject Vehicle 2 and Drug 2 (rapamycin) via IP to designated groups
   (7) Days 9 and 10 (Wednesday and Thursday):
      ▪ Inject Vehicle 2 and Drug 2 (rapamycin) via IP to designated groups on each day
   (8) Day 11 (Friday):
      ▪ Inject Vehicle 1 and Drug 1 (RAP-536) via SC to designated groups
      ▪ Inject Vehicle 2 and Drug 2 (rapamycin) via IP to designated groups
   (9) Day 12 (Saturday):
      ▪ Inject Vehicle-2 and Drug-2 (rapamycin) via IP to designated groups
   (10) Day 14 (Monday):
      ▪ Inject Vehicle-1 and Drug 1 (RAP-536) via SC to designated groups
      ▪ Inject Vehicle-2 and Drug-2 (rapamycin) via IP to designated groups
   (11) Day 15 (Tuesday):
      ▪ Euthanize mice and collect blood, bone marrow and spleen.
- **Tissue collection and analysis.**
   (1) Measure body weights.
   (2) Collect all blood into wide EDTA tubes via cardiac puncture. CBC-differential and Retic-IRF (immature reticulocyte fraction) and CHr analysis.
   (3) Collect spleen, measure spleen weight.
   (4) Flush bone marrows from femurs and tibia from both hind limbs using 5 mL HBSS+2%bovine for flow cytometry analysis.

### (b) Experiment 2:

- 9-18 week old *B6.129P2-Hbb-b1^{tmlUnc} Hbb-b2^{tmlUnc}*/*J* on 000664 C57BL/6J background (Jackson Lab Stock# 002683, beta-thalassemia mouse model) female mice were used.
- **Drug 1:** A murine version of luspatercept (RAP-536) in 1X PBS: 10 mg/kg, Subcutaneous (SC), Twice a week starting from first day.
- **Vehicle 1:** 1X PBS
- **Drug 2:** Rapamycin in 1X PBS that contains 5% Tween 80, 5% PEG400, 4% Ethanol. 4 mg/kg dose, IP (4 mg/kg dose).
- **Vehicle 2:** 5% Tween 80, 5% PEG400, 4% Ethanol in 1X PBS
- **Treatment Groups:** *B6.129P2-Hbb-b1^{tmlUnc} Hbb-b2^{tm1Unc}*/*J* female mice on 000664 C57BL/6J background (Jackson Lab Stock# 002683, beta-thalassemia mouse model, In these mice, both hemoglobin chain genes, Hbb-b1 and Hbb-b2 are knocked out), 8 animals/group. See, *e.g.,* Yang el al., 1995, Proc. Natl. Acad. Sci. 92(25):11608-12. The mice were distributed into each group equally based on age and body weight.
   (1) Group 1: Vehicle 1 (SC) + Vehicle 2 (IP)
   (2) Group 2: Drug 1 (RAP-536) (SC) + Vehicle 2 (IP)
   (3) Group 3: Vehicle 1 (SC) + Drug 2 (rapamycin) (IP)
   (4) Group 4: Drug 1 (RAP-536) (SC) + Drug 2 (rapamycin) (IP)
- **Dosing and Harvest Schedule:** same as Experiment 1
- **Tissue collection and analysis:** same as Experiment 1

### 7.1.4 Results

The changes of red blood cell (RBC) levels in the blood of wild-type mice (in Experiment 1) and of beta-thalassemia model mice (th3/+ mice; in Experiment 2) in the different treatment groups were analyzed. As shown in **FIGS. 2A** and **2B****,** the co-dosing of rapamycin and RAP-536 (group 4) increased the RBC levels in both wild-type mice and th3/+ mice more than the single agent of either RAP-536 or rapamycin (as compared to RAP-536 dosing only (group 2) and rapamycin dosing only (group 3)). The RAP-536 dosing alone and rapamycin dosing alone each increased RBC levels at similar levels. The percentage increase of RBC levels in th3/+ mice was greater than in wild-type mice (upon RAP-536 dosing, rapamycin dosing, as well as co-dosing of both). The RBC level in th3/+ mice reached the RBC level in wild-type mice upon administration of either RAP-536 or rapamycin alone, and exceeded the wild-type levels upon co-dosing of RAP-536 or rapamycin.

The changes of hemoglobin (HGB) levels in the blood of wild-type mice (in Experiment 1) and of beta-thalassemia model mice (th3/+ mice; in Experiment 2) in the different treatment groups were analyzed. As shown in **FIGS. 3A** and **3B****,** the co-dosing of rapamycin and RAP-536 (group 4) increased the HGB levels in both wild-type mice and th3/+ mice more than the single agent of either RAP-536 or rapamycin (as compared to RAP-536 dosing only (group 2) and rapamycin dosing only (group 3)). The percentage increase in HGB levels was similar to the percentage increase in RBC levels upon. HGB levels in th3/+ is about half of the HGB levels in wild-type mice. The co-dosing of RAP-536 and rapamycin increased the HGB levels in th3/+ mice by more than 40%, resulting in HGB levels that were still lower than the HGB levels in wild-type mice.

The changes of HCT levels in the blood of wild-type mice (in Experiment 1) and of th3/+ mice (in Experiment 2) in the different treatment groups were analyzed. As shown in **FIGS. 4A** and **4B****,** as compared to **FIGS. 2A-2B** and **FIGS. 3A-3B****,** the HCT percentage increase in th3/+ mice correlates with RBC and HGB increase. The HCT levels increase in wild-type mice was limited, as compared to in th3/+ mice (reaching a HCT level close to the HCT level in wild-type mice). Thus, the co-dosing of RAP-536 and rapamycin is very effective in increasing RBC, HGB and HCT levels, especially in th3/+ mice.

The changes of RBC cell size (MCV) in wild-type mice (in Experiment 1) and th3/+ mice (in Experiment 2) in the different treatment groups were analyzed. As shown in **FIGS. 5A** and **5B****,** RAP-536 dosing reduced MCV in wild-type mice but increased the already low MCV levels in th3/+ mice. The co-dosing of RAP-536 and rapamycin reduced MCV in wild-type mice.

The total amount of HGB/cell (MCH) in wild-type mice (in Experiment 1) and th3/+ mice (in Experiment 2) in the different treatment groups were analyzed. As showed in **FIGS. 6A** and **6B****,** MCH slightly decreased in wild-type mice but did not change th3/+ mice upon RAP-536 dosing. In th3/+ mice, the increase in RBC, HGB and HCT levels were similar causing no change in MCH values. It was observed that the MCH values were significantly lower in th3/+ mice as compared to in wild-type mice. The levels of HGB and RBC in th3/+ mice are lower than the levels of HGB and RBC wild-type mice, explaining why there is less total amount of HGB/cell (MCH) in th3/+ mice. MCH levels were higher upon rapamycin dosing but were lower upon RAP-536 dosing, which indicates different mechanisms of action and therefore potential for additive/synergistic effect.

The changes of the corpuscular HGB concentration (CHC) levels in the blood of wild-type mice (in Experiment 1) and of th3/+ mice (in Experiment 2) in the different treatment groups were analyzed. As shown in **FIGS. 7A** and **7B****,** the co-dosing of rapamycin and RAP-536 increased CHC in wild-type mice but decreased CHC in th3/+ mice. The reason why the CHC level increased in wild-type mice upon RAP-536 dosing (group 2) or upon the co-dosing (group 4) is probably because the MCV decreased (cells became smaller), resulting in higher HGB concentration in each cell (although less total HGB in each cell). In the end, the HCT level increase was less than the HGB level increase in wild-type mice upon RAP-536 dosing. MCV did not decrease after rapamycin dosing in wild-type mice. As shown in **FIG. 7B****,** in th3/+ mice, CHC levels decreased upon RAP-536 dosing (group 2) or upon the co-dosing (group 4) as MCV was increased (rectified) upon RAP-536 dosing in th3/+ mice. Thus, in can be concluded that RBC levels in th3/+ mice have less total HGB/cell because they are smaller, but the HGB concentration (CHC) in each RBC in th3/+ mice was similar to CHC the RBC in wild-type mice.

The changes of the red blood cell distribution width (RDW) in the blood of wild-type mice (in Experiment 1) and of th3/+ mice (in Experiment 2) in the different treatment groups were analyzed. As shown in **FIGS. 8A** and **8B****,** the co-dosing of rapamycin and RAP-536 significantly decreased the RDW in th3/+ mice. The RDW increased upon RAP-536 dosing (group 2) or upon the co-dosing (group 4) in wild-type mice. It was noted that the RDW was high in th3/+ mice as compared to the RDW in wild-type mice. In th3/+ mice, RDW is decreased in correlation with improvement of anemia by RAP-536 dosing (group 2) and the co-dosing of both (group 4).

The percentage and the absolute number changes of the reticulocytes in the blood of wild-type mice (Experiment 1) in the different treatment groups were analyzed. As shown in **FIGS. 9A** and **9B****,** RAP-536 dosing increased the reticulocyte levels in blood and rapamycin dosing reduced the reticulocyte levels in blood in wild-type mice. Reticulocyte percentage **(****FIG 9A****)** and absolute numbers **(****FIG. 9B****)** changed in the same way in blood in wild-type mice upon dosing. RAP-536 dosing increased and rapamycin dosing decreased reticulocyte levels in the blood in wild-type mice. The co-dosing of both normalized reticulocyte levels. This is another dataset that suggests that the mechanisms of action of luspatercept and rapamycin on red blood cell production are different and shows why the co-dosing exhibits an additive or synergistic effect (increasing the RBC or HGB levels more than a single agent dosing).

The percentage and the absolute number changes of the reticulocytes in the blood of th3/+ mice (Experiment 2) in the different treatment groups were analyzed. As shown in **FIGS. 10A** and **10B****,** the co-dosing of rapamycin and RAP-536 increased the reticulocyte numbers but not the reticulocyte percentage in blood in th3/+ mice. It was noted that the reticulocyte percentage and absolute numbers were much higher in th3/+ mice as compared to in wild-type mice. The reticulocyte absolute numbers increased significantly upon rapamycin dosing (group 3), upon RAP-536 dosing (group 2), and upon the co-dosing (group 4) in th3/+ mice. The fact that the reticulocyte absolute numbers in th3/+ mice increased after both RAP-536 dosing and rapamycin dosing suggests that reticulocyte survival was enhanced in th3/+ mice. More reticulocyte survival means more RBC production. If reticulocyte survival increases, RBC lifespan also probably increases. The reason the reticulocyte levels did not decrease even though the ineffective erythropoiesis was partially reduced in th3/+ mice is that these mice were still anemic. So, it reached a balance between the increased reticulocyte survival/less anemia ending up high reticulocyte levels after dosing.

The change of the reticulocyte hemoglobin levels (CHr) in the blood of wild-type mice (in Experiment 1) and of th3/+ mice (in Experiment 2) in the different treatment groups were studied. As shown in **FIGS. 11A** and **11B****,** the co-dosing of rapamycin and RAP-536 slightly reduced the reticulocyte hemoglobin levels in both wild-type mice and th3/+ mice. Note that the reticulocyte hemoglobin levels were already low in th3/+ mice. This is probably because of the presence of immature reticulocytes in blood.

The changes of the reticulocyte composition in blood of wild-type mice (in Experiment 1) and of th3/+ mice (in Experiment 2) in the different treatment groups were studied. **FIG. 12** shows an exemplary reticulocyte composition analyzed by an automated blood cell analyzer (e.g., by an ADIVA analyzer) in the reticulocyte channel. In the obtained exemplary reticulocyte scattered absorption graph (FIG. 12), H reticulocytes represent reticulocytes that have High RNA absorption levels (immature reticulocytes), M reticulocytes represent reticulocytes that have Medium RNA absorption levels, and L reticulocytes are reticulocytes that have Low RNA absorption levels (mature reticulocytes). *See e.g.*, Mori et al., 2003, Nephrology Dialysis Transplantation, 18(suppl 4):416). As shown in **FIGS. 13A** and **13B****,** the reticulocyte composition in blood in wild-type mice changed upon the dosing of rapamycin or RAP-536, or the co-dosing of both. Specifically, the H reticulocyte percentage increased and M reticulocyte percentage decreased upon RAP-536 dosing. Overall reticulocyte numbers increased upon RAP-536 dosing. RAP-536 dosing might induced more reticulocyte production and release from bone marrow and more reticulocyte maturation in blood. The H reticulocyte percentage decreased and the L and M reticulocyte percentages increased upon rapamycin dosing. Overall reticulocyte levels in blood also decreased. This could be due to reticulocytes maturing faster and/or an increased lifespan of RBCs. This is another dataset that suggests that the mechanisms of action of luspatercept and rapamycin on red blood cell production are different and shows why the co-dosing exhibits an additive or synergistic effect (increasing the RBC or HGB levels more than a single agent dosing).

**FIGS. 14A** and **14B** show that the reticulocyte composition in the blood in th3/+ mice changed upon the dosing of rapamycin or RAP-536, or the co-dosing of both. Specifically, the H reticulocyte percentage decreased upon the dosing of rapamycin or RAP-536, or the co-dosing of both in th3/+ mice. Rapamycin and RAP-536 co-dosing did not reduce the overall reticulocyte numbers in th3/+ mice. Yet, the H reticulocyte percentage decreased significantly, suggesting that reticulocyte maturation/survival was probably enhanced.

The changes of bodyweight of wild-type mice (in Experiment 1) and of th3/+ mice (in Experiment 2) in the different treatment groups were studied, as shown in **FIGS. 15A** and **15B****.** The changes of spleen size of wild-type mice (in Experiment 1) and of th3/+ mice (in Experiment 2) in the different treatment groups were studied. As shown in **FIGS. 16A** and **16B****,** the spleen size th3/mice were normalized upon the co-dosing of rapamycin and RAP-536. Therefore, the co-dosing not only reduced anemia, but it also reduced the enlarged spleen size in th3/+ mice. RAP-536 dosing slightly increased the spleen size and rapamycin dosing reduced spleen size in wild-type mice.

The changes of the erythroblast frequency in the bone marrow of wild-type mice (in Experiment 1) and of th3/+ mice (in Experiment 2) in the different treatment groups were studied. As shown by **FIGS. 17A** and **17B****,** the co-dosing of rapamycin and RAP-536 decreased the erythroblast frequency in the bone marrow in th3/+ mice. Specifically, the erythroblast /CD45(+) ratio in bone marrow was high in th3/+ mice and were normalized by the co-dosing of rapamycin and RAP-536. **FIGs. 18A** and **18B** demonstrate that more reticulocytes were produced and/or retained in the bone marrow in wild-type mice and th3/+ mice upon the co-dosing of rapamycin and RAP-536 than the dosing of either rapamycin or RAP-536 alone. The ratio of reticulocytes to erythroblast increased in wild-type mice and in th3/+ mice upon RAP-536 dosing and the co-dosing of rapamycin and RAP-536.

The changes of the erythropoiesis in spleen of wild-type mice (in Experiment 1) and of th3/+ mice (in Experiment 2) in the different treatment groups were also studied. As shown in **FIGS. 19A** and **19B****,** demonstrate that both RAP-536 dosing and the co-dosing of both increased the Ter119(+) cell frequency in spleen of both wild-type mice and th3/+ mice. **FIG. 20A** demonstrates that in wild-type mice RAP-536 dosing increased and rapamycin decreased the erythroblast/CD45(+) ratio in spleen. **FIGS. 21A** and **21B** demonstrate that the co-dosing of rapamycin and RAP-536 had milder direct effects on the erythropoiesis in spleen in th3/+ mice than in wild-type mice. The reticulocyte/erythroblast ratio in wild-type mice spleen was higher than in th3/+ mice spleen. The spleen size in th3/+ mice was reduced by rapamycin dosing and the co-dosing but not much by RAP-536 dosing as RAP-536 induced erythropoiesis in spleen. It was noted that, the reticulocyte/erythroblast ratio in the spleen of wild-type mice was much higher as compared to the reticulocyte/erythroblast ratio in th3/+ mice. This is because in wild-type mice the erythroblast levels are very low and reticulocyte wild-type spleen are not the ones that are produced there. They are there for a different reason.

### 8. DESCRIPTION OF THE SEQUENCES

**Table 1. Sequence Information.**

| **SEQ ID NO.** | **DESCRIPTION** | **SEQUENCE** |
|---|---|---|
| 1 | human ActRIIB soluble (extracellular), processed polypeptide sequence with the N-terminal 6 amino acids of the EC domain deleted and the C-terminal 4 amino acids of the EC domain deleted (amino acids 25-130 of SEQ ID NO:14) and with an L79D mutation | ETRECIYYNANWELERTNQSGLERCEGEQDKRLHCYAS WRNSSGTIELVKKGCWDDDFNCYDRQECVATEENPQVY FCCCEGNFCNERFTHLPEAGGPEVTYEPPP |
| 2 | human ActRIIB precursor protein sequence (A64) | MTAPWVALALLWGSLWPGSGRGEAETRECIYYNANWE LERTNQSGLERCEGEQDKRLHCYASWANSSGTIELVKK GCWLDDFNCYDRQECVATEENPQVYFCCCEGNFCNERF THLPEAGGPEVTYEPPPTAPTLLTVLAYSLLPIGGLSLIVL LAFWMYRHRKPPYGHVDIHEDPGPPPPSPLVGLKPLQLL EIKARGRFGCVWKAQLMNDFVAVKIFPLQDKQSWQSER EIFSTPGMKHENLLQFIAAEKRGSNLEVELWLITAFHDKG SLTDYLKGNIITWNELCHVAETMSRGLSYLHEDVPWCR GEGHKPSIAHRDFKSKNVLLKSDLTAVLADFGLAVRFEP GKPPGDTHGQVGTRRYMAPEVLEGAINFQRDAFLRIDM YAMGLVLWELVSRCKAADGPVDEYMLPFEEEIGQHPSL EELQEVVVHKKMRPTIKDHWLKHPGLAQLCVTIEECWD HDAEARLSAGCVEERVSLIRRSVNGTTSDCLVSLVTSVT NVDLPPKESSI |
| 3 | human ActRIIB soluble (extracellular), processed polypeptide sequence (amino acids 19-134 of SEQ ID NO:2) | SGRGEAETRECIYYNANWELERTNQSGLERCEGEQDKR LHCYASWANSSGTIELVKKGCWLDDFNCYDRQECVATE ENPQVYFCCCEGNFCNERFTHLPEAGGPEVTYEPPPTAPT |
| 4 | human ActRIIB soluble (extracellular), processed polypeptide sequence with the C-terminal 15 amino acids deleted (amino acids 19-119 of SEQ ID NO:2) | SGRGEAETRECIYYNANWELERTNQSGLERCEGEQDKR LHCYASWANSSGTIELVKKGCWLDDFNCYDRQECVATE ENPQVYFCCCEGNFCNERFTHLPEA |
| 5 | nucleic acid sequence encoding a human ActRIIB (A64) precursor protein | ATGACGGCGCCCTGGGTGGCCCTCGCCCTCCTCTGGG GATCGCTGTGGCCCGGCTCTGGGCGTGGGGAGGCTGA GACACGGGAGTGCATCTACTACAACGCCAACTGGGAG CTGGAGCGCACCAACCAGAGCGGCCTGGAGCGCTGCG AAGGCGAGCAGGACAAGCGGCTGCACTGCTACGCCTC CTGGGCCAACAGCTCTGGCACCATCGAGCTCGTGAAG AAGGGCTGCTGGCTAGATGACTTCAACTGCTACGATA GGCAGGAGTGTGTGGCCACTGAGGAGAACCCCCAGGT GTACTTCTGCTGCTGTGAAGGCAACTTCTGCAACGAGC GCTTCACTCATTTGCCAGAGGCTGGGGGCCCGGAAGT CACGTACGAGCCACCCCCGACAGCCCCCACCCTGCTC ACGGTGCTGGCCTACTCACTGCTGCCCATCGGGGGCCT TTCCCTCATCGTCCTGCTGGCCTTTTGGATGTACCGGC ATCGCAAGCCCCCCTACGGTCATGTGGACATCCATGA GGACCCTGGGCCTCCACCACCATCCCCTCTGGTGGGCC TGAAGCCACTGCAGCTGCTGGAGATCAAGGCTCGGGG GCGCTTTGGCTGTGTCTGGAAGGCCCAGCTCATGAAT GACTTTGTAGCTGTCAAGATCTTCCCACTCCAGGACAA GCAGTCGTGGCAGAGTGAACGGGAGATCTTCAGCACA CCTGGCATGAAGCACGAGAACCTGCTACAGTTCATTG CTGCCGAGAAGCGAGGCTCCAACCTCGAAGTAGAGCT GTGGCTCATCACGGCCTTCCATGACAAGGGCTCCCTCA CGGATTACCTCAAGGGGAACATCATCACATGGAACGA ACTGTGTCATGTAGCAGAGACGATGTCACGAGGCCTC TCATACCTGCATGAGGATGTGCCCTGGTGCCGTGGCG AGGGCCACAAGCCGTCTATTGCCCACAGGGACTTTAA AAGTAAGAATGTATTGCTGAAGAGCGACCTCACAGCC GTGCTGGCTGACTTTGGCTTGGCTGTTCGATTTGAGCC AGGGAAACCTCCAGGGGACACCCACGGACAGGTAGG CACGAGACGGTACATGGCTCCTGAGGTGCTCGAGGGA GCCATCAACTTCCAGAGAGATGCCTTCCTGCGCATTGA CATGTATGCCATGGGGTTGGTGCTGTGGGAGCTTGTGT CTCGCTGCAAGGCTGCAGACGGACCCGTGGATGAGTA CATGCTGCCCTTTGAGGAAGAGATTGGCCAGCACCCT TCGTTGGAGGAGCTGCAGGAGGTGGTGGTGCACAAGA AGATGAGGCCCACCATTAAAGATCACTGGTTGAAACA CCCGGGCCTGGCCCAGCTTTGTGTGACCATCGAGGAG TGCTGGGACCATGATGCAGAGGCTCGCTTGTCCGCGG GCTGTGTGGAGGAGCGGGTGTCCCTGATTCGGAGGTC GGTCAACGGCACTACCTCGGACTGTCTCGTTTCCCTGG TGACCTCTGTCACCAATGTGGACCTGCCCCCTAAAGA GTCAAGCATCTAA |
| 6 | fusion protein comprising a soluble extracellular domain of ActRIIB (A64; SEQ ID NO:3) fused to an Fc domain | SGRGEAETRECIYYNANWELERTNQSGLERCEGEQDKR LHCYASWANSSGTIELVKKGCWLDDFNCYDRQECVATE ENPQVYFCCCEGNFCNERFTHLPEAGGPEVTYEPPPTAPT GGGTHTCPPCPAPELLGGPSVFLFPPKPKDTLMISRTPEV TCVVVDVSHEDPEVKFNWYVDGVEVHNAKTKPREEQY NSTYRVVSVLTVLHQDWLNGKEYKCKVSNKALPVPIEK TISKAKGQPREPQVYTLPPSREEMTKNQVSLTCLVKGFY PSDIAVEWESNGQPENNYKTTPPVLDSDGSFFLYSKLTV DKSRWQQGNVFSCSVMHEALHNHYTQKSLSLSPGK |
| 7 | fusion protein comprising a soluble extracellular domain of ActRIIB (A64) with the C-terminal 15 amino acids deleted (SEQ ID NO:4) fused to an Fc domain | SGRGEAETRECIYYNANWELERTNQSGLERCEGEQDKR LHCYASWANSSGTIELVKKGCWLDDFNCYDRQECVATE ENPQVYFCCCEGNFCNERFTHLPEAGGGTHTCPPCPAPE LLGGPSVFLFPPKPKDTLMISRTPEVTCVVVDVSHEDPEV KFNWYVDGVEVHNAKTKPREEQYNSTYRVVSVLTVLH QDWLNGKEYKCKVSNKALPVPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGK |
| 8 | human ActRIIB soluble (extracellular), processed polypeptide sequence with the N-terminal 6 amino acids of the EC domain deleted and the C-terminal 5 amino acids of the EC domain deleted (amino acids 25-129 of SEQ ID NO:14) and with an L79D mutation | ETRECIYYNANWELERTNQSGLERCEGEQDKRLHCYAS WRNSSGTIELVKKGCWDDDFNCYDRQECVATEENPQVY FCCCEGNFCNERFTHLPEAGGPEVTYEPP |
| 9 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:14) and with an L79D mutation | ETRECIYYNANWELERTNQSGLERCEGEQDKRLHCYAS WRNSSGTIELVKKGCWDDDFNCYDRQECVATEENPQVY FCCCEGNFCNERFTHLPEAGGPEVTYEPPPT |
| 10 | Unprocessed ActRIIB-Fc fusion protein with the N-terminal 6 amino acids of the EC domain deleted and the C-terminal 3 amino acids of the EC domain deleted (amino acids 25-131 of SEQ ID NO:14) and with an L79D mutation and with TPA leader sequence | MDAMKRGLCCVLLLCGAVFVSPGAAETRECIYYNANW ELERTNQSGLERCEGEQDKRLHCYASWRNSSGTIELVKK GCWDDDFNCYDRQECVATEENPQVYFCCCEGNFCNERF THLPEAGGPEVTYEPPPTGGGTHTCPPCPAPELLGGPSVF LFPPKPKDTLMISRTPEVTCVVVDVSHEDPEVKFNWYVD GVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPP VLDSDGSFFLYSKLTVDKSRWQQGNVFSCSVMHEALHN HYTQKSLSLSPGK |
| 11 | Processed ActRIIB-Fc fusion protein with the N-terminal 6 amino acids of the EC domain deleted and the C-terminal 3 amino acids of the EC domain deleted (amino acids 25-131 of SEQ ID NO:14) and with an L79D mutation (Luspatercept) | ETRECIYYNANWELERTNQSGLERCEGEQDKRLHCYAS WRNSSGTIELVKKGCWDDDFNCYDRQECVATEENPQVY FCCCEGNFCNERFTHLPEAGGPEVTYEPPPTGGGTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSREEMTKNQVSLTCLVKGFYPSDIAVEWES NGQPENNYKTTPPVLDSDGSFFLYSKLTVDKSRWQQGN VFSCSVMHEALHNHYTQKSLSLSPGK |
| 12 | human ActRIIB soluble (extracellular), processed polypeptide sequence (amino acids 20-134 of SEQ ID NO:2) | GRGEAETRECIYYNANWELERTNQSGLERCEGEQDKRL HCYASWANSSGTIELVKKGCWLDDFNCYDRQECVATEE NPQVYFCCCEGNFCNERFTHLPEAGGPEVTYEPPPTAPT |
| 13 | human ActRIIB soluble (extracellular), processed polypeptide sequence with the C-terminal 15 amino acids deleted (amino acids 20-119 of SEQ ID NO:2) | GRGEAETRECIYYNANWELERTNQSGLERCEGEQDKRL HCYASWANSSGTIELVKKGCWLDDFNCYDRQECVATEE NPQVYFCCCEGNFCNERFTHLPEA |
| 14 | human ActRIIB precursor protein sequence (R64) | MTAPWVALALLWGSLWPGSGRGEAETRECIYYNANWE LERTNQSGLERCEGEQDKRLHCYASWRNSSGTIELVKKG CWLDDFNCYDRQECVATEENPQVYFCCCEGNFCNERFT HLPEAGGPEVTYEPPPTAPTLLTVLAYSLLPIGGLSLIVLL AFWMYRHRKPPYGHVDIHEDPGPPPPSPLVGLKPLQLLEI KARGRFGCVWKAQLMNDFVAVKIFPLQDKQSWQSEREI FSTPGMKHENLLQFIAAEKRGSNLEVELWLITAFHDKGS LTDYLKGNIITWNELCHVAETMSRGLSYLHEDVPWCRG EGHKPSIAHRDFKSKNVLLKSDLTAVLADFGLAVRFEPG KPPGDTHGQVGTRRYMAPEVLEGAINFQRDAFLRIDMY AMGLVLWELVSRCKAADGPVDEYMLPFEEEIGQHPSLE ELQEVVVHKKMRPTIKDHWLKHPGLAQLCVTIEECWDH DAEARLSAGCVEERVSLIRRSVNGTTSDCLVSLVTSVTN VDLPPKESSI |
| 15 | human ActRIIB soluble (extracellular), processed polypeptide sequence (amino acids 19-134 of SEQ ID NO:14) | SGRGEAETRECIYYNANWELERTNQSGLERCEGEQDKR LHCYASWRNSSGTIELVKKGCWLDDFNCYDRQECVATE ENPQVYFCCCEGNFCNERFTHLPEAGGPEVTYEPPPTAPT |
| 16 | human ActRIIB soluble (extracellular), processed polypeptide sequence with the C-terminal 15 amino acids deleted (amino acids 19-119 of SEQ ID NO:14) | SGRGEAETRECIYYNANWELERTNQSGLERCEGEQDKR LHCYASWRNSSGTIELVKKGCWLDDFNCYDRQECVATE ENPQVYFCCCEGNFCNERFTHLPEA |
| 17 | human ActRIIB soluble (extracellular), processed polypeptide sequence (amino acids 20-134 of SEQ ID NO:14) | GRGEAETRECIYYNANWELERTNQSGLERCEGEQDKRL HCYASWRNSSGTIELVKKGCWLDDFNCYDRQECVATEE NPQVYFCCCEGNFCNERFTHLPEAGGPEVTYEPPPTAPT |
| 18 | human ActRIIB soluble (extracellular), processed polypeptide sequence with the C-terminal 15 amino acids deleted (amino acids 20-119 of SEQ ID NO:14) | GRGEAETRECIYYNANWELERTNQSGLERCEGEQDKRL HCYASWRNSSGTIELVKKGCWLDDFNCYDRQECVATEE NPQVYFCCCEGNFCNERFTHLPEA |
| 19 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:2) and with an L79D mutation | ETRECIYYNANWELERTNQSGLERCEGEQDKRLHCYAS WANSSGTIELVKKGCWDDDFNCYDRQECVATEENPQV YFCCCEGNFCNERFTHLPEAGGPEVTYEPPPT |
| 20 | Unprocessed ActRIIB-Fc fusion protein with the N-terminal 6 amino acids of the EC domain deleted and the C-terminal 3 amino acids of the EC domain deleted (amino acids 25-131 of SEQ ID NO:2) and with an L79D mutation and with TPA leader sequence | MDAMKRGLCCVLLLCGAVFVSPGAAETRECIYYNANW ELERTNQSGLERCEGEQDKRLHCYASWANSSGTIELVKK GCWDDDFNCYDRQECVATEENPQVYFCCCEGNFCNERF THLPEAGGPEVTYEPPPTGGGTHTCPPCPAPELLGGPSVF LFPPKPKDTLMISRTPEVTCVVVDVSHEDPEVKFNWYVD GVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPP VLDSDGSFFLYSKLTVDKSRWQQGNVFSCSVMHEALHN HYTQKSLSLSPGK |
| 21 | Processed ActRIIB-Fc fusion protein with the N-terminal 6 amino acids of the EC domain deleted and the C-terminal 3 amino acids of the EC domain deleted (amino acids 25-131 of SEQ ID NO:2) and with an L79D mutation | ETRECIYYNANWELERTNQSGLERCEGEQDKRLHCYAS WANSSGTIELVKKGCWDDDFNCYDRQECVATEENPQV YFCCCEGNFCNERFTHLPEAGGPEVTYEPPPTGGGTHTC PPCPAPELLGGPSVFLFPPKPKDTLMISRTPEVTCVVVDV SHEDPEVKFNWYVDGVEVHNAKTKPREEQYNSTYRVVS VLTVLHQDWLNGKEYKCKVSNKALPAPIEKTISKAKGQ PREPQVYTLPPSREEMTKNQVSLTCLVKGFYPSDIAVEW ESNGQPENNYKTTPPVLDSDGSFFLYSKLTVDKSRWQQG NVFSCSVMHEALHNHYTQKSLSLSPGK |
| 22 | human ActRIIB soluble (extracellular), processed polypeptide sequence (amino acids 20-134 of SEQ ID NO:14) with L79D mutation | GRGEAETRECIYYNANWELERTNQSGLERCEGEQDKRL HCYASWRNSSGTIELVKKGCWDDDFNCYDRQECVATEE NPQVYFCCCEGNFCNERFTHLPEAGGPEVTYEPPPTAPT |
| 23 | human ActRIIB soluble (extracellular), processed polypeptide sequence (amino acids 20-134 of SEQ ID NO:2) with L79D mutation | GRGEAETRECIYYNANWELERTNQSGLERCEGEQDKRL HCYASWANSSGTIELVKKGCWDDDFNCYDRQECVATEE NPQVYFCCCEGNFCNERFTHLPEAGGPEVTYEPPPTAPT |
| 24 | human ActRIIB soluble (extracellular), processed polypeptide sequence (amino acids 20-134 of SEQ ID NO:14) with L79D mutation fused to an Fc domain with a GGG linker | GRGEAETRECIYYNANWELERTNQSGLERCEGEQDKRL HCYASWRNSSGTIELVKKGCWDDDFNCYDRQECVATEE NPQVYFCCCEGNFCNERFTHLPEAGGPEVTYEPPPTAPT GGGTHTCPPCPAPELLGGPSVFLFPPKPKDTLMISRTPEV TCVVVDVSHEDPEVKFNWYVDGVEVHNAKTKPREEQY NSTYRVVSVLTVLHQDWLNGKEYKCKVSNKALPAPIEK TISKAKGQPREPQVYTLPPSREEMTKNQVSLTCLVKGFY PSDIAVEWESNGQPENNYKTTPPVLDSDGSFFLYSKLTV DKSRWQQGNVFSCSVMHEALHNHYTQKSLSLSPGK |
| 25 | human ActRIIB soluble (extracellular), processed polypeptide sequence (amino acids 20-134 of SEQ ID NO:2) with L79D mutation fused to an Fc domain | GRGEAETRECIYYNANWELERTNQSGLERCEGEQDKRL HCYASWANSSGTIELVKKGCWDDDFNCYDRQECVATEE NPQVYFCCCEGNFCNERFTHLPEAGGPEVTYEPPPTAPT GGGTHTCPPCPAPELLGGPSVFLFPPKPKDTLMISRTPEV TCVVVDVSHEDPEVKFNWYVDGVEVHNAKTKPREEQY NSTYRVVSVLTVLHQDWLNGKEYKCKVSNKALPAPIEK TISKAKGQPREPQVYTLPPSREEMTKNQVSLTCLVKGFY PSDIAVEWESNGQPENNYKTTPPVLDSDGSFFLYSKLTV DKSRWQQGNVFSCSVMHEALHNHYTQKSLSLSPGK |
| 26 | human ActRIIB soluble (extracellular), processed polypeptide sequence (amino acids 20-134 of SEQ ID NO:14) with L79D mutation fused to an Fc domain and with TPA leader sequence | MDAMKRGLCCVLLLCGAVFVSPGASGRGEAETRECIYY NANWELERTNQSGLERCEGEQDKRLHCYASWRNSSGTI ELVKKGCWDDDFNCYDRQECVATEENPQVYFCCCEGN FCNERFTHLPEAGGPEVTYEPPPTAPTGGGTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQ VYTLPPSREEMTKNQVSLTCLVKGFYPSDIAVEWESNGQ PENNYKTTPPVLDSDGSFFLYSKLTVDKSRWQQGNVFSC SVMHEALHNHYTQKSLSLSPGK |
| 27 | human ActRIIB soluble (extracellular), processed polypeptide sequence (amino acids 20-134 of SEQ ID NO:2) with L79D mutation fused to an Fc domain and with TPA leader sequence | MDAMKRGLCCVLLLCGAVFVSPGASGRGEAETRECIYY NANWELERTNQSGLERCEGEQDKRLHCYASWANSSGTI ELVKKGCWDDDFNCYDRQECVATEENPQVYFCCCEGN FCNERFTHLPEAGGPEVTYEPPPTAPTGGGTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQ VYTLPPSREEMTKNQVSLTCLVKGFYPSDIAVEWESNGQ PENNYKTTPPVLDSDGSFFLYSKLTVDKSRWQQGNVFSC SVMHEALHNHYTQKSLSLSPGK |
| 28 | human ActRIIB soluble (extracellular), processed polypeptide sequence having a variant C-terminal sequence (disclosed in WO2007/053775) | GRGEAETRECIYYNANWELERTNQSGLERCEGEQDKRL HCYASWRNSSGTIELVKKGCWLDDFNCYDRQECVATEE NPQVYFCCCEGNFCNERFTHLPEAGGPEGPWASTTIPSG GPEATAAAGDQGSGALWLCLEGPAHE |
| 29 | human ActRIIB soluble (extracellular), processed polypeptide sequence having a variant C-terminal sequence (disclosed in WO2007/053775) having an L79D mutation | GRGEAETRECIYYNANWELERTNQSGLERCEGEQDKRL HCYASWRNSSGTIELVKKGCWDDDFNCYDRQECVATEE NPQVYFCCCEGNFCNERFTHLPEAGGPEGPWASTTIPSG GPEATAAAGDQGSGALWLCLEGPAHE |
| 30 | human ActRIIB soluble (extracellular), processed polypeptide sequence having a variant C-terminal sequence (disclosed in WO2007/053775) having an L79D mutation fused to an Fc domain with a TGGG linker | GRGEAETRECIYYNANWELERTNQSGLERCEGEQDKRL HCYASWRNSSGTIELVKKGCWDDDFNCYDRQECVATEE NPQVYFCCCEGNFCNERFTHLPEAGGPEGPWASTTIPSG GPEATAAAGDQGSGALWLCLEGPAHETGGGTHTCPPCP APELLGGPSVFLFPPKPKDTLMISRTPEVTCVVVDVSHED PEVKFNWYVDGVEVHNAKTKPREEQYNSTYRVVSVLT VLHQDWLNGKEYKCKVSNKALPAPIEKTISKAKGQPREP QVYTLPPSREEMTKNQVSLTCLVKGFYPSDIAVEWESNG QPENNYKTTPPVLDSDGSFFLYSKLTVDKSRWQQGNVFS CSVMHEALHNHYTQKSLSLSPGK |
| 31 | Nucleic Acid Sequence Encoding SEQ ID NO:10 | ATGGATGCAATGAAGAGAGGGCTCTGCTGTGTGCTGC TGCTGTGTGGAGCAGTCTTCGTTTCGCCCGGCGCCGCC GAAACCCGCGAATGTATTTATTACAATGCTAATTGGG AACTCGAACGGACGAACCAATCCGGGCTCGAACGGTG TGAGGGGGAACAGGATAAACGCCTCCATTGCTATGCG TCGTGGAGGAACTCCTCCGGGACGATTGAACTGGTCA AGAAAGGGTGCTGGGACGACGATTTCAATTGTTATGA CCGCCAGGAATGTGTCGCGACCGAAGAGAATCCGCAG GTCTATTTCTGTTGTTGCGAGGGGAATTTCTGTAATGA ACGGTTTACCCACCTCCCCGAAGCCGGCGGGCCCGAG GTGACCTATGAACCCCCGCCCACCGGTGGTGGAACTC ACACATGCCCACCGTGCCCAGCACCTGAACTCCTGGG GGGACCGTCAGTCTTCCTCTTCCCCCCAAAACCCAAGG ACACCCTCATGATCTCCCGGACCCCTGAGGTCACATGC GTGGTGGTGGACGTGAGCCACGAAGACCCTGAGGTCA AGTTCAACTGGTACGTGGACGGCGTGGAGGTGCATAA TGCCAAGACAAAGCCGCGGGAGGAGCAGTACAACAG CACGTACCGTGTGGTCAGCGTCCTCACCGTCCTGCACC AGGACTGGCTGAATGGCAAGGAGTACAAGTGCAAGGT CTCCAACAAAGCCCTCCCAGCCCCCATCGAGAAAACC ATCTCCAAAGCCAAAGGGCAGCCCCGAGAACCACAGG TGTACACCCTGCCCCCATCCCGGGAGGAGATGACCAA GAACCAGGTCAGCCTGACCTGCCTGGTCAAAGGCTTC TATCCCAGCGACATCGCCGTGGAGTGGGAGAGCAATG GGCAGCCGGAGAACAACTACAAGACCACGCCTCCCGT GCTGGACTCCGACGGCTCCTTCTTCCTCTATAGCAAGC TCACCGTGGACAAGAGCAGGTGGCAGCAGGGGAACG TCTTCTCATGCTCCGTGATGCATGAGGCTCTGCACAAC CACTACACGCAGAAGAGCCTCTCCCTGTCCCCGGGTA AATGA |

| **Q ID NO.SE** | **DESCRITION** | **SEQUENCE** |
|---|---|---|
| 32 | fusion protein comprising a soluble extracellular domain of ActRIIB (R64; SEQ ID NO:15) fused to an Fc domain | SGRGEAETRECIYYNANWELERTNQSGLERCEGEQDKR LHCYASWRNSSGTIELVKKGCWLDDFNCYDRQECVATE ENPQVYFCCCEGNFCNERFTHLPEAGGPEVTYEPPPTAPT GGGTHTCPPCPAPELLGGPSVFLFPPKPKDTLMISRTPEV TCVVVDVSHEDPEVKFNWYVDGVEVHNAKTKPREEQY NSTYRVVSVLTVLHQDWLNGKEYKCKVSNKALPVPIEK TISKAKGQPREPQVYTLPPSREEMTKNQVSLTCLVKGFY PSDIAVEWESNGQPENNYKTTPPVLDSDGSFFLYSKLTV DKSRWQQGNVFSCSVMHEALHNHYTQKSLSLSPGK |
| 33 | fusion protein comprising a soluble extracellular domain of ActRIIB (R64) with the C-terminal 15 amino acids deleted (SEQ ID NO: 16) fused to an Fc domain | SGRGEAETRECIYYNANWELERTNQSGLERCEGEQDKR LHCYASWRNSSGTIELVKKGCWLDDFNCYDRQECVATE ENPQVYFCCCEGNFCNERFTHLPEAGGGTHTCPPCPAPE LLGGPSVFLFPPKPKDTLMISRTPEVTCVVVDVSHEDPEV KFNWYVDGVEVHNAKTKPREEQYNSTYRVVSVLTVLH QDWLNGKEYKCKVSNKALPVPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGK |
| 34 | Nucleic acid sequence that encodes SEQ ID NO: 11 and tissue plasminogen activator (TPA signal peptide sequence) | ATGGATGCAATGAAGAGA GGGCTCTGCTGTGTGCTG CTGCTGTGTGGAGCAGTC   TTCGTTTCGCCCGGCGCC GCCGAAACCCGCGAATGT   ATTTATTACAATGCTAAT TGGGAACTCGAACGGACG   AACCAATCCGGGCTCGAA CGGTGTGAGGGGGAACAG   GATAAACGCCTCCATTGC TATGCGTCGTGGAGGAAC   TCCTCCGGGACGATTGAA CTGGTCAAGAAAGGGTGC   TGGGACGACGATTTCAAT TGTTATGACCGCCAGGAA   TGTGTCGCGACCGAAGAG AATCCGCAGGTCTATTTC   TGTTGTTGCGAGGGGAAT TTCTGTAATGAACGGTTT   ACCCACCTCCCCGAAGCC GGCGGGCCCGAGGTGACC   TATGAACCCCCGCCCACC GGTGGTGGAACTCACACA   TGCCCACCGTGCCCAGCA CCTGAACTCCTGGGGGGA   CCGTCAGTCTTCCTCTTC CCCCCAAAACCCAAGGAC   ACCCTCATGATCTCCCGG ACCCCTGAGGTCACATGC   GTGGTGGTGGACGTGAGC CACGAAGACCCTGAGGTC   AAGTTCAACTGGTACGTG GACGGCGTGGAGGTGCAT   AATGCCAAGACAAAGCCG CGGGAGGAGCAGTACAAC   AGCACGTACCGTGTGGTC AGCGTCCTCACCGTCCTG   CACCAGGACTGGCTGAAT GGCAAGGAGTACAAGTGC   AAGGTCTCCAACAAAGCC CTCCCAGCCCCCATCGAG   AAAACCATCTCCAAAGCC AAAGGGCAGCCCCGAGAA   CCACAGGTGTACACCCTG CCCCCATCCCGGGAGGAG   ATGACCAAGAACCAGGTC AGCCTGACCTGCCTGGTC   AAAGGCTTCTATCCCAGC GACATCGCCGTGGAGTGG   GAGAGCAATGGGCAGCCG GAGAACAACTACAAGACC   ACGCCTCCCGTGCTGGAC TCCGACGGCTCCTTCTTC   CTCTATAGCAAGCTCACC GTGGACAAGAGCAGGTGG   CAGCAGGGGAACGTCTTC TCATGCTCCGTGATGCAT   GAGGCTCTGCACAACCAC TACACGCAGAAGAGCCTC   TCCCTGTCTCCGGGTAAA TGA |
| | (TPA signal peptide sequence underlined and shown in bold). | |

### 9. EQUIVALENTS

Although the invention is described in detail with reference to specific embodiments thereof, it will be understood that variations which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims. Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference in their entireties.

### Numbered Embodiments

1. A method for treating anemia or for enhancing late stage erythropoiesis in a subject in need thereof, comprising:
   administering to the subject an ActRIIB ligand trap; and
   administering to the subject an mTOR inhibitor.
2. The method of embodiment 1, wherein the anemia is an anemia associated with ineffective erythropoiesis, thalassemia, alpha-thalassemia, beta-thalassemia, myelodysplastic syndromes (MDS), or non-proliferative chronic myelomonocytic leukemia (CMML).
3. The method of embodiment 1 or 2, wherein the mTOR inhibitor is rapamycin.
4. The method of any of embodiments 1 to 3, wherein the mTOR inhibitor is a pharmaceutically acceptable salt or hydrate of rapamycin.
5. The method of any one of embodiments 1 to 4, wherein the ActRIIB ligand trap is a polypeptide comprising:
   (a) 90% identical to SEQ ID NO:11;
   (b) 95% identical to SEQ ID NO:11;
   (c) 98% identical to SEQ ID NO:11; or
   (d) SEQ ID NO:11
6. The method of any one of embodiments 1 to 5, wherein the ActRIIB ligand trap is a polypeptide comprising an amino acid sequence of SEQ ID NO:11.
7. The method of any one of embodiments 1 to 6, wherein the method increases hemoglobin (HGB) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject prior to said treating.
8. The method of any one of embodiments 1 to 7, wherein the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HCT levels in the subject prior to said treating.
9. The method of any one of embodiments 1 to 8, wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than MCV levels in the subject prior to said treating.
10. The method of any one of embodiments 1 to 9, wherein the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than CHC levels in the subject prior to said treating.
11. The method of any one of embodiments 1 to 10, wherein the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than the RDW levels in the subject prior to said treating.
12. The method of any one of embodiments 1 to 11, wherein the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in the subject prior to said treating.
13. The method of any one of embodiments 1 to 11, wherein the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in a reference population.
14. The method of any one of embodiments 1 to 13, wherein the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in the subject prior to said treating.
15. The method of any one of embodiments 1 to 13, wherein the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in a reference population.
16. The method of any one of embodiments 1 to 15, wherein the mTOR inhibitor is administered before or concurrently with the administration of ActRIIB ligand trap.
17. The method of any one of embodiments 1 to 15, wherein the subject has been previously treated with the mTOR inhibitor prior the administration of ActRIIB ligand trap.
18. The method of any one of embodiments 1 to 15, wherein the subject has been previously treated with the ActRIIB ligand trap prior the administration of mTOR inhibitor.
19. The method of any one of embodiments 1 to 18, wherein the subject is red blood cell non-transfusion-dependent.
20. The method of any one of embodiments 1 to 18, wherein the subject is red blood cell transfusion-dependent.
21. The method of any one of embodiments 1 to 20, wherein the ActRIIB ligand trap is administered with a dose of 0.6 mg/kg, 0.8 mg/kg, 1 mg/kg, 1.33 mg/kg, or 1.75 mg/kg.
22. The method of any one of embodiments 1 to 20, wherein the ActRIIB ligand trap is administered to the subject once every 21 days.
23. The method of any one of embodiments 1 to 22, wherein the ActRIIB ligand trap is administered to the subject subcutaneously.
24. The method of any one of embodiments 1 to 23, wherein the subject is a human.
25. The method of any one of embodiments 13, 15, and 16 to 24, wherein the reference population consists of 1, 5, 10, 25, 50, 75, 100, 200, 250, 300, 400, 500, or 1000 individuals.
26. The method of any one of embodiments 13, 15, and 16 to 25, wherein the reference population consists of healthy individuals.
27. The method of any one of embodiments 13, 15, and 16 to 26 wherein the reference population consists of people of the same age, weight, and/or gender as the subject.
28. The method of any one of embodiments 1 to 27, wherein the mTOR inhibitor is administered orally.
29. The method of any one of embodiments 1 to 28, wherein the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg.
30. The method of any one of embodiments 1 to 29, wherein the mTOR inhibitor is administered daily.
31. A method for treating anemia or for enhancing late stage erythropoiesis in a subject in need thereof, comprising:
   administering to the subject an ActRIIB ligand trap; and
   administering to the subject an mTOR inhibitor,
   wherein the method increases hemoglobin (HGB) levels in a reference population to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject who received either an ActRIIB ligand trap or an mTOR inhibitor, but not together.
32. A method for treating anemia or for enhancing late stage erythropoiesis in a subject in need thereof, comprising:
   administering to the subject an ActRIIB ligand trap; and
   administering to the subject an mTOR inhibitor,
   wherein the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together.
33. A method for treating anemia or for enhancing late stage erythropoiesis in a subject in need thereof, comprising:
   administering to the subject an ActRIIB ligand trap; and
   administering to the subject an mTOR inhibitor,
   wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than hemoglobin (HGB) levels in a reference population who received either an ActRIIB ligand trap or an mTOR inhibitor, but not together.
34. A method for treating anemia or for enhancing late stage erythropoiesis in a subject in need thereof, comprising:
   administering to the subject an ActRIIB ligand trap; and
   administering to the subject an mTOR inhibitor,
   wherein the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together.
35. A method for treating anemia or for enhancing late stage erythropoiesis in a subject in need thereof, comprising:
   administering to the subject an ActRIIB ligand trap; and
   administering to the subject an mTOR inhibitor,
   wherein the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than hemoglobin (HGB) levels in a reference population who received either an ActRIIB ligand trap or an mTOR inhibitor, but not together.
36. A method for treating anemia or for enhancing late stage erythropoiesis in a subject in need thereof, comprising:
   administering to the subject an ActRIIB ligand trap; and
   administering to the subject an mTOR inhibitor,
   wherein the levels of reticulocytes in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together.
37. A method for treating anemia or for enhancing late stage erythropoiesis in a subject in need thereof, comprising:
   administering to the subject an ActRIIB ligand trap; and
   administering to the subject an mTOR inhibitor,
   wherein the levels of white blood cells in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together.
38. The method of any one of embodiments 31 to 37, wherein the subject is a human.
39. The method of any one of embodiments 31 to 38, wherein the reference population consists of 1, 5, 10, 25, 50, 75, 100, 200, 250, 300, 400, 500, or 1000 individuals.
40. The method of any one of embodiments 31 to 39 wherein the reference population consists of people of the same age, weight, and/or gender as the subject.
41. The method of any one of embodiments 31 to 40, wherein the reference population consists of individuals with anemia.
42. The method of any one of embodiments 31 to 41, wherein the anemia is an anemia associated with ineffective erythropoiesis, thalassemia, alpha-thalassemia, beta-thalassemia, myelodysplastic syndromes (MDS), or non-proliferative chronic myelomonocytic leukemia (CMML).
43. The method of any one of embodiments 31 to 42, wherein the mTOR inhibitor is rapamycin.
44. The method of any one of embodiments 31 to 43, wherein the mTOR inhibitor is a pharmaceutically acceptable salt or hydrate of rapamycin.
45. The method of any one of embodiments 31 to 44, wherein the ActRIIB ligand trap is a polypeptide comprising:
   (a) 90% identical to SEQ ID NO:11;
   (b) 95% identical to SEQ ID NO:11;
   (c) 98% identical to SEQ ID NO:11; or
   (d) SEQ ID NO:11
46. The method of any one of embodiments 31 to 45, wherein the ActRIIB ligand trap is a polypeptide comprising an amino acid sequence of SEQ ID NO:11.
47. The method of any one of embodiments 31 to 46, wherein the subject is red blood cell non-transfusion-dependent.
48. The method of any one of embodiments 31 to 46, wherein the subject is red blood cell transfusion-dependent.
49. The method of any one of embodiments 31 to 48, wherein the ActRIIB ligand trap is administered with a dose of 0.6 mg/kg, 0.8 mg/kg, 1 mg/kg, 1.33 mg/kg, or 1.75 mg/kg.
50. The method of any one of embodiments 31 to 49, wherein the ActRIIB ligand trap is administered to the subject once every 21 days.
51. The method of any one of embodiments 31 to 50, wherein the ActRIIB ligand trap is administered to the subject subcutaneously.
52. The method of any one of embodiments 31 to 51, wherein the mTOR inhibitor is administered orally.
53. The method of any one of embodiments 31 to 52, wherein the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg.
54. The method of any one of embodiments 31 to 53, wherein the mTOR inhibitor is administered daily.

## Claims

1. An ActRIIB ligand trap for use in a method for treating anemia or for enhancing late stage erythropoiesis in a subject in need thereof, comprising:
administering to the subject the ActRIIB ligand trap; and
administering to the subject an mTOR inhibitor.

2. An mTOR inhibitor for use in a method for treating anemia or for enhancing late stage erythropoiesis in a subject in need thereof, comprising:
administering to the subject an ActRIIB ligand trap; and
administering to the subject the mTOR inhibitor.

3. A pharmaceutical composition compising an ActRIIB ligand trap and an mTOR inhibitor and a pharmaceutically acceptable carrier for use in a method for treating anemia or for enhancing late stage erythropoiesis in a subject in need thereof.

4. The ActRIIB ligand trap for use according to claim 1, the mTOR inhibitor for use for use according to claim 2, or the pharmaceutical composition for use according to claim 3, wherein:
a) the anemia is an anemia associated with ineffective erythropoiesis, thalassemia, alpha-thalassemia, beta-thalassemia, myelodysplastic syndromes (MDS), or non-proliferative chronic myelomonocytic leukemia (CMML); and/or
b) the mTOR inhibitor is rapamycin; and/or
c) the mTOR inhibitor is a pharmaceutically acceptable salt or hydrate of rapamycin; and/or
d) the ActRIIB ligand trap is a polypeptide comprising:
(a) 90% identical to SEQ ID NO:11;
(b) 95% identical to SEQ ID NO:11;
(c) 98% identical to SEQ ID NO:11; or
(d) SEQ ID NO:11; and/or
e) the ActRIIB ligand trap is a polypeptide comprising an amino acid sequence of SEQ ID NO:11.

5. The ActRIIB ligand trap for use according to claims 1 or 4, the mTOR inhibitor for use for use according to claims 2 or 4, or the pharmaceutical composition for use according to claims 3 or 4, wherein:
a) the method increases hemoglobin (HGB) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject prior to said treating and/or
b) the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HCT levels in the subject prior to said treating and/or
c) the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than MCV levels in the subject prior to said treating and/or
d) the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than CHC levels in the subject prior to said treating and/or
e) the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, or 100%, less than the RDW levels in the subject prior to said treating.

6. The ActRIIB ligand trap for use according to claims 1, 4 or 5, the mTOR inhibitor for use for use according to claims 2, 4 or 5, or the pharmaceutical composition for use according to claims 3, 4 or 5, wherein:
a) the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in the subject prior to said treating or
b) the levels of reticulocytes in the subject remain in the range equal to or about 0.1%, 0.5%, 1%, 2%, 3%, 4%, 5%, 6%, 7%, 8%, 9%, 10%, 15%, or 20% above or below the levels of reticulocytes in a reference population.

7. The ActRIIB ligand trap for use according to claims 1 or 4-6, the mTOR inhibitor for use for use according to claims 2 or 4-6, or the pharmaceutical composition for use according to claims 3 or 4-6, wherein:
a) the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in the subject prior to said treating or
b) the levels of white blood cells in the subject remain in the range equal to or about 0.1%, 0.5%, 1%, 2%, 3%, 4%, 5%, 6%, 7%, 8%, 9%, 10%, 15%, or 20% above or below the levels of white blood cells in a reference population.

8. The ActRIIB ligand trap for use according to claims 1 or 4-7, the mTOR inhibitor for use for use according to claims 2 or 4-7, or the pharmaceutical composition for use according to claims 3 or 4-7, wherein the mTOR inhibitor is administered before or concurrently with the administration of ActRIIB ligand trap or the subject has been previously treated with the mTOR inhibitor prior the administration of ActRIIB ligand trap or the subject has been previously treated with the ActRIIB ligand trap prior the administration of mTOR inhibitor.

9. The ActRIIB ligand trap for use according to claims 1 or 4-8, the mTOR inhibitor for use for use according to claims 2 or 4-8, or the pharmaceutical composition for use according to claims 3 or 4-8, wherein:
a) the subject is red blood cell non-transfusion-dependent or wherein the subject is red blood cell transfusion-dependent; and/or
b) the ActRIIB ligand trap is administered with a dose of 0.6 mg/kg, 0.8 mg/kg, 1 mg/kg, 1.33 mg/kg, or 1.75 mg/kg or the ActRIIB ligand trap is administered to the subject once every 21 days.

10. The ActRIIB ligand trap for use according to claims 1 or 4-9, the mTOR inhibitor for use for use according to claims 2 or 4-9, or the pharmaceutical composition for use according to claims 3 or 4-9, wherein the ActRIIB ligand trap is administered to the subject subcutaneously and/or the subject is a human.

11. The ActRIIB ligand trap for use according to claims 6b), 7b), or 8-10, the mTOR inhibitor for use for use according to claims 6b), 7b), or 8-10, or the pharmaceutical composition for use according to claims 6b), 7b), or 8-10, wherein the reference population consists of 1, 5, 10, 25, 50, 75, 100, 200, 250, 300, 400, 500, or 1000 individuals and/or the reference population consists of healthy individuals and/or the reference population consists of people of the same age, weight, and/or gender as the subject.

12. The ActRIIB ligand trap for use according to claims 1 or 4-11, the mTOR inhibitor for use for use according to claims 2 or 4-11, or the pharmaceutical composition for use according to claims 3 or 4-11, wherein the mTOR inhibitor is administered orally and/or wherein the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg and/or wherein the mTOR inhibitor is administered daily.

13. The ActRIIB ligand trap for use according to claim 1, the mTOR inhibitor for use for use according to claim 2, or the pharmaceutical composition for use according to claim 3, wherein:
a) the method increases hemoglobin (HGB) levels in a reference population to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in the subject who received either an ActRIIB ligand trap or an mTOR inhibitor, but not together; or
b) wherein the method increases hematocrit (HCT) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than HGB levels in a reference population who received either an ActRIIB ligand trap or an mTOR inhibitor, but not together; or
c) wherein the method reduces mean corpuscular volume (MCV) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than hemoglobin (HGB) levels in a reference population who received either an ActRIIB ligand trap or an mTOR inhibitor, but not together; or
d) wherein the method increases corpuscular hemoglobin concentration (CHC) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% greater than hemoglobin (HGB) levels in a reference population who received either an ActRIIB ligand trap or an mTOR inhibitor, but not together; or
e) wherein the method reduces red blood cell distribution width (RDW) levels in the subject to levels equal to or about 1%, 2%, 3%, 4%, 5%, 6%, 7%, 8%, 9%, 10%, 20%, 25%, 30%, 40%, 50%, 60%, 70%, 75%, 80%, 90%, 100%, 200%, or 500% less than hemoglobin (HGB) levels in a reference population who received either an ActRIIB ligand trap or an mTOR inhibitor, but not together; or
f) wherein the levels of reticulocytes in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together; or
g) wherein the levels of white blood cells in the subject deviant from normal levels of reticulocytes equal to or about 0.1%, 0.5%, 1%, 2%, 3%, 4%, 5%, 6%, 7%, 8%, 9%, 10%, 15%, or 20% less as compared to the levels of reticulocytes in a reference population who received either an ActRIIB ligand trap or an mTOR inhibitor, but not together.

14. The ActRIIB ligand trap for use according to claim 13, the mTOR inhibitor for use for use according to claim 13, or the pharmaceutical composition for use according to claim 13, wherein:
a) the subject is a human; and/or
b) the reference population consists of 1, 5, 10, 25, 50, 75, 100, 200, 250, 300, 400, 500, or 1000 individuals; and/or
c) the reference population consists of people of the same age, weight, and/or gender as the subject; and/or
d) the reference population consists of individuals with anemia; and/or
e) the anemia is an anemia associated with ineffective erythropoiesis, thalassemia, alpha-thalassemia, beta-thalassemia, myelodysplastic syndromes (MDS), or non-proliferative chronic myelomonocytic leukemia (CMML); and/or
f) the mTOR inhibitor is rapamycin; and/or
g) the mTOR inhibitor is a pharmaceutically acceptable salt or hydrate of rapamycin; and/or
h) the ActRIIB ligand trap is a polypeptide comprising:
(a) 90% identical to SEQ ID NO:11;
(b) 95% identical to SEQ ID NO:11;
(c) 98% identical to SEQ ID NO:11; or
(d) SEQ ID NO:11; and/or
i) the ActRIIB ligand trap is a polypeptide comprising an amino acid sequence of SEQ ID NO:11.

15. The ActRIIB ligand trap for use according to claims 13 or 14, the mTOR inhibitor for use for use according to claims 13 or 14, or the pharmaceutical composition for use according to claims 13 or 14, wherein:
a) the subject is red blood cell non-transfusion-dependent or wherein the subject is red blood cell transfusion-dependent; and/or
b) the ActRIIB ligand trap is administered with a dose of 0.6 mg/kg, 0.8 mg/kg, 1 mg/kg, 1.33 mg/kg, or 1.75 mg/kg; and/or
c) the ActRIIB ligand trap is administered to the subject once every 21 days; and/or
d) the ActRIIB ligand trap is administered to the subject subcutaneously; and/or
e) the mTOR inhibitor is administered orally; and/or
f) the mTOR inhibitor is administered at a dose of 0.1 mg/kg, 0.2 mg/kg, 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, or 15 mg/kg; and/or
g) the mTOR inhibitor is administered daily.
